# EUROPEAN PATENT SPECIFICATION

(11) **EP 2 417 155 B1**
(45) Date of publication and mention of the grant of the patent: **19.06.2013**
(21) Application number: 10715162.3
(22) Date of filing: 06.04.2010
(51) Int. Cl.: C07K 14/755, A61K 38/37, A61K 38/48, C07K 19/00

(54) **TARGETED DELIVERY OF FACTOR VIII PROTEINS TO PLATELETS**
GEZIELTE FRESETZUNG VON FAKTOR-VIII-PROTEINEN AUF THROMBOZYTEN
ADMINISTRATION CIBLÉE AUX PLAQUETTES DE PROTÉINES DE FACTEUR VIII

(30) Priority: 06.04.2009 US 212004 P; 11.06.2009 US 186075 P
(43) Date of publication of application: 15.02.2012
(73) Proprietor: Novo Nordisk A/S, 2880 Bagsværd (DK)
(72) Inventor: ØSTERGAARD, Henrik, DK-3650 Ølstykke (DK); PUSATERI, Tony, New Jersey 08844 (US); BARNETT, Thomas R., North Carolina 27514-1415 (US); BUCHARDT, Jens, DK-2820 Gentofte (DK); PESCHKE, Bernd, DK-2760 Måløv (DK); KOFOD-HANSEN, Mikael, DK-2200 København N (DK); ZUNDEL, Magali A., DK-2870 Dyssegaard (DK); BEHRENS, Carsten, DK-2200 København N (DK); OLSEN, Eva H. Norling, DK-2750 Ballerup (DK); STENNICKE, Henning, DK-2980 Kokkedal (DK)
(86) International application number: PCT/EP2010/054495
(87) International publication number: WO 2010/115866

(56) References cited:
- WO-A1-89/11538
- WO-A1-2008/077616
- WO-A1-2009/140598
- NEYMAN MICHAEL ET AL: "Analysis of the spatial and temporal characteristics of platelet-delivered factor VIII-based clots." BLOOD 15 AUG 2008 LNKD- PUBMED:18559671, vol. 112, no. 4, 15 August 2008 (2008-08-15), pages 1101-1108, XP002591257 ISSN: 1528-0020
- DAMON ANDREA L ET AL: "Megakaryotyte/platelet-restricted FVIII expression using a platelet factor 4 promoter: Determination of activation state and in vivo efficacy during thrombocytosis." BLOOD, vol. 110, no. 11, Part 1, November 2007 (2007-11), page 346A, XP008124220 & 49TH ANNUAL MEETING OF THE AMERICAN-SOCIETY-OF-HEMATOLOGY; ATLANTA, GA, USA; DECEMBER 08 -11, 2007 ISSN: 0006-4971
- GEWIRTZ JAMIE E ET AL: "Characterizing factor VIII inhibitor resistance of platelet-delivered factor VIII in the treatment of hemophilia A" BLOOD, vol. 110, no. 11, Part 1, November 2007 (2007-11), page 240A, XP008124212 & 49TH ANNUAL MEETING OF THE AMERICAN-SOCIETY-OF-HEMATOLOGY; ATLANTA, GA, USA; DECEMBER 08 -11, 2007 ISSN: 0006-4971
- SHI QIZHEN ET AL: "Factor VIII ectopically targeted to platelets is therapeutic in hemophilia A with high-titer inhibitory antibodies." THE JOURNAL OF CLINICAL INVESTIGATION JUL 2006 LNKD- PUBMED:16823491, vol. 116, no. 7, July 2006 (2006-07), pages 1974-1982, XP002591260 ISSN: 0021-9738
- FERGUSON ET AL: "Fundamentals of coagulation and glycoprotein IIb/IIIa receptor inhibition" AMERICAN HEART JOURNAL, MOSBY- YEAR BOOK INC, US LNKD- DOI:10.1016/S0002-8703(98)70296-0, vol. 135, no. 4, 1 April 1998 (1998-04-01) , pages S35-S42, XP005689968 ISSN: 0002-8703
- SCHWARZ MEIKE ET AL: "Conformation-specific blockade of the integrin GPIIb/IIIa: a novel antiplatelet strategy that selectively targets activated platelets." CIRCULATION RESEARCH 7 JUL 2006 LNKD- PUBMED:16778135, vol. 99, no. 1, 7 July 2006 (2006-07-07), pages 25-33, XP002591261 ISSN: 1524-4571 DOI: 10.1161/01.RES.0000232317.84122.0c
- CHUNG JUNHO ET AL: "Integrin alphaIIbbeta3-specific synthetic human monoclonal antibodies and HCDR3 peptides that potently inhibit platelet aggregation." THE FASEB JOURNAL : OFFICIAL PUBLICATION OF THE FEDERATION OF AMERICAN SOCIETIES FOR EXPERIMENTAL BIOLOGY FEB 2004 LNKD- PUBMED:14688205, vol. 18, no. 2, February 2004 (2004-02), pages 361-363, XP002591262 ISSN: 1530-6860

## Description

### FIELD OF THE INVENTION

The invention described herein relates to polypeptides comprising at least one amino acid sequence having significant identity with (homology to) human Factor VIII or biologically active portion(s) thereof, related molecules (such as nucleic acids encoding such polypeptides), compositions (such as pharmaceutical formulations) comprising such polypeptides, and methods of making and using such polypeptides and related biological molecules.

### BACKGROUND OF THE INVENTION

Coagulation Factor VIII ("Factor VIII" or "FVIII") is an essential clotting factor. The lack of normal FVIII causes Hemophilia A, an inherited bleeding disorder. Factor VIII participates in the intrinsic pathway of blood coagulation. Specifically, Factor VIII is a cofactor for Factor IXa which converts Factor X to activated Factor Xa (in the presence of Ca⁺² and phospholipids).

The Factor VIII gene produces two alternatively spliced transcripts. Transcript variant 1 encodes a large glycoprotein, isoform a, which circulates in plasma and associates with von Willebrand factor ("vWF") in a noncovalent complex. This protein undergoes multiple cleavage events. Transcript variant 2 encodes a putative small protein, isoform b, which consists primarily of the phospholipid binding domain of factor VIIIc. This binding domain is essential for coagulant activity. The structure of FVIII is well known (see, e.g., Thompson, Semin Thromb Hemost. 2003 Feb;29(1):11-22).

Upon activation by thrombin, (Factor IIa), Factor VIIIa (FVIIIa) dissociates from the FVIII:vWF complex to interact with Factor IXa causing a well characterized chain of events that leads to the production of more thrombin. Thrombin cleaves fibrinogen into fibrin which polymerizes and crosslinks (using Factor XIII) into a blood clot. FVIIIa is proteolytically inactivated in this process by activated Protein C ("aPC") and Factor IXa and quickly cleared from the blood stream.

FVIII concentrated from donated blood plasma or alternatively recombinant FVIII can be given to hemophiliacs to restore hemostasis. Thus, FVIII is also known as Anti-hemophilic factor.

FVIII has a circulatory half life of about 10-12 hours *in vivo* and it is therefore highly desirable to provide FVIII analogues with a prolonged half life in order to decrease the frequency of the therapeutic/prophylactic IV FVIII infusions.
Neymal *et al.* (Neyman M et al, "Analysis of the spatial and temporal characteristics of platelet-delivered factor VIII-based clots", Blood, vol. 112, no. 4, pages 1101-1108) disclose a method wherein factor VIII is targeted to the platelets via its ectopic expression.

W02009140598 discloses a concept including hybrid molecules comprising FVIII and GPIIb/IIIa specific antibodies. This concept may, however, result in inhibition of the ability of the GPIIb/IIIa receptors to bind fibrinogen and the ability to form a primary clot will thus be decreased upon administration of such hybrid FVIII molecules. There is thus a need in the art for biologically active Factor VIII molecules with a capacity to bind to platelets with high affinity. There is furthermore a need in the art for biologically active Factor VIII molecules with a capacity to bind to platelets while essentially retaining the capacity of the platelets to aggregate and thus form a primary clot.

### SUMMARY OF THE INVENTION

The present invention, in its broadest form, provides a FVIII molecule comprising an amino acid sequence that is at least 95% identical to the mature portion of an amino acid sequence selected from the list consisting of SEQ ID NO: 1 and SEQ ID NO: 3, which molecule is covalently attached to a platelet-specific molecule, wherein said platelet-specific molecule is a GPIIb/IIIa antibody that does not inhibit platelet aggregation (as recited in independent claim 1).
The present application also provides a nucleic acid coding for such a molecule, a host cell comprising said nucleic acid molecule as well as a method of producing such a FVIII molecule, a pharmaceutical composition comprising such a FVIII molecule and the use of such a FVIII molecule as a medicament for treatment of haemophilia A (as defined by the independent claims).
Preferable embodiments are defined by the dependent claims.

These and other aspects, features, and advantages of the invention will be more fully described herein.

### DESCRIPTION OF THE DRAWINGS

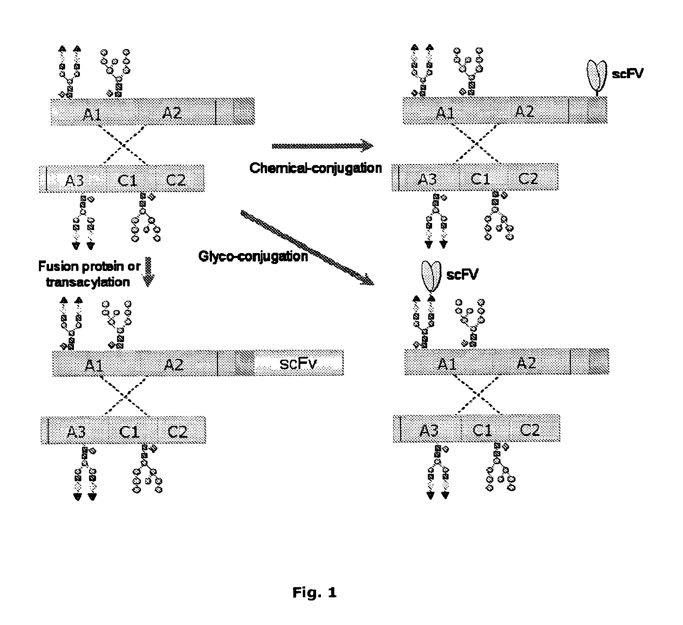

Figure 1 illustrates potential methods by which a scFv may be attached chemically to, or expressed in cis as part of the polypeptide with, a FVIII or FVIII analog amino acid sequence, in order to obtain a modified FVIII molecule. Specifically shown are glycoconjugation, chemical conjugation, and fusion protein approaches to the production of such a molecule. These methods can similarly be used, where suitable, to generate other FVIII molecules of the invention.
Figure 2 illustrates platelet aggregation. The bars show aggregation in SFLLRN (10 µM) activated platelets pre-treated with either F8-500 AP3-LC-HC scFV-Δa3 (AP3-N8) or ReoPro®. Data is shown as mean±sem of duplicate determinations in which SFLLRN (10 µM) alone was set as 100 %.
Figure 3 illustrates the final targeting vector

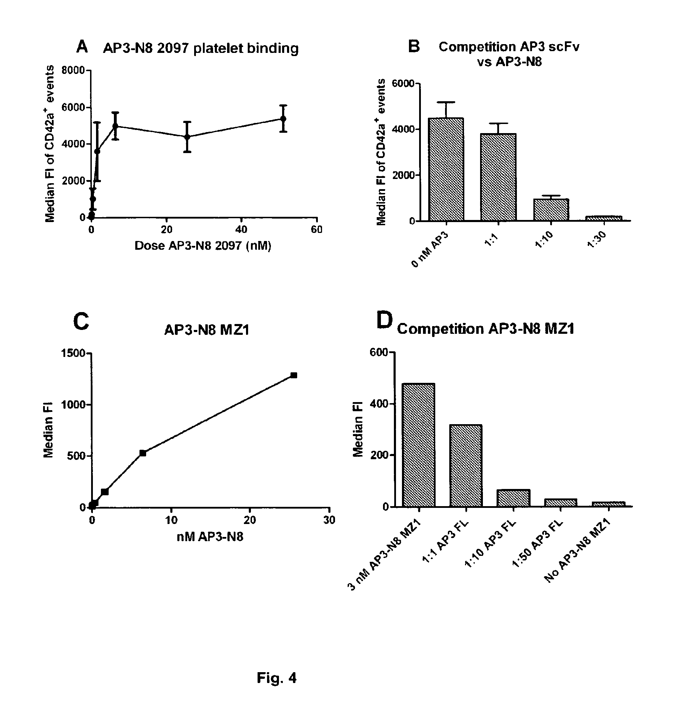

Figure 4 illustrates binding of AP3-N8 2097 (A) and AP3-N8 MZ1 (C) to human platelets in a dose-dependent manner. The binding of both compounds can be almost completely competed with excess AP3-LC-HC scFV-FLAG (SEQ ID NO 21).

### DESCRIPTION OF THE INVENTION

In the context of this invention, a Factor VIII protein (or FVIII sprotein) is a protein comprising an amino acid sequence exhibiting at least about 70% amino acid sequence identity to an amino acid sequence selected from human factor VIII (SEQ ID NO:1) or the mature portion thereof (i.e., residues 20-2351 thereof), or the thrombin activated portion thereof, or a B domain deleted/truncated version thereof as shown below.

### Factor VIII (SEQ ID NO:1)

### Factor VIII B-domain-deleted (SEQ ID NO:2)

### Factor VIII B-domain-truncated ("N8") (SEQ ID NO:3)

In a more particular aspect, the invention relates to targeted FVIII derivatives that exhibit at least about 75%, at least about 80%, or at least about 85%, or at least about 90%, or at least about 95% identity to SEQ ID NO:1 or the mature portion, or the thrombin activated portion thereof, or a B domain deleted/truncated version thereof.

In an even further particular aspect, the invention relates to targeted FVIII derivatives that exhibit at least about 90% identity (such as at least about 93% identity) to SEQ ID NO:1, SEQ ID NO: 2, or the mature portion or the thrombin activated portion thereof, or a B domain deleted/truncated version thereof.

In another aspect, the invention provides targeted FVIII derivatives that exhibit at least about 95% identity to SEQ ID NO:1 or the mature portion or ht ethrombin activated portion thereof. In more particular aspects, the invention provides targeted FVIII derivatives that exhibit at least about 96%, at least about 97%, at least about 98%, at least about 98.5%, at least about 99%, at least about 99.5%, or at least about 99.7%, at least about 99.8%, or even at least about 99.9% identity to SEQ ID NO:1 or the mature portion thereof, or the thrombin activated portion thereof, or a B domain deleted/truncated version thereof. Stated another way, in one aspect the invention provides targeted FVIII derivatives characterized by comprising an amino acid sequence in at least 1, typically at least 2-3, frequently at least 1-5, and commonly at least in which 1-20 (such as 1-15, 1-12, 1-10, 1-7, 1-3, 2-20, 2-15, 2-12, 2-10, 2-7, 2-5, 3-20, 3-15, 3-12, 3-10, 3-7, 3-5, 4-20, 4-15, 4-12, 4-10, 4-7, 5-20, 5-15, 5-12, 5-10, or 5-7) amino acid residues are deleted, inserted, and/or substituted with respect to SEQ ID NO:1, or the mature portion or the thrombin activated portion thereof, or a B domain deleted/truncated portion thereof.

Commonly, at least about 50%, at least about 60%, at least about 65%, at least about 70%, such as about 75% or more, about 80% or more, about 85% or more, about 90% or more, or even about 95% or more (such as at least about 97%, 98%, 99%, 99.3%, 99.5%, 99.7%, 99.8%) of the substitutions in the FVII "analog" sequence can be characterized as "conservative substitutions." Conservative substitutions can be defined by substitutions within the classes of amino acids reflected in one or more of the following three amino acid classification tables:

**Table 1 - Amino Acid Residue Classes for Conservative Substitutions**

| Amino Acid Class | Amino Acid Residues |
|---|---|
| Acidic Residues | ASP and GLU |
| Basic Residues | LYS, ARG, and HIS |
| Hydrophilic Uncharged Residues | SER, THR, ASN, and GLN |
| Aliphatic Uncharged Residues | GLY, ALA, VAL, LEU, and ILE |
| Non-polar Uncharged Residues | CYS, MET, and PRO |
| Aromatic Residues | PHE, TYR, and TRP |

**Table 2 - Alternative Conservative Amino Acid Residue Substitution Groups**

| | | | |
|---|---|---|---|
| 1 | Alanine (A) | Serine (S) | Threonine (T) |
| 2 | Aspartic acid (D) | Glutamic acid (E) | |
| 3 | Asparagine (N) | Glutamine (Q) | |
| 4 | Arginine (R) | Lysine (K) | |
| 5 | Isoleucine (I) | Leucine (L) | Methionine (M) |
| 6 | Phenylalanine (F) | Tyrosine (Y) | Tryptophan (W) |

**Table 3 - Further Alternative Physical and Functional Classifications of Amino Acid Residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | E, Q, T, K, S, G, P, D, E, and R |

Even more conservative amino acid residue substitution groupings include: valine-leucine-isoleucine, phenylalanine-tyrosine, lysine-arginine, alanine-valine, and asparagine-glutamine. Additional groups of amino acids can also be formulated using the principles described in, e.g., Creighton (1984) PROTEINS: STRUCTURE AND MOLECULAR PROPERTIES (2d Ed. 1993), W.H. Freeman and Company. In some instances it can be useful to further characterize substitutions based on two or more of such features (e.g., substitution with a "small polar" residue, such as a Thr residue, can represent a highly conservative substitution in an appropriate context).

The terms "targeted", "targeting" and the like are used to indicate that the FVIII molecule binds to one or more biological molecules (typically other proteins, and frequently cellular receptors) and/or cells with greater affinity and/or avidity than wild-type FVIII (or that are not typically bound by wild-type FVIII). As already noted, targeted molecules provided by the invention can exhibit increased stability as compared to a corresponding unmodified protein (a protein that lacks a "targeting factor," which usually is an amino acid sequence (a targeting domain) or molecule (a targeting moiety), but is otherwise identical to the targeted FVIII protein). In a particular aspect, targeted molecules that exhibit this or other desirable/modified properties are provided wherein one or more biological activities of FVIII are not significantly diminished (e.g., Factor IX binding). Typically, the amount of inhibition of such activity is about 40% or less, about 30% or less, about 25% or less, about 20% or less, about 15% or less, about 10% or less, about 7% or less, about 5% or less, or about 3% or less). Assays for FVIII biological activity are known in the art (see, e.g., Mikaelsson et al., Semin Hematol. 2001 Apr;38(2 Suppl 4):13-23).

The term "reduced capacity to bind vWF" is herein meant to encompass Factor VIII variants, wherein the capacity to bind vWF is decreased by at least 50%, preferably by at least 60%, more preferably by at least 70%, more preferably by at least 80%, more preferably by at least 90%, and most preferably about 100%. FVIII binding to vWF may be measured either by an ELISA like assay or as direct binding to immobilized vWF using surface plasmon resonance. The region in Factor VIII responsible for binding to vWF is the region spanning residues 1670-1684 as disclosed in EP0319315. It is envisaged that Factor VIII point and/or deletion mutants involving this area will modify the ability to bind to vWF. Examples of particularly preferred point mutations according to the present invention include variants comprising the following point mutations: Y1680F, Y1680R, Y1680N, Y1680C, and E1682T.

Side group: The Factor VIII molecules according to the present invention may be conjugated with a side group that is not a non-inhibitory GPIIb/IIIa antibody (i.e. an antibody that does not inhibit platelet aggregation). In this connection, a "side group" is to be understood as a covalent attachment of any moiety that is not naturally part of Factor VIII molecule. Preferably said side group is providing the Factor VIII molecule with a prolonged circulatory half-life. Conjugation of a side chain may be in the form of a fusion protein, and/or chemical conjugation and/or enzymatic conjugation processes. The side chain according to the present invention is typically selected from one or more of the list consisting of: hydrophilic polymers, fatty acids and derivates thereof (sometimes referred to as "albumin binders"), albumin, transferrin, elastin like peptides, isolated Fc domains, fragments of vWF, antibodies as well as fragments thereof comprising antigen binding sequences. Examples of suitable antibodies include antibodies, or fragments thereof, with the capacity to bind to blood components with a relatively long circulatory half life such as e.g. erythrocytes, platelets, fibrinogen etc. or binding to the vessel wall, e.g. collagen.

Without being bound by theory it is envisaged that the reason why it functions more efficiently to attach side groups to Factor VIII molecules with reduced vWF binding capacity rather than attaching side groups to Factor VIII molecules with normal vWF binding capacity is that the relative size of the side group is relatively small in the large Factor VIII/vWF complex. It is hypothesized that a relatively large side group functions more efficiently in shielding the free Factor VIII from clearance. It is further hypothesized that the half life of FVIII is related to that of vWF. FVIII molecules with reduced ability to bind vWF most likely have exposed clerance epitopes which would normally have been shielded by vWF. By attaching side groups it is thus hypothesized that this "clearance shielding" can be regained. In other cases, attachment of side groups such as e.g. antibody fragments may function by e.g. attaching the molecule to proteins, cells, or platelets having a relatively long circulatory half life. In connection with FVIII molecules according to the present invention, such molecules may furthermore be targeted to the platelets in a more efficient way if the FVIII part of the molecule has a reduced capacity to bind vWF.

Hydrophilic polymer: The modifying group/hydrophilic polymer according to the present invention is preferably non-naturally occurring. In one example, the "non-naturally occurring modifying group" is a polymeric modifying group, in which at least one polymeric moiety is non-naturally occurring. In another example, the non-naturally occurring modifying group is a modified carbohydrate. The locus of functionalization with the modifying group is selected such that it does not prevent the "modified sugar" from being added enzymatically to a polypeptide. "Modified sugar" also refers to any glycosyl mimetic moiety that is functionalized with a modifying group and which is a substrate for a natural or modified enzyme, such as a glycosyltransferase.

The polymeric modifying group added to a polypeptide can alter a property of such polypeptide, for example, its bioavailability, biological activity or its half-life in the body. Exemplary polymers according to the invention include water soluble polymers that can be linear or branched and can include one or more independently selected polymeric moieties, such as poly(alkylene glycol) and derivatives thereof. The polymeric modifying group according to the invention may include a water-soluble polymer, e.g. poly(ethylene glycol) and derivatived thereof (PEG, m-PEG), poly(propylene glycol) and derivatives thereof (PPG, m-PPG) and the like.

The term "water-soluble" refers to moieties that have some detectable degree of solubility in water. Methods to detect and/or quantify water solubility are well known in the art. Exemplary water-soluble polymers according to the invention include peptides, saccharides, poly(ethers), poly(amines), poly(carboxylic acids) and the like. Peptides can have mixed sequences and be composed of a single amino acid, e.g., poly(lysine). An exemplary polysaccharide is poly(sialic acid). An exemplary poly(ether) is poly(ethylene glycol), e.g., m-PEG. Poly(ethylene imine) is an exemplary polyamine, and poly(acrylic) acid is a representative poly(carboxylic acid).

The polymer backbone of the water-soluble polymer according to the invention can be poly(ethylene glycol) (i.e. PEG). The term PEG in connection with the present invention includes poly(ethylene glycol) in any of its forms, including alkoxy PEG, difunctional PEG, multiarmed PEG, forked PEG, branched PEG, pendent PEG (i.e. PEG or related polymers having one or more functional groups pendent to the polymer backbone), or PEG with degradable linkages therein.

The polymer backbone can be linear or branched. Branched polymer backbones are generally known in the art. Typically, a branched polymer has a central branch core moiety and a plurality of linear polymer chains linked to the central branch core. PEG is commonly used in branched forms that can be prepared by addition of ethylene oxide to various polyols, such as glycerol, pentaerythritol and sorbitol. The central branch moiety can also be derived from several amino acids, such as lysine or cysteine. In one example, the branched poly(ethylene glycol) can be represented in general form as R(-PEG-OH)m in which R represents the core moiety, such as glycerol or pentaerythritol, and m represents the number of arms. Multi-armed PEG molecules, such as those described in U.S. Patent No. 5,932,462, which is incorporated by reference herein in its entirety, can also be used as the polymer backbone.

Many other polymers are also suitable for the invention. Polymer backbones that are non-peptidic and water-soluble, are particularly useful in the invention. Examples of suitable polymers include, but are not limited to, other poly(alkylene glycols), such as poly(propylene glycol) ("PPG"), copolymers of ethylene glycol and propylene glycol and the like, poly(oxyethylated polyol), poly(olefmic alcohol), poly(vinylpyrrolidone), poly(hydroxypropylmethacrylamide), poly([alpha] -hydroxy acid), poly( vinyl alcohol), polyphosphazene, polyoxazoline, poly(N-acryloylmorpholine), such as described in U.S. Patent No. 5,629,384, which is incorporated by reference herein in its entirety, as well as copolymers, terpolymers, and mixtures thereof.

Although the molecular weight of each chain of the polymer backbone can vary, it is typically in the range of from about 100 Da to about 160,000 Da, such as e.g. from about 5,000 Da to about 100,000 Da. More specifically, the size of each conjugated hydrophilic polymer according to the present invention may vary from about 500 Da to about 80,000 Da, such as e.g. about 1000 Da to about 80,000 Da; about 2000 Da to about 70,000 Da; about 5000 to about 70,000 Da; about 5000 to about 60,000 Da; about 10,000 to about 70,000 Da; about 20,000 to about 60,000 Da; about 30,000 to about 60,000 Da; about 30,000 to about 50,000 Da; or about 30,000 to about 40,000 Da. It should be understood that these sizes represent estimates rather than exact measures. According to a preferred embodiment, the molecules according to the invention are conjugated with a heterogenous population of hydrophilic polymers, such as e.g. PEG of a size of e.g. 10,000, 40,000, or 80,000 Da +/- about 5000, about 4000, about 3000, about 2000, or about 1000 Da.

### Albumin binder conjugates/side groups

It is known that the in vivo properties of such proteins can be improved by the use of albumin binding side chains. Such side chains, or albumin binders, can be attached to the protein prior to administration and can, for example, stabilise the protein in vivo or improve or extend the in vivo half-life of the protein.

The albumin binder may thereby promote the circulation of the derivative with the blood stream. The albumin binder may have the effect of extending or protracting the time of action of the protein that it is bound to it, due to the fact that the complexes of the peptide derivative and albumin are only slowly disintegrated to release the active pharmaceutical ingredient. Thus, a preferred substituent, or side chain, as a whole may be referred to as an albumin binding moiety.

The albumin binder (albumin binding moiety) may comprise a portion which is particularly relevant for the albumin binding and thereby the protraction of circulation in the blood stream, which portion may accordingly be referred to as a protracting moiety. The protracting moiety is preferably at, or near, the opposite end of the albumin binding moiety as compared to its point of attachment to the peptide.

In a preferred embodiment, the albumin binder is, or comprises, a side chain that is capable of forming non-covalent complexes with albumin. The albumin binder may bind albumin non-covalently and/or reversibly. The albumin binder may bind albumin specifically. As is clear from the methods described below, the albumin binder may bind to cyclodextrin. The albumin binder may bind cyclodextrin non-covalently and/or reversibly. The albumin binder may bind cyclodextrin specifically.

The other portion of the albumin binding moiety, i.e. the portion in-between the protracting moiety and the point of attachment to the peptide, may be referred to as a linker moiety, linker, spacer, or the like. However, the presence of such a linker is optional, and hence the albumin binding moiety may be identical to the protracting moiety. In particular embodiments, the albumin binding moiety and/or the protracting moiety is lipophilic, and/or negatively charged at physiological pH (7.4).

The albumin binding moiety and/or the protracting moiety may be covalently attached to an amino group of the peptide by conjugation chemistry such as by alkylation, acylation, or amide formation; or to a hydroxyl group, such as by esterification, alkylation, oximation. In a preferred embodiment, an active ester of the albumin binding moiety and/or the protracting moiety is covalently linked to an amino group of a sialic acid residue or a sialic acid derivative, under formation of an amide bond (this process being referred to as acylation). Unless otherwise stated, when reference is made to an acylation of a protein, it is understood to be to an amino-group linked to a sialic acid residue on on glycoprotein.

For the present purposes, the terms "albumin binding moiety", "protracting moiety", and "linker" include the un-reacted as well as the reacted forms of these molecules. Whether or not one or the other form is meant is clear from the context in which the term is used. The albumin binding moiety may be, or may comprise a fatty acid or fatty diacid or a derivative or either thereof. The term "fatty acid" refers to aliphatic monocarboxylic acids having from 4 to 28 carbon atoms, such as 16 carbon atoms. It is preferably unbranched, and/or even numbered, and it may be saturated or unsaturated. The term "fatty diacid" refers to fatty acids as defined above but with an additional carboxylic acid group in the omega position. Thus, fatty diacids are dicarboxylic acids. The nomenclature is as is usual in the art, for example - COOH, as well as HOOC-, refers to carboxy; -C₆H₄- to phenylen; -CO-, as well as -OC-, to carbonyl (O=C<); and C₆H₅-O- to phenoxy. In a preferred embodiment the linker moiety, if present, has from 2 to 80 C-atoms, preferably from 5 to 70 C-atoms. In additional preferred embodiments, the linker moiety, if present, has from 4 to 20 hetero atoms, preferably from 2 to 40 hetero atoms, more preferably from 3 to 30 hetero atoms. Particularly preferred examples of hetero atoms are N-, and O-atoms. H-atoms are not hetero atoms.

In another embodiment, the linker comprises at least one OEG molecule, and/or at least one glutamic acid residue, or rather the corresponding radicals (OEG designates 8-amino-3,6-dioxaoctanic acid, i.e. this radical: -NH-(CH₂)₂-O-(CH₂)₂-O-CH₂-CO-). In one preferred embodiment, the linker moiety comprises a di-carboxyl residue linked to a sialic acid residue by an amide bond. In preferred examples, the di-carboxyl residue has from 2-30 C-atoms, preferably 4-20 C-atoms, more preferably 4-10 C-atoms. In additional preferred examples, the di-carboxyl residue has from 0-10 hetero-atoms, preferably 0-5 hetero-atoms.

In another preferred example, the linker moiety comprises a group containing both an amino and a distal carboxyl-group linked to a sialic acid residue by an amide bond through its distal carboxyl groups. In one preferred embodiment the this group is an OEG group.

The amino acid glutamic acid (Glu) comprises two carboxylic acid groups. Its gamma-carboxy group is preferably used for forming an amide bond with an amino group of a sialic acid residue or a sialic acid derivative, or with an amino group of an OEG molecule, if present, or with the amino group of another Glu residue, if present. The amino group of Glu in turn forms an amide bond with the carboxy group of the protracting moiety, or with the carboxy group of an OEG molecule, if present, or with the gamma-carboxy group of another Glu, if present. This way of inclusion of Glu is occasionally briefly referred to as "gamma-Glu".

N- and O-linked oligosaccharides: Both N-glycans and O-glycans are attached to proteins by the cells producing the protein. The cellular N-glycosylation machinery recognizes and glycosylates N-glycosylation signals (N-X-S/T motifs) in the amino acid chain, as the nascent protein is translocated from the ribosome to the endoplasmic reticulum. Likewise, O-glycans are attached to specific O-glycosylation sites in the amino acid chain, but the motifs triggering O-glycosylation are much more heterogenous than the N-glycosylation signals, and our ability to predict O-glycosylation sites in amino acid sequences is still inadequate. Methods of conjugating polypeptides with various polymeric side groups is described e.g. in WO0331464.

"Glycoprotein IIb/IIIa" or "GPIIb/IIIa" is an integrin found on platelets. It is a receptor for fibrinogen and aids in platelet activation. The complex is formed via calcium-dependent association of GPIIb and GPIIIa, a required step in normal platelet aggregation and endothelial adherence. Platelet activation leads to a conformational change in platelet GPIIb/IIIa receptors that induces binding to fibrinogen. A non-inhibitory GPIIb/IIIa antibody, that does not inhibit platelet aggregation, according to the invention should not be directed to the fibrinogen recognizing part which comprises the ligand binding pocket in the globular head of the integrin. Which parts of the GPIIb/IIIa that are directly involved in the RGD recognition and hence fibrinogen binding is not completely understood. However, in GPIIIa amino acids 109-171 are described to be engaged in the interaction (D'Souza et al., Science 242; 91-93, 1988) whereas in GPIIb the amino acids of importance are thought to comprise amino acids 294-314 (D'Souza et al., JBC 265:6 ;3440-46, 1990) or amino acids 145-224 (Kamata et al., JBC 271:18610-15, 1996, Tozer et al., Blood 93:918-24 1999, Basani et al., Blood 95.180-88, 2000). The non-inhibitory AP3 antibody according to the present invention is thought to have the capacity to bind to an epitope situated within amino acids 348-421 of GPIIIa and consequently does not interfere with fibrinogen binding (Kouns et al. Blood 15;78(12):3215-23, 1991).

The term "GPIIb/IIIa antibody", "targeting factor" as used herein, is intended to refer to immunoglobulin molecules and fragments thereof that have the ability to specifically bind to the GPIIb/IIIa integrin. Said GPIIb/IIIa antibodies furthermore bind to the GPIIb/IIIa receptor in an essentially non-inhibitory fashion, meaning that the ability of the platelets to bind fibrinogen and to aggregate to form the primary clot is not significantly decreased (such as e.g. less than 20%, 15%, 10%, 5%, or 1% decreased compared to addition of a reference antibody) upon binding of the GPIIb/IIIa antibody. Platelet aggregation was measured by monitoring the change in light transmission through a suspension of isolated platelets. This method was first described essentially by Gustav von Born in the 1960s (Born, Nature 1962) and is today one of the most used methods for evaluation of platelet function. In brief, the method measures the capability of light to transverse through a suspension of platelets. This suspension of platelets might either be platelet rich plasma or isolated platelets. The sample is illuminated and the amount of light going through the sample is measured. Upon activation the GPIIb/IIIa change its conformation to a fibrinogen high-binding state and in the presence of fibrinogen the platelets will start to form aggregates. This is registered as an increase in light transmission since more light will go through a sample with few large aggregates than many single platelets.

Full-length antibodies comprise four polypeptide chains, two heavy (H) chains and two light (L) chains interconnected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Thus, within the definition of an antibody is also one or more fragments of an antibody that retain the ability to specifically bind to GPIIb/IIIa.

It has been shown that the antigen-binding function of an antibody can be performed by fragments of a full-length antibody. Examples of binding fragments encompassed within the term "antibody" include (i) a Fab fragment, a monovalent fragment consisting of the VL, VH, CL and CH I domains; (ii) F(ab)2 and F(ab')2 fragments, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546 ), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426: and Huston et al. (1988) Proc. Natl. Acad. Sci. USA 85:5879-5883). Such single chain antibodies are also intended to be encompassed within the term "antibody".

Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger, P., et al. (1993) Proc. Natl. Acad. Sci. USA 90:6444-6448; Poljak, R. J., et al. (1994) Structure 2:1121-1123). It is understood that protein X may have one or more antigenic determinants comprising (1) peptide antigenic determinants which consist of single peptide chains within protein X, (2) conformational antigenic determinants which consist of more than one spatially contiguous peptide chains whose respective amino acid sequences are located disjointedly along the protein X polypeptide sequence; and (3) post-translational antigenic determinants which consist, either in whole or part, of molecular structures covalently attached to protein X after translation, such as carbohydrate groups, or the like.

In another preferred embodiment, the non-inhibitory GPIIb/IIIa antibody is an AP3, or a SZ22 antibody or fragment thereof.

The terms "human antibody", "human antibodies", as used herein, means antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo), for example in the CDRs and in particular CDR3.

The term "epitope" as used herein means any antigenic determinant on an antigen to which the antibody binds. Epitopic determinants usually consist of chemically active surface groupings of molecules such as amino acids or sugar side chains and usually have spe-cific three dimensional structural characteristics, as well as specific charge characteristics.

In general, the GPIIb/IIIa antibody can be bound to the FVIII or FVIII analog amino acid sequence in any suitable manner. Thus, for example, the antibody can be expressed as part of a "fusion protein" with the FVIII or FVIII analog amino acid sequence or separately attached via chemical and/or enzymatic methods. In this latter respect, the antibody can be bound to the FVIII or FVIII analog sequence by a sortase- or carboxypeptidase-mediated transacylation (see, e.g., Stennicke, International Patent Publication W02006/013202 A2; Hoess et al., International Patent Application WO 2006/015879 A1; Zhang et al., Protein Exp. Purif. (2004) 36, 292-299 for description of relevant methods and principles).

In one aspect, the GPIIb/IIIa antibody is bound to the FVIII or FVIII analog amino acid sequence (i.e., is "engineered") such that it can be cleaved from the FVIII or FVIII analog sequence under, e.g. by insertion into the B-domain.

In one aspect, the invention provides FVIII molecules that comprise two or more GPIIb/IIIa antibodies or that comprise a single antibody that specifically binds to two or more targets. For example, in one aspect, the invention provides FVIII molecules that comprise a bispecific or multispecific antibody or antibody fragment. In one aspect, the antibody is a multispecific antibody molecule (full-length antibody or antibody fragment) in which one target of the antibody (such as one "arm" of a bispecific antibody) specifically binds to a portion of the B-domain that is included in the FVIII molecule and another portion binds to a target associated with platelets and/or megakaryocytes. Examples of B-domain-specific antibodies are known in the art (see, e.g., Lavigne-Lissalde et al., THROMBOSIS AND HAEMOSTASIS, Volume: 98, Issue: 1, Pages: 138-147, 2007. From the aspect of the foregoing paragraph it should be clear that a GPIIb/IIIa antibody can be associated with a FVIII or FVIII analog amino acid sequence-containing portion of the FVIII molecule by other suitable means other than covalent bonding including by non-covalent protein-protein, protein-moiety, or moiety-moiety interactions. In other aspects, as described elsewhere herein, a/the GPIIb/IIIa antibody is bound to FVIII or FVIII analog amino acid sequence by at least one covalent bond. In one aspect, the GPIIb/IIIa antibody is bound to the FVIII or FVIII analog amino acid sequence via an amide bond (e.g., in the case of a "fusion protein" of a FVIII amino acid sequence and the GPIIb/IIIa antibody). It also should be clear that the bond to the amino acid sequence can be direct or indirect. For example, the bond can comprise a suitable linker, which may be a chemical moiety (examples of which are described in, e.g., US Patent Publication No. 20030236190) or an amino acid sequence, such as a flexible Gly(X)Ser(Y) linker. In another aspect, the linkage is through a glycan that is associated with an amino acid of the amino acid sequence. Even such glycan linkages can comprise additional linkage elements, e.g., where the glycan itself is derivatized with an element that binds to the targeting molecule.

It will be more generally appreciated that the various aspects of the invention can be combined in any suitable manner. Thus, for example, in one facet the invention provides a FVIII molecule that comprises a bispecific GPIIb/IIIa antibody molecule portion which binds to the B-domain of the FVIII molecule and a cleavage site (which may be the natural thrombin cleavage site) that permits the B-domain to be removed from the FVIII molecule, releasing the targeting antibody molecule portion from the active FVIII molecule.

The biologic activity of an FVIII molecule of the invention can also be readily tested using routine and known methods. For example, FVIII molecule can be incubated with resting platelets, the bound platelets by purified, by, e.g., gel-filtration or differential centrifugation, and the FVIII molecule activated with thrombin; upon which catalytic efficiency and demonstration of activity can be visualized by standard chromogenic assays (e.g. COATEST) or a clot assay. Prolongation of *in vivo* half-life similarly can be assessed using standard methods. For example, hemophilia A mice (FVIII^{null/null}) can be monitored for specific FVIII activity by several different methods, including, clot assays (FVIII^{null/null} mice) or FX activity assays. It should be noted that the method of this invention, while described with reference to human Factor VIII, can be applied to Factor VIII proteins from other mammals, such as dogs, mice, etc. Such proteins are known in the art and the application of the methods of the invention to such homologs of FVIII and other species requires no more than routine experimentation.

The FVIII molecules of the invention can be provided in a dosage that is similar to or somewhat less than the amount of FVIII typically administered to a subject or patient for the relevant desired physiologic effect. In general, FVIII formulations known in the art also can be used in the preparation of pharmaceutical compositions comprising the FVIII molecules of the present invention.

The invention also provides a nucleic acid that encodes a fusion protein FVIII molecule according to the present invention. The nucleic acid can be any suitable type of nucleic acid that encodes such a molecule (e.g., a ssDNA, dsDNA, or an RNA, which may comprise various suitable modifications known to those of skill in the art such as a phosphothioate backbone). A nucleic acid may further include expression elements, such as promoters, enhancers, polyA sequences, and the like. Such a nucleic acid can also be incorporated into a suitable vector comprising even further elements, such as resistance genes and the like, which may be any suitable type of vector (e.g., a viral vector, such as an adeno-viral, pox viral, or adeno-associated-viral vector or a plasmid vector). Such nucleic acids and vectors can further be incorporated into suitable host cells for expression or maintenance, which typically will be mammalian cells, such as COS or HEK cells. In this and other respects, the invention provides a method of producing FVIII molecules of the invention and potential FVIII molecules of the invention.

In a preferred embodiment, the FVIII molecule according to the invention furthermore has modulated, preferably reduced vWF binding capacity, preferably by comprising an amino acid substitution in position 1680, such as e.g. one of the following substitutions: Y1680F, Y1680R, Y1680N, or Y1680C. In other preferred embodiments, the FVIII molecule according to the invention comprise amino acid mutations that may result in e.g. modulated binding to e.g. LPR, various receptors, other coagulation factors, cell surfaces, etc.

In yet another embodiment, the Factor VIII molecule according to the invention is covalently attached to the non-inhibitory GPIIb/IIIa antibody via a linker. Preferably, the linker comprises an N-linked or an O-linked glycan on the FVIII molecule. According to a particularly preferred embodiment, the glycan is placed in the B domain of the Factor VIII molecule according to the invention. The B-domain is preferably a truncated B-domain. In a particular preferred embodiment the non-inhibitory GPIIb/IIIa antibody and FVIII are linked through a linker connecting the O-linked glycan of the B-domain of FVIII and an N-linked glycan of the non-inhibitory GPIIb/IIIa antibody. In a further preferred embodiment, the non-inhibitory GPIIb/IIIa antibody is a full length antibody. In a preffered embodiment this full length antibody is an AP3-antibody, SEQ 1 and 2, or SEQ 1 and 3.

In a further preferred embodiment the N-linked glycan of the non-inhibitory GPIIb/IIIa antibody is part of the constant region of the antibody. In another preferred embodiment the N-linked glycan of the non-inhibitory GPIIb/IIIa antibody is part of the light chain of the antibody. The linker may comprise a polyethylene glycol polymer.

In a further preferred embodiment the non-inhibitory GPIIb/IIIa antibody and FVIII are linked through a linker connecting an intact O-linked glycan of the B-domain of FVIII and an intact N-linked glycan of the non-inhibitory GPIIb/IIIa antibody.

In a different preferred embodiment the non-inhibitory GPIIb/IIIa antibody and FVIII are linked through a linker connecting an O-linked glycan of the B-domain of FVIII nd the N-terminus of the non-inhibitory GPIIb/IIIa antibody. In a preferred embodiment, the non-inhibitory GPIIb/IIIa antibody is an AP3 Fab-fragment.

In another preferred embodiment the non-inhibitory GPIIb/IIIa antibody and FVIII are linked through a linker connecting an O-linked glycan of the B-domain of FVIII and a Cys residue of the non-inhibitory GPIIb/IIIa antibody. In a preferred embodiment, the non-inhibitory GPIIb/IIIa antibody is an AP3 ScFv.

In yet another preferred embodiment the non-inhibitory GPIIb/IIIa antibody and FVIII are linked through a linker connecting an O-linked glycan of the B-domain of FVIII and one or more Lys residues of the non-inhibitory GPIIb/IIIa antibody. In a preferred embodiment, the non-inhibitory GPIIb/IIIa antibody is an AP3 full length antibody (SEQ 1 and 2 or SEQ 1 and 3).

In yet another preferred embodiment, the non-inhibitory GPIIb/IIIa antibody is fused to the B-domain of a B domain truncated Factor VIII molecule according to the invention. The present invention therefore also comprises nucleic acids and vectors encoding such molecules, as well as host cells comprising such nucleic acids and/or vectors.

In yet another preferred embodiment, the A3 domain of the VIII molecule according to the invention is replaced with the non-inhibitory GPIIb/IIIa antibody.

According to a particularly preferred embodiment, the FVIII molecule according to the invention comprises the sequence as set forth in SEQ ID NO 3, and the linker comprises an O-linked glycan placed in the B domain.

Another aspect of the invention relates to a method of producing a FVIII molecule according to the invention, said method comprising expressing a nucleic acid according to the invention. Alternatively, the method according to the invention comprises conjugation of the FVIII molecule with the GPIIb/IIIa antibody.

Yet another aspect relates to a pharmaceutical composition comprising a FVIII molecule according to the invention.

In yet another aspect, the invention relates to use of a FVIII molecule according to the invention for producing a medicament for treatment of haemophilia A.

In a final aspect, the present invention relates to a method of treating hemophilia A in a mammalian host comprising administering to the host a therapeutically effective amount of a molecule according to the invention

### Construction

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law), regardless of any separately provided incorporation of particular documents made elsewhere herein.

The use of the terms "a" and "an" and "the" and similar referents in the context of describing the invention are to be construed to cover both the singular and the plural, unless otherwise indicated herein or clearly contradicted by context. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or embodiment of the invention using terms such as "comprising", "having," "including," or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

All headings and sub-headings are used herein for convenience only and should not be construed as limiting the invention in any way. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents. This invention includes all modifications and equivalents of the subject matter recited in the claims and/or aspects included herein as permitted by applicable law.

### EXAMPLES

### EXAMPLE 1

### FVIII frameworks and fusion partners

The fusion proteins of the present invention consist of a FVIII protein (FVIII part) joined to a polypeptide (fusion partner) from another protein.

The FVIII part of the fusion protein can be any protein with FVIII activity. The FVIII part can be a B domain-deleted/truncated (BDD) FVIII protein, in which parts of the FVIII B domain has been removed from the protein. F8-500 is a BDD human FVIII protein. Starting at the N-terminus, F8-500 consists of FVIIIs signal peptide (amino acid -19 to -1) followed by FVIII HC without the B domain (amino acid 1-740), a 21 amino acid linker (SFSQNSRHPSQNPPVLKRHQR) (SEQ ID NO 4), and FVIII LC (amino acid 1649-2332 of wild-type human FVIII. The sequence of the 21 amino acid linker is derived from the B domain of FVIII and consists of amino acid 741-750 and 1638-1648 of full-length wild-type human FVIII.

F8-500-Δa3 consists of F8-500 without the a3 region. In F8-500-Δa3 amino acid 1647-1687 of wild-type human FVIII is eliminated from F8-500. Thereby, the furin site at amino acid 1645-1648 is destroyed. A combined furin and thrombin site is, however, created by the R1645-H1646-P1688-R1689 amino acid stretch in F8-500-Δa3. The a3 region is important for binding of FVIII to vWF and therefore, the affinity of F8-500-Δa3 for vWF is reduced compared to wild-type FVIII.

F8-500-His consists of F8-500 with a His tag inserted in the linker of F8-500. Thus the linker sequence of F8-500-His is SFSQNSRHPSHHHHHHSQNPPVLKRHQR (SEQ ID NO 5).

F8-500-Δa3-His consists of F8-500 without the a3 region but with a His tag inserted in the linker of F8-500. Thus, in F8-500-Δa3-His amino acid 1647-1687 of wild-type human FVIII has been eliminated from F8-500 and the linker sequence is SFSQNSRHPSHHHHHHSQNPPVLKRHQR (SEQ ID NO 6).

F8-500-Y1680F and F8-500-Y1680C consist of F8-500 in which amino acid 1680 of full-length wild-type human FVIII has been changed from tyrosine to phenylalanine and cysteine, respectively. Both these amino acid replacements reduce the affinity of FVIII to vWF factor. Furthermore, the Y1680C amino acid replacement introduces a free cysteine that can be used as a handle for conjugating protracting moieties to the fusion protein.

The fusion partner can be joined to several positions on the FVIII part of the fusion protein. Non-limiting examples of positions on FVIII for joining to the fusion partner are in the B domain or the B-domain-derived linker between the FVIII HC and LC, at the position of a3, and at the C-terminus of FVIII LC.

### EXAMPLE 2

### Construction of expression vectors encoding FVIII frameworks and fusion proteins

The fusions between FVIII and fusions partners all involves PCR for amplifying the fusion partner. Restriction sites are added to the ends of the PCR primers used. Restriction enzymes are used for cloning of fusion partner cDNA or synthetis DNA into FVIII cDNA.

Fusions in the B-domain of F8-500 take place between aa750 and aa1638. Restriction sites AvrII, NruI, AgeI and MIuI within or flanking the B-domain are used for insertion of the fusion partner encoding DNA.

For fusions at the carboxy terminus of FVIII light chain, the F8-500 coding construct is modified. The internal BamHI site (aa 604-606) is eliminated by site-directed mutagenesis and DNA encoding the flexible (GGGS)₆ linker is inserted 3' to the coding region. A new BamHI site is introduced in the 3' end of the linker-coding DNA in order to ease cloning of C-terminal fusion partners between BamHI and NotI sites. Subsequently, fusion partner DNA is inserted.

For insertion of the fusion partner coding DNA at a3 positions thus replacing a3 with the fusion partner in the encoded protein, the SacII restriction site is introduced 3' to the coding region of a3. Thus, fusion partner coding DNA can be introduced by insertion between the AgeI and SacII sites or between the AvrII and SacII sites.

### EXAMPLE 3

### Full length AP3 mIgG1 ab

The variable regions of the heavy and light chain of the anti-GPIIa/IIIB antibody, AP3 were amplified from RNA isolated from hybridoma cells expressing the AP3 antibody, using the SMART™ RACE cDNA Amplification Kit (Clontech, Ca, USA). The primers used for the amplification of the variable regions of the two AP3 chains were:

### Heavy chain:

Universal Primer Mix A (Clontech, CA):
   Long (0.4 µM):
      5'-ctaatacgactcactatagggcAAGCAGTGGTATCACGCAGAGT-3' (SEQ ID NO 7)
   Short (2 µM):
      5'-ctaatacgactcactatagggc-3' (SEQ ID NO 8)
   Primer 69 (10 µM)
      5'-gctctagactaacactcattcctgttgaagctcttg-3' (SEQ ID NO 9)

### Light chain

Universal Primer Mix A (Clontech:
   Long (0.4 µM):
      5'-ctaatacgactcactatagggcAAGCAGTGGTATCACGCAGAGT-3' (SEQ ID NO 10)
   Short (2 µM):
      5'-ctaatacgactcactatagggc-3' (SEQ ID NO 11)
   Primer 312 (10 µM)
      5'-gtctaccacaacacacgtgac-3' (SEQ ID NO 12)

The variable regions were cloned into the pCR4 vector using the Zero Blunt® TOPO® PCR Cloning Kit for Sequencing (Cat. No. K287520, Invitrogen, CA, USA). The heavy chain variable region was subsequently subcloned into the Eco-RI/BamHI sites of a pTT5 based expression vector containing a murine IgG1 framework to generate an AP3 mIgG1 heavy chain. The amino acids sequence of the AP3 mIgG1 heavy chain is shown below. The AP3 IgK light chain was amplified from the pCR4 vector using the primer AP-3 LC kl1 Sense (5'-GACTTTTTG-TATGAATTCCTCACCATGAGGTGC-3'; SEQ ID NO 13) and a M13 Reverse primer. The PCR fragment was subcloned into the *EcoRI* site of an empty pTT5 based expression vector. The amino acids sequence of the AP3 mIgK light chain protein is shown below. The two vectors encoding the full length AP3 ab were transiently expressed in Hek293 6E cells. Transfections were carried out using 293fectin as transfection agent (cat. no 12347-019, Invitrogen, Ca, USA) following the instructions supplied by the manufacturer. Transfections were left for 5 days before harvest.

A potential problematic cystein at position 39 (position 34 according to the Kabat numbering system) was identified in the Light chain of the AP3 antibody. The cystein residue was successfully mutated to a serine through site directed mutagenesis using the QuikChange Site Directed Mutagenesis Kit (Cat no 200518, Stratagene, CA, USA) and the following two primers:
AP3 LC C39S S
   5'-caacacttacttgtcctggttcctgcag-3' (SEQ ID NO 14)
AP3 LC C39S AS
   5'-ctgcaggaaccaggacaagtaagtgttg-3' (SEQ ID NO 15)

The sequence of the resulting protein is shown below. A full length AP3 mIgG1 ab containing the C39S mutation could be expressed by combining the two vector constructs expressing SEQ ID NO 16 and SEQ ID NO 18.

### EXAMPLE 4

### Purification of AP3 FL mIgG1 wt and AP3 FL mIgG1 wt HC LC C39S.

Purification of the AP3 FL mIgG1 wt and AP3 FL mIgG1 wt HC LC C39S proteins (EXAMPLE 3) were conducted by a 1-step process composed of affinity chromatography using a Protein A MabSelect SuRe resin (GE Healthcare, cat. no. 17-5438-01). The purification was conducted using an ÄktaExplorer chromatography system (GE Healthcare, cat. no. 18-1112-41). The buffer systems used for the purification step was an equilibration buffer composed of Tris, 3 M NaCl, pH 8.5 and an elution buffer composed of 10 mM Formic acid pH 3.5. The supernatant was adjusted to 3 M NaCl and pH 8.5 prior to application to the MabSelect SuRe column. The column was washed with 15 column volumes of equilibration buffer and the protein was eluted isocratically in approx. 1 column volume of elution buffer. The AP3 FL mIgG1 1 wt HC LC C39S was analyzed using SDS-PAGE/Coomassie and SEC-HPLC, showing that a pure and homogenous protein of approx. 150 kDa (approx. 50 kDa heavy chain component and approx. 25 kDa light chain component) was obtained from the purification with a purity of >98% as measured by SEC-HPLC. To measure the final protein concentration, a NanoDrop spectrophotometer (Thermo Scientific) was used together with an extinction coefficient of 1.56.

Characterisation of the antibody was performed by LC-MS analysis of the reduced intact mAb. The heavy chain was shown to contain G0F and G1F glycans. The light chain was shown to be glycosylated with biantennary glycans with one sialic acid (G2FS) as the major structure. Treatment with sialidase gave the expected shift in mass values of 291 amu confirming the presence of a sialic acid

Binding of the full length AP3 FL mIgG1 wt antibody to resting platelets was confirmed by FACS analysis.
SEQ ID **NO** 16: AP3 mIgG1 HC
SEQ ID **NO** 17: AP3 mIgK LC
SEQ ID **NO** 18: AP3 IgK LC C39S

### EXAMPLE 5

### AP3 scFV constructs

Based on the DNA sequences encoding the variable regions of the light and heavy chains of the AP3 antibody, two single chain antibody formats of AP3 ab (AP3 LC-HC scFV and AP3 HC-LC scFV) were ordered from MWG biotech, Germany. The amino acids sequence of the two scFV protein is indicated in SEQ ID NO 21 and SEQ ID NO 22. The construction included a 15 aa (G₄S)₃) linker region introduced between the two variable regions in order to allow correct pairing of the V_{H} and the V_{L} fragments. A Flag tag was included in the C-terminus of both proteins for purification purposes.

The genes encoding the two single chain formats of the AP3 ab were subsequently subcloned into the HindIII site of a pTT5 based expression vector. The two construct encoding the two single-chain AP3 antibodies were transiently expressed in Hek293 6E cells, using 293fectin as transfection agent (cat. no 12347-019, Invitrogen, Ca, USA) following the instructions supplied by the manufacturer. Transfections were left for 5 days before harvest.

The binding of the two single-chain AP3 antibodies AP3-LC-HC scFV-FLAG, AP3-HC-LC scFV-FLAG to GPIIa/IIIB of resting platelets was confirmed by FACS analysis.

The potential problematic cystein at position 39 (position 34 according to the Kabat numbering system) identified in the Light chain of the AP3 antibody was mutated to a serine in the AP3 LC-HC scFV construct. This was performed using the QuikChange Site Directed Mutagenesis Kit (Cat no 200518, Stratagene, CA, USA) and following the instructions supplied by the manufacturer and using the following two primers
AP3 LC C39S S
   5'-caacacttacttgtcctggttcctgcag-3' (SEQ ID NO 19)
AP3 LC C39S AS
   5'-ctgcaggaaccaggacaagtaagtgttg-3' (SEQ ID NO 20)

The sequence of the resulting AP3 LC-HC scFV-FLAG C39S protein is shown in SEQ ID NO 23.
SEQ ID **NO** 21: AP3-LC-HC scFV-FLAG
SEQ ID **NO** 22: AP3-HC-LC **scFV-FLAG**
SEQ ID **NO** 23: AP3-LC-HC scFV-FLAG C39S

Binding of the single chain antibody AP3 LC-HC scFV-FLAG C39S to resting platelets was confirmed by FACS analysis.

### EXAMPLE 6

### AP3 scFV-Cys constructs

In order to facilitate the conjugation of AP3 LC-HC scFV C39S to FVIII, a free Cys residue was introduced in the scFV by site directed mutagenesis. Two constructs were made. In one construct an unpaired Cystein residue was introduced in the C-terminus of the **AP3 LC-HC scFV** protein by site directed mutagenesis through use of the QuikChange Site Directed Mutagenesis Kit (Cat no 200518, Stratagene, CA, USA), following the instructions supplied by the manufacturer and using the following two primers:
AP3 scFV Cys S
   5'-cgacgacgacaagtgctgaaagcttcgtacg-3' (SEQ ID NO 24)
AP3 scFV Cys AS
   5'-cgtacgaagctttcagcacttgtcgtcgtcg-3' (SEQ ID NO 25)

In another construct an unpaired Cystein was introduced by mutating a serine at position 248 in AP3 LC-HC scFV to cystein. The potential problematic cystein at position 39 (position 34 according to the Kabat numbering system) identified in the Light chain of the AP3 antibody was subsequently mutated to a serine using the QuikChange Site Directed Mutagenesis Kit (Cat no 200518, Stratagene, CA, USA), following the instructions supplied by the manufacturer and using the following two primer sets:

### Primer set A

AP3 scFV LC-HC S248C S
   5'-gtgaccgtgagctgcgactacaaggac-3' (SEQ ID NO 26)
AP3 scFV LC-HC S248C AS
   5'-gtccttgtagtcgcagctcacggtcac-3' (SEQ ID NO 27)

### Primer set B

AP3 LC C39S S
   5'-caacacttacttgtcctggttcctgcag-3' (SEQ ID NO 28)
AP3 LC C39S AS
   5'-ctgcaggaaccaggacaagtaagtgttg-3' (SEQ ID NO 29)

All AP3 scFV constructs were transiently expressed in Hek293 6E cells. Transfections were carried out using 293fectin as transfection agent (cat. no 12347-019, Invitrogen, Ca, USA) following the instructions supplied by the manufacturer. Transfections were left for 5 days before harvest.

All scFV fragments were purified according to the following procedure:

### EXAMPLE 7

### Purification and characterization of AP3 LC-HC scFV proteins.

Purification of AP3 LC-HC scFV proteins (EXAMPLES 5 and 6) were conducted using a 2-step process composed of affinity chromatography using an anti-FLAG M2 affinity gel (Sigma, cat. no. A2220) followed by a Superdex 75pg gelfiltration column to remove aggregates and other high-Mw contaminates if these were observed (GE Healthcare, cat.no. 17-1068-01). The purification was conducted using an ÄktaExplorer chromatography system (GE Healthcare, cat. no. 18-1112-41). The buffer systems used for the first purification step was an equilibration buffer composed of 20 mM Hepes, 150 mM NaCl, 0.01% Tween-80 (v/v), pH 7.5 and an elution buffer composed of 100 mM Glycine pH 3.5/NaOH. The supernatant was either first adjusted to pH 6.7 with 0.5 M Hepes pH 10.5 or applied directly onto a pre-equilibrated anti-FLAG M2 affinity column. The column was washed with 10 column volumes of equilibration buffer and the protein was eluted isocratically in approx. 2 column volumes of elution buffer. The eluted protein was diluted 1:1 in 100 mM Hepes, 150 mM NaCl, pH 7.5 and analyzed using SDS-PAGE/Coomassie and SEC-HPLC. If a pure (>75%) and homogenous protein of approx. 28 kDa was obtained from the first purification step, no further purification was conducted. If not, then the second gelfiltration step was performed using 20mM Tris, 1M NaCl, pH 7.5. Between 2-3.5% load was applied and the fractions containing the eluted protein was analyzed using SDS-PAGE/Coomassie and SEC-HPLC. Based on the analyses, a pool was prepared containing a pure (>80%) and homogenous protein. To measure the final protein concentration, a NanoDrop spectrophotometer (Thermo Scientific) was used together with an extinction coefficient of 1.79.
SEQ ID **NO** 30: AP3 LC-HC **scFV** INS257C FLAG
SEQ ID **NO** 31: AP3 LC-HC scFV C39S **S248C** FLAG

### EXAMPLE 8

### AP3 Fab construct

A truncated version of AP3 heavy chain was generated by introduction of a stop codon in the construct encoding SEQ ID NO 16 using the QuikChange Site Directed Mutagenesis Kit (Cat no 200518, Stratagene, CA, USA) and the following two primers:
JP433 AP3 HC Fab S
   Cagggattgtggttgaaagccttgcatatg (SEQ ID NO 32)
JP434 AP3 HC Fab S
   Catatgcaaggctttcaaccacaatccctg (SEQ ID NO 33)

The sequence of the resulting protein is shown in SEQ 21.

A functional Fab fragment of the AP3 antibody were expressed by combining the two constructs expressing SEQ ID NO 18 and SEQ ID NO 34. The two chains were transiently expressed in Hek293 6E cells. Transfections were carried out using 293fectin as transfection agent (cat. no 12347-019, Invitrogen, Ca, USA) following the instructions supplied by the manufacturer. Transfections were left for 5 days before harvest.

### Purification and characterization of AP3 Fab LC C39S protein.

Purification of the AP3 Fab LC C39S protein was conducted using a 2-step process composed of a cation-exchange using a Source 30S (GE Healhcare, cat. no. 17-1273-01) followed by a Superdex 75pg gelfiltration column (GE Healthcare, cat.no. 17-1068-01). The purification was conducted using an ÄktaExplorer chromatography system (GE Healthcare, cat. no. 17-1068-01). The buffer systems used for the first purification step was an equilibration buffer composed of 10 mM Na-acetate pH 5.0 and an elution buffer composed of 10 mM Na-acetate, 1 M NaCl pH 5.0. The harvest was adjusted to < 3 mS/cm with the addition of milliQ prior to pH adjustment to pH 5.0 with 0.5 M HCl and applied to the pre-equilibrated Source 30S column. The column was washed with 15 column volumes of equilibration buffer. The protein was eluted by a linear gradient of equlibration buffer and elution buffer over 20 column volumes. The protein eluted in approximately 8 column volumes. The second gelfiltration purification step was performed using 20 mM Na-phosphate, 150 mM NaCl, pH 7.2. Between 2-3.5% load was applied and the protein was collected in 5-7% of a column volume. The AP3 Fab LC C39S protein was analyzed using SDS-PAGE/Coomassie and SEC-HPLC, showing that a pure and homogenous protein of approx. 50 kDa was obtained from the purification with an approximate purity of 91.9% as measured by SEC-HPLC. To measure the final protein concentration, a NanoDrop spectrophotometer (Thermo Scientific) was used together with an extinction coefficient of 1.67.
**SEQ ID NO 34: AP3 Fab HC**

### EXAMPLE 9

### FVIII- AP3 scFV fusions

AP3 LC-HC scFV or AP3 HC-LC scFV was fused to a B-domain deleted and a3-domain deleted FVIII variant using the AgeI and SacII restriction enzymes. The B-domain deleted and a3-domain deleted FVIII lacks aa 751-1637 and aa 1649-1685. The AP3 LC-HC scFV or AP3 HC-LC scFV was inserted between aa R1648 and Q1686. The AP3 coding sequence was amplified by PCR using primers harboring sites recognizable for the respective restriction enzymes, AgeI and SacII. Partial restriction digestion of PCR products were performed as the DNA has an endogenous AgeI site.

As the furin site (RHQR) is situated N-terminal the AP3 scFV, the AP3 scFV will after processing constitute the N-terminal of FVIII light chain. The thrombin site R1688-R1689 is also conserved and upon thrombin activation of these FVIII variants the AP3 scFVs is deliberated from FVIII.

### Primers:

AP3-HC-LC-Da3-AgeI
   Aacccaccggtcttgaaacgccatcaacggcaggtccagctgcagcagagc (SEQ ID NO 35)
AP3-HC-LC-Da3-SacII
   Gaaagctccgcgggctctgccgcttgatttccagcttgg (SEQ ID NO 36)
AP3-LC-HC-Da3-AgeI
   Aacccaccggtcttgaaacgccatcaacgggacatcgtgatgacccaggct (SEQ ID NO 37)
AP3-LC-HC-Da3-SacII
   Gaaagctccgcgggctctggctgctcacggtcacggagg (SEQ ID NO 38)

### EXAMPLE 10

### Transient expression of FVIII frameworks and fusion proteins

HKB11 cells at a density of 0.9 - 1.1 x 10⁶ are transfected with a complex of plasmid 0.7 mg/l and the transfection agent, 293Fectin (Invitrogen) 1.4 ml/l. The transfection complex is prepared by diluting the plasmid and the transfection separately in OPTIMEM (Invitrogen), mixing of the two solutions, and incubation of the mixture at room temperature for 20 minutes. The complex mixture is added to the cell suspension and the suspension is incubated in shaker incubator for 5 days at 36.5 °C and 5 % CO₂. The cell culture harvest is filtered on a 0.22 µm membrane filter.

### EXAMPLE 11

### General procedure for purification of FVIII frameworks and fusion proteins

A column was packed with the resin VIIISelect (GE Healthcare), with the dimensions 1.6cm in diameter and 4cm in bed height giving 8mL, and was equilibrated with 20mM Imidazol + 10mM CaCl₂ + 0.01% Tween80+250mM NaCl, pH7.3 at 500cm/h. The culture filtrate prepared as described in Example 3 was applied to the column, and the column was subsequently washed with first equilibration buffer and then 20mM Imidazol + 10mM CaCl₂ + 0.01% Tween80+1.5M NaCl, pH7.3. The bound FVIII was eluted isocratic at 90cm/h with 20mM Imidazol + 10mM CaCl₂ + 0.01% Tween80 + 2.5 M NaCl + 6.5M Propylenglycol, pH7.3. The fractions containing FVIII were pooled and diluted 1:10 with 20mM Imidazol + 10mM CaCl₂ + 0.01% Tween80, pH7.3 and applied to a column packed with F25-Sepharose (Thim *et al*., Haemophilia, 2009). The column dimension was 1.6cm in diameter and 2cm in bed height giving 4mL in column volume. The column was equilibrated at 180cm/h with 20mM Imidazol + 10mM CaCl₂ + 0.01% Tween80 + 150mM NaCl + 1M Glycerol, pH7.3 prior to application. After application the column was washed first with equilibration buffer and then 20mM Imidazol + 10mM CaCl₂ + 0.01% Tween80 + 650mM NaCl, pH7.3. The bound FVIII was isocratic eluted with 20mM Imidazol + 10mM CaCl₂ + 0.01% Tween80 + 2.5M NaCl + 50%(v/v) Ethylenglycol, pH7.3 at 30cm/h. The fractions containing FVIII were pooled and diluted 1: 15 with 20mM Imidazol + 10mM CaCl₂ + 0.01% Tween80, pH7.3, except FVIII-variants with deletions of the a3 domain which were diluted 1:45 in the same buffer. The diluted pool was applied to a column packed with Poros 50HQ (PerSeptive Biosystem), with the column dimensions 0.5cm in diameter and 5cm in bed height giving 1mL in column volume. The column was equilibrated at 300cm/h with 20mM Imidazol + 10mM CaCl₂ + 0.01% Tween80 + 50mM NaCl + 1M Glycerol, pH7.3 prior to application. The column was washed with equilibration buffer before the elution using a linear gradient over 5 column volumes from equilibration buffer to 20mM Imidazol + 10mM CaCl₂ + 0.01% Tween80 + 1M NaCl + 1M Glycerol, pH7.3. The fractions containing FVIII were pooled and the pool was stored at -80° until use.

The FVIII-variants with HIS-tag was purified essentially as described above, however the second purification step (F25-sepharose) was exchanged to Chelating Sepharose FF (GE Healtcare) charged with 2 column volumes of 1M NiSO₄. The column dimension was 0.5cm in diameter and 5cm bed height giving 1mL column volume. The column was equilibrated with 30mM Imidazol + 10mM CaCl₂ + 0.01% Tween80 + 1.5M NaCl, pH7.3 at 180cm/h prior to application. After application the column was washed with 30 column volumes of equilibration buffer prior to elution using a linear gradient over 5 column volumes to 250mM Imidazol + 10mM CaCl₂ + 0.01% Tween80 + 1.5M NaCl, pH7.3. The fractions containing FVIII were pooled and diluted 1:30 with 20mM Imidazol + 10mM CaCl₂ + 0.01% Tween80, pH7.3. The final purification step (Poros 50HQ) was performed as described above.

### EXAMPLE 12

### Purification and characterization of FVIII- AP3 scFV fusions

Purification of the said AP3 F8 fusion protein (EXAMPLE 9) was conducted using a 3-step process com-posed of an immunoaffinity chromatography step based on the VIIISelect resin (GE Healhcare, cat. no. 17-5455-02) followed by a second immunoaffinity chromatography step based on the antibody F25 coupled to CNBr-Sepharose FF (Thim L, Vandahl B, Karlsson J, Klausen NK, Pedersen J, Krogh TN, Kjalke M, Petersen JM, Johnsen LB, Bolt G, Nørby PL, Steenstrup TD (2009) Purification and characterization of a new recombinant factor VIII (N8) Haemophilia 16: 349-59) and finally an anion exchange chromatography step based on the resin Poros 50HQ (Applied Biosystems cat. no. 1-2559-07). The purification was conducted using an ÄktaExplorer chromatography system (GE Health-care, cat. no. 17-1068-01). The buffer systems used for the first purification step was an equilibration buffer composed of 20mM imidazole, 10mM CaCl₂, 0.02% Tween80, 250mM NaCl, pH 7.3, a wash buffer composed of 20mM Imidazole, 10mM CaCl₂, 0.02% Tween80, 1.5M NaCl, pH 7.3 and an elution buffer composed of 20mM imidazole, 10mM CaCl₂, 0.02% Tween80, 1M Ammoniumacetate, 6.5M 1,2-propanediol, pH 7.3. The harvest was diluted 2x with an dilution buffer composed of 20mM Imidazole, 10mM CaCl₂, 0.02% tween80, pH 7.3 and applied to the pre-equilibrated VIIISelect column. The column was washed with 6 column volumes of equilibration buffer and 6 column volumes of wash buffer. The protein was eluted isocratically in approx. 3 column volumes. Prior to the second immunoaffinity purification step, the elution pool from the first purification step was diluted 10x using the above mentioned dilution buffer. The buffer systems used for the second purification step was an equilibration buffer composed of 20 mM Imidazole, 10 mM CaCl₂, 0.02% Tween 80, 150 mM NaCl, pH 7.3, a wash buffer composed of 20mM Imidazole, 10mM CaCl₂, 0.02%Tween80, 1.5M NaCl, pH 7.3 and an elution buffer composed of 0.5 M Imidazole, 10 mM CaCl₂, 0.02% Tween 80, 150 mM NaCl, pH 7.3. Following application of the sample to the pre-equilibrated F25-Sepharose FF column, the column was washed with 6 column volumes of equilibration buffer and with 6 column volumes of wash buffer. The protein was eluted isocratically in approx. 1.5 column volumes. Prior to the third anion exchange purification step, the elution pool from the second purification step was diluted 15x using the above mentioned dilution buffer. The buffer systems used for the third purification step was an equilibration buffer composed of 20mM imidazole, 10mM CaCl₂, 0.02% Tween80, 50mM NaCl, 1M Glycerol, pH 7.2 and an elution buffer composed of 20mM imidazole, 10mM CaCl₂, 0.02% Tween80, 1M NaCl, 1M Glycerol, pH 7.3. Following application of the sample to the pre-equilibrated Poros HQ50 column, the column was washed with 8 column volumes of equilibration buffer. The protein was eluted from the column using a linear gradient from 0 - 100 % over 5 column volumes followed by 10 column volumes of 100 % elution buffer. The protein eluted early on the gradient, typically between 10 - 30 % of elution buffer. Yields were followed using a chromogenic FVIII assay COATEST® Factor VIII (Chromogenix, COATEST SP FVIII cat. no. 82 4086 63) and a SpectraMax spectrophotometer (Molecular Devices, cat. no. M3). The protein quality was analyzed using SDS-PAGE/SilverStain and RP-HPLC, showing that a pure and homogenous protein preparation composed of light chain, heavy chain and single chain. The final protein concentration was determined based on the RP-HPLC analyses.
**SEQ ID NO 39: F8-500 AP3-LC-HC scFV -Δa3**
**SEQ ID NO 40: F8-500 AP3-HC-LC scFV -Δa3**
**SEQ ID NO 41: F8-500 AP3-HC-LC scFV**
**SEQ ID NO 42: F8-500 AP3-LC-HC scFV**

### EXAMPLE 13

### Step 1:

A reagent of the general formula 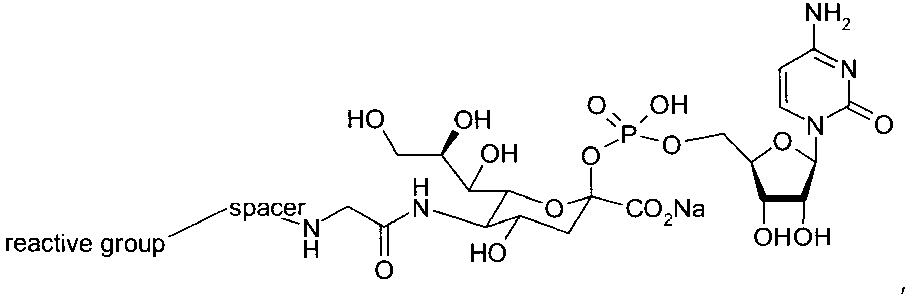 wherein non-exclusive examples for the reactive group are groups comprising maleimide, azide, alkynes, aldehydes and a non-exclusive example for the spacer is a PEG moiety with an average molecular weight of e.g. 3, kDa, 5 kDa, 10 kDa, or 20 kDa may be reacted with a platelet binding protein such as e.g. Abciximab or AP3 or proteins derived from Abciximab or AP3 such as but not exclusive e.g. single chain variants or FAB-fragments, leading to a compound with the general formula of intermediate B1.

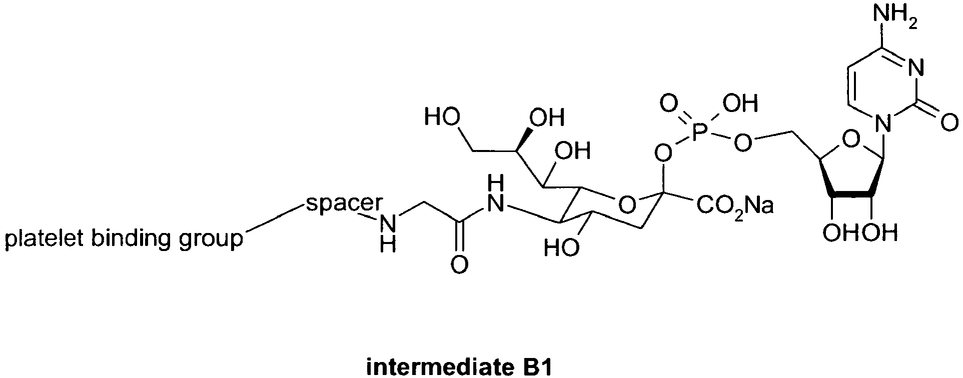

The attachment point of the spacer at the platelet binding protein may depend on the type of reactive group which had been used to assemble a compound of the general formula of intermediate B1. If e.g. the reactive group had been an aldehyde, one possibility could be that the spacer has been attached to one of the N-termini of the platelet binding protein by reductive alkylation in the presence of NaCNBH₃. If e.g. the reactive group had been a maleimide, one possibility could be that the spacer has been attach to a free Cystein in the platelet binding protein. The Cystein may be liberated prior reaction by treatment with a suitable reagent such as but not exclusively e.g. and enzyme or tris(carboxyethyl)posphine hydrochloride.

### Step 2:

Sialic acids may be removed from glycans of FVIII by reaction with a sialidase. The compound with the general formula of intermediate B1 may be reacted in the presence of a suitable enzyme such as e.g. ST3-Gal-I - when reacted with an *O*-glycan of FVIII - or ST3-GAlIII - when reacted with a *N*-glycan of FVIII - to give a compound of the general structure of product:

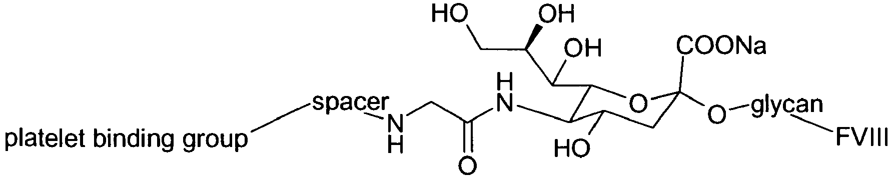

### Intermediates

### Intermediate Example 1

### N-((3-(ω-(4-formylbenzoylamino)10 kDa PEGyl)propionylamino)acetyl)-O²-[5']cytidylyl-ξ-neuraminic acid:

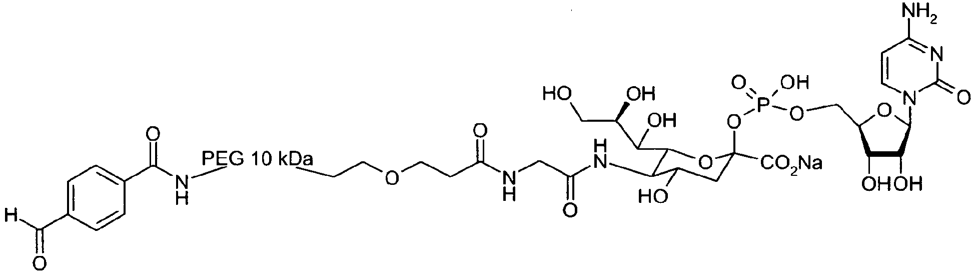

### Step 1:

### N-((3-(ω-(9H-Fluoren-9-ylmethoxycarbonylamino)10 kDa PEGyl)propionylamino)acetyl)-O²-[5']cytidylyl-ξ-neuraminic acid:

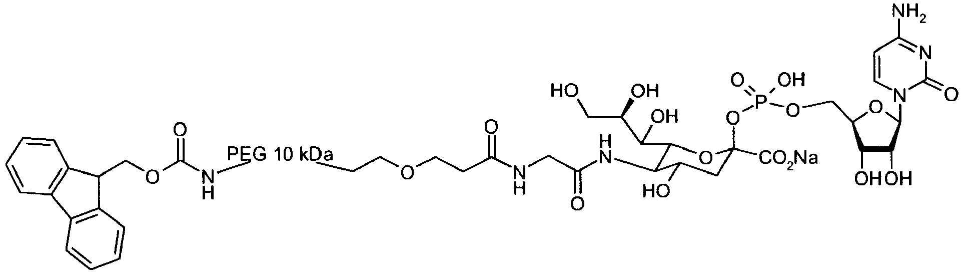

*N*-(aminoacetyl)-*O*²-[5']cytidylyl-ξ-neuraminic acid (18 mg, 0.029 mmol) was dissolved in a buffer consisting of 50 mM TRIS which had been adjusted to pH 8.9 (4 ml). The current pH was checked and was adjusted to pH 8.9 by addition of 0.1 N hydrochloric acid. THF (16 ml) was added. Approximately half of the final amount of 3-(ω-(9H-fluoren-9-ylmethoxycarbonylamino)10 kDa PEGyl)propionic acid N-hydroxysuccinimidyl ester (commercially available at for example at Rapp Polymere GmbH, 200 mg in total, 0.019 mmol) was added. The reaction mixture was stirred at room temperature. After 1 h, the second half of 3-(ω-(9H-fluoren-9-ylmethoxycarbonylamino)10 kDa PEGyl)propionic acid N-hydroxysuccinimidyl ester was added. The reaction mixture was stirred for 16 h at room temperature. The THF was removed in vacuo with a bath temperature of 25 °C. The remaining mixture was filtered and subjected to a size exclusion chromatography, using a G25 gel with a bed size of 26 mm in diameter and 10 cm in length at a flow of 7 ml/min, utilizing a buffer of 25 mM ammonium hydrogencarbonate. The fractions containing the desired compound were pooled and lyophilized to give 453 mg of material containing *N*-((3-(ω-(9H-fluoren-9ylmethoxycarbonylamino)10 kDa PEGyl)propionylamino)acetyl)-*O*²-[5']cytidylyl-ξ-neuraminic acid.
The ¹H-NMR-spectrum performed in DMSO-d₆ showed the presence of the cytidylyl moiety as well as the fluorenyl-9-ylmethoxycarbonyl moiety. The material was stored in the freezer.

This reaction was repeated with 1 g of 3-(ω-(9H-fluoren-9-ylmethoxycarbonylamino)10 kDa PEGyl)propionic acid N-hydroxysuccinimidyl ester and 90 mg of *O*²-[5']cytidylyl-ξ-neuraminic acid. The purification was performed on a HPLC C4-column with a diameter of 2 cm, using a gradient of 30-50% of a mixture consisting of a 5 mM aqueous solution of ammonium hydrogencarbonate in acetontrile in a 50 mM solution of ammonium hydrognecarbonate in water. The fractions containing the desired compound were collected and lyophilized to give 288 mg of the desired compound. The ¹H-NMR spectrum corresponded to the ¹H-NMR spectrum found in the experiment described above.

### Step 2:

### N-((3-(ω-Amino10 kDa PEGyl)propionylamino)acetyl)-O²-[5']cytidylyl-ξ-neuraminic acid

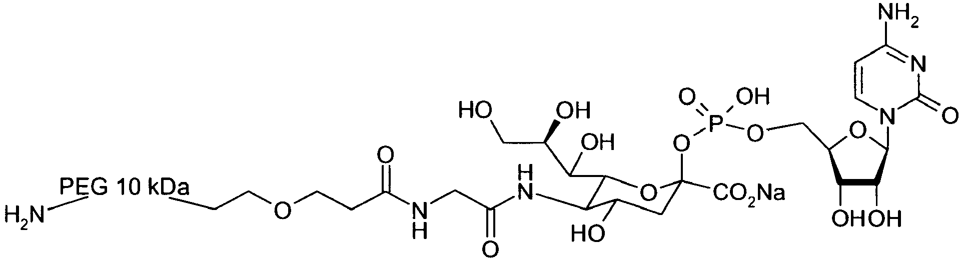

N-((3-(ω-(9H-Fluoren-9-ylmethoxycarbonylamino)10 kDa PEGyl)propionylamino)acetyl)-*O*²-[5']cytidylyl-ξ-neuraminic acid (453 mg) were dissolved in N,N-dimethylformamide (12 ml). Piperidine (1.25 ml) was added. The clear solution was stirred for 20 min at room temperature. Ether (200 ml) was added. The mixture was left at room temperature for 1.5 h, in order to let the formed precipitation grow old. The precipitation was isolated by riltration. It was dissolved in dichloromethane (4 ml). Ethyldiisopropylamine (1 ml) was added. Ether (250 ml) was added. The mixture was left at room temperature for 16 h in order to let the formed precipitation grow oled. The precipitation was isolated by filtration and dried in vacuo. The ¹H-NMR spectrum in DMSO-d₆ showed no signals of a 9H-fluoren-9-ylmethoxycarbonyl group, whereas signals assigned to a cytidylyl moiety could be found. The material was stored in the freezer.

### Step 3:

### 4-Formylbenzoic acid 2,5-dioxopyrrolidin-1-yl ester

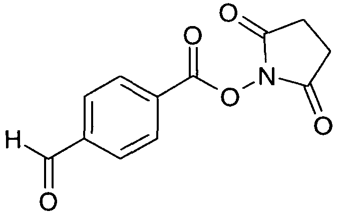

Triethylamine (2.04 ml, 14.65 mmol) and 2-succinimido-1,1,3,3-tetramethyluronium tetrafluoroborate (TSTU, 4.44 g, 14.65 mmol) were successively added to a solution of 4-formylbenzoic acid (2.0 g, 13.3 mmol) in N,N-dimethylformamide (30 ml). The reaction mixture was stirred at room temperature for 16 h. It was diluted with ethyl acetate (150 ml) and washed with a 10% aqueous solution of sodium hydrogen sulphate (100 ml). The aqueous phase was extracted with ethyl acetate (2 x 30 ml). The combined organic layers were washed with a mixture of brine (50 ml) and water (50 ml). The combined organic layers were dried over magnesium sulphate. The solvent was removed in vacuo. The crude product was recrystallized from ethyl acetate to give 1.89 g of 4-formylbenzoic acid 2,5-dioxopyrrolidin-1-yl ester.
¹H-NMR (CDCl₃). δ 2.95 (s, 4 H); 8.04 (d, 2 H), 8.32 (d, 2 H); 10.15 (s, 1 H).

### Step 4:

*N*-((3-(ω-Amino10 kDa PEGyl)propionylamino)acetyl)-*O*²-[5']cytidylyl-ξ-neuraminic acid (42 mg, 0.004 mmol) was dissolved in dichloromethane (2 ml). Ethyldiisopropylamine (0.002 ml, 0.012 mmol) was added to the solution. A solution of 4-formylbenzoic acid 2,5-dioxopyrrolidin-1-yl ester (19.32 mg, 0.078 mmol) was in dichloromethane (0.5 ml) was added. The reaction mixture was stirred for 16 h at room temperature. The solvent was removed in vacuo with a bath temperature of 25 °C. The residue was suspended in a 25 mM aqueous solution of ammonium hydrogencarbonate (15 ml). The non-soluble material was removed by filtration. It was divided into 5 parts. Each of them were subjected to a size exclusion chromatography using a G25 on a column with diameter of 26 mm and a length of 10 cm with a flow of 7 ml/min utilizing a buffer of 25 mM ammonium hydrogencarbonate. All the fractions containing the desired material were combined and lyophilized. The ¹H-NMR spectrum in DMSO-d6 showed the presence of both the aldehyde moiety and the cytidylyl moiety. The obtained material was kept in the freezer.

### Intermediate Example 2:

### N-((3-(ω-(3-(2,5-dioxo-2,5-dihydropyrrol-1-yl)propionylamino)10 kDa PEGyl)propionylamino)acetyl)-O²-[5']cytidylyl-ξ-neuraminic acid

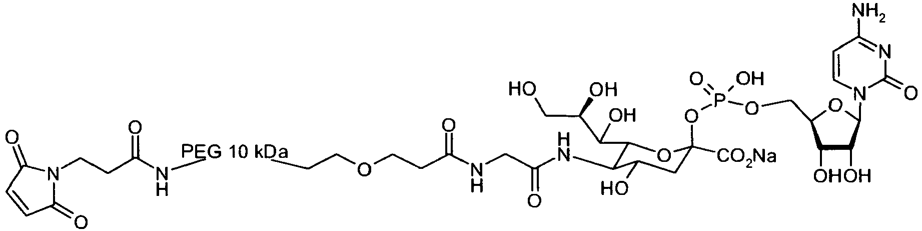

### Step 1:

### 3-(2,5-Dioxo-2,5-dihydropyrrol-1-yl)propionic acid 2,5-dioxopyrrolidiny-1-yl ester

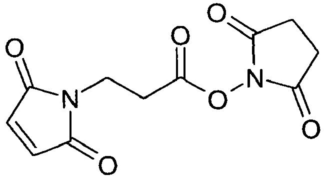

3-Maleimidopropionic acid (1.0 g, 5.9 mmol) was dissolved in tetrahydrofuran (20 ml). 2-Succinimido-1,1,3,3-tetramethyluronium tetrafluoroborate (TSTU, 2.14 g, 7.1 mmol) and ethyldiisopropylamine (1.24 ml, 7.1 mmol) were added subsequently. N,N-Dimethylformamide (5 ml) was added. The reaction mixture was stirred at room temperature, while it was turning sluggish. The mixture was stirred for 2 min. N,N-Dimethylformamide (5 ml) was added. The mixture was stirred for 2.5 h at room temperature. It was diluted with dichloromethane (150 ml) and was washed subsequently with a 10% aqueous solution of sodium hydrogensulphate (150 ml), a saturated aqueous solution of sodium hydrogencarbonate (150 ml) and water (150 ml). It was dried over magnesium sulphate. The solvent was removed in vacuo. The crude product was recrystallized from ethyl acetate to give 1.20 g of 3-(2,5-dioxo-2,5-dihydropyrrol-1-yl)propionic acid 2,5-dioxopyrrolidin-1-yl ester.
MS: m/z = 289, required for [M+Na]⁺: 289
¹H-NMR (CDCl₃) δ 2.82 (m, 4 H); 3.02 (t, 2 H); 3.94 (t, 2 H), 6.73 (s, 2 H).

### Step 2:

N-((3-(ω-Amino10 kDa PEGyl)propionylamino)acetyl)-*O*²-[5']cytidylyl-ξ-neuraminic acid (100 mg, 0.009 mmol) was dissolved in a mixture of tetrahydrofuran (2 ml) and dichloromethane (10 ml). A solution of 3-(2,5-dioxo-2,5-dihydropyrrol-1-yl)propionic acid 2,5-dioxopyrrolidiny-1-yl ester (50 mg, 0.18 mmol) in dichloromethane (3 ml) was added. Ethyldiisopropylamine (0.005 ml, 0.028 mmol) was added. The reaction mixture was stirred at room temperature fro 16 h. Dichloromethane (2 ml) and ethyldiisopropylamine (0.5 ml) were added. Amionomethylated polystyrene resin (commercially available at e.g. Novabiochem, loading 0.85 mmol/g, 438 mg, 0.372 mmol) was added. The mixture was slowly stirred at room temperature for 1 h. The resin was removed by filtration. The solvent was removed in vacuo with a bath temperature of 25 °C. The residue was dissolved in dichloromethane (4 ml). Ether (200 ml) was added. The mixture was left at room temperature for 2 h in order to let the formed precipitation grow old. The precipitation was isolated by filtration and dried in vacuo to give 38 mg of the title compound. The ¹H-NMR spectrum in DMSO-d₆ showed the presence of a maleimide group.

### Intermediate Example 3

### Attachment of N-((3-(ω-(3-(2,5-dioxo-2,5-dihydropyrrol-1-yl)propionylamino)10 kDa PEGyl)propionylamino)acetyl)-O²-[5']cytidylyl-ξ-neuraminic acid to Cys 248 of AP3 scFV C34S S248C

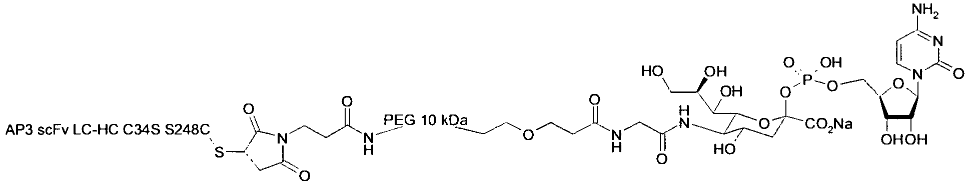

A solution of Tris(2-carboxyethyl)phosphine hydrochloride (0.40 mg) in a buffer (0.40 ml)consisting of 20 mM imidazole, 10 mM CaCl₂, 0.02% Tween80, 1 M glycerol which had been adjusted to pH 7.35 was added to a solution with a concentration of 0.53 mg/ml of AP3 scFv LC-HC C39S S248C with a Flag tag at its C-terminus and an extrac Cys attached to the Cysteine at position 248 via a S-S bridge in a solution of 100 mM HEPES and 150 mM NaCl which had been adjusted to pH 7.5 with (4 ml, 2.12 mg, 76 nmol). The reaction mixture was gently shaken at 20 °C. It was divided into two parts. Each of them were added to a PD-10 column (GE-Healthcare), using a buffer of 25 mM HEPES which had been adjusted to pH 7.0. The eluates of the column (each of them 3.5 ml) were combined.

A solution of *N*-((3-(ω-(3-(2,5-dioxo-2,5-dihydropyrrol-1-yl)propionylamino)10 kDa PEGyl)propionylamino)acetyl)-*O*²-[5']cytidylyl-ξ-neuraminic acid (3.3 mg, 305 nmol) in a buffer of 25 mM HEPES, which had been adjusted to pH 7.0 (0.43 ml) was added to the solution of the protein. The reaction mixture was gently shaken at 20 °C for 4 h. It was placed in an Amicon ultracentrifugation device with a cut off of 10 kDa. It was subjected to an ultracentrifugation at 4000 rpm at 10 °C for 10 min. The mixture was kept in the freezer until purification.

For purification the mixture was thawed. It was subjected to a size exclusion chromatography utilizing a Superdex 200 gel with a bed size of 16 mm in diameter and 60 cm in length at a flow of 1 ml/min and a buffer of 25 mM TRIS and 150 mM NaCl which had been adjusted to pH 8.0. The fractions containing the desired product were pooled to give 1.61 mg of the desired protein. The SDS-PAGE gel was in accordance with the expectation. It was found that the material contained a lot of aggregated protein.

### Intermediate Example 4

### Conjugation of N-((3-(ω-(4-formylbenzoylamino)10 kDa PEGyl)propionylamino)acetyl)-O²-[5']cytidylyl-ξ-neuraminic acid to one N-terminus of Abxicimab

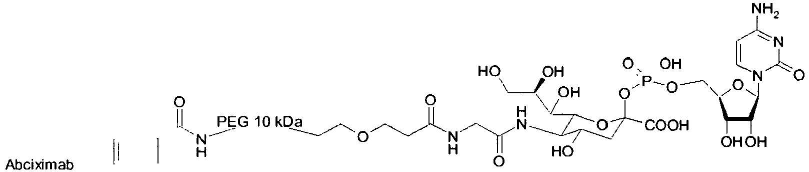

A solution of commercially available Abciximab (ReoPro, 10 mg, 215 nmol, in a 2 mg/ml solution the commercial buffer) was placed in an Amicon ultracentrifugation device with a cut off of 10 kDa. Buffer consisting of 25 mM HEPES, which had been adjusted to pH 7.4 (5 ml) was added. An ultracentrifugation was performed at 4000 rpm at 10 °C for 10 min. Buffer consisting of 25 mM HEPES, which had been adjusted to pH 7.4 (10 ml) was added. An ultracentrifugation was performed at 4000 rpm at 10 °C for 10 min. Buffer consisting of 25 mM HEPES, which had been adjusted to pH 7.4 (10 ml) was added. An ultracentrifugation was performed at 4000 rpm at 10 °C for 10 min. The remaining solution of 0.65 ml was placed in a plastic reactor. Buffer consisting of 25 mM HEPES, which had been adjusted to pH 7.4 (3.85 ml) was added. A solution of N-((3-(ω-(4-formylbenzoylamino)10 kDa PEGyl)propionylamino)acetyl)-*O*²-[5']cytidylyl-ξ-neuraminic acid (14 mg, 1290 nmol) in a buffer consisting of 25 mM HEPES which had been adjusted to pH 7.0 (1.5 ml) was added. The reaction mixture was gently shaken at 300 rpm for 3 min at 20 °C. A freshly prepared 1.0 M solution of sodium cyanoborohydride in water (0.025 ml) was added. The reaction mixture was gently shaken at 300 rpm at 20 °C. After 1 h, another portion of the solution of sodium cyanoborohydride (0.025 ml) was added. After 1 h, another portion of the solution of sodium cyanoborohydrice (0.025 ml) was added. After 1 h, another portion of the solution of sodium cyanoborhydride was added. The solution was gently shaken at 300 rpm at 20 °C for 16 h. The reaction mixture was placed in an Amicon ultracentrifugation device with a cut off of 10 kDa. It was subjected to an ultracentrifiugation at 4000 rpm for 10 min at 18 oC. The remaining solution of 0.360 ml was filtered and subjected to a size exclusion chromatography on a Superdex200 gel with a bed size of 016 mm X 60 cm at a flow of 1 ml/min utilizing a buffer of 25 mM TRIS, 150 mM NaCl, which had been adjusted to pH 8.0 as eluent. Fractions containing the desired product were combined into two batches. Each of those batches were placed in an Amicon ultracentrifugation device with a cut off of 10 kDa. They were subjected to an ultracentrifugation at 4000 rpm at 10 °Cfor 10 min, yielding 2.67 mg and 3.27 mg respectively. For quantification a molar absorbance of 10.94 was used on a Nanodrop ® spectrophotometer. The analysis of the product by SDS-PAGE were in accordance with the expectation for a conjugate of *N*-((3-(ω-(4-formylbenzoylamino)10 kDa PEGyl)propionylamino)acetyl)-*O*²-[5']cytidylyl-ξ-neuraminic acid to one N-terminus of Abxicimab.

### Intermediate Example 5

### Reaction of N-((3-(ω-(4-formylbenzoylamino)10 kDa PEGyl)propionylamino)acetyl)-O²-[5']cytidylyl-ξ-neuraminic acid with AP3-FAB-fragment at one of its N-termini

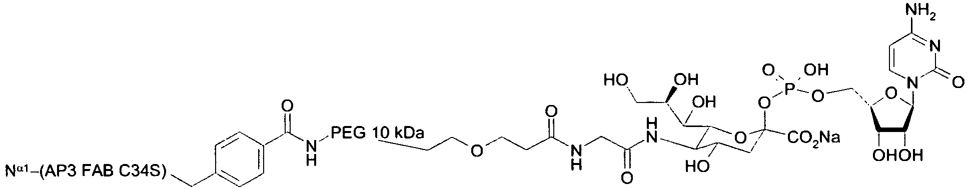

A 0.34 mg/ml solution of AP3 C39S FAB fragment in a PGS buffer pH 7.2 (1.7 mg, 35 nmol) was placed in an Amicon ultracentrifugation device with a cut off of 10 kDa. A buffer consisting of 25 mM HEPES, 25 mM NaCl pH 7.0 (7 ml) was added. The mixture was submitted to an ultracentrifugation at 4000 rpm at 20 °C for 15 min. Buffer consisting of 25 mM HEPES, 25 mM NaCl pH 7.0 (10 ml) was added. The mixture was submitted to an ultracentrifugation at 4000 rpm at 20 °C for 15 min. Buffer consisting of 25 mM HEPES, 25 mM NaCl pH 7.0 (10 ml) was added. The mixture was submitted to an ultracentrifugation at 4000 rpm at 20 °C for 15 min. Approximately 0.480 ml of solution was left. A solution of N-((3-(ω-(4-formylbenzoylamino)10 kDa PEGyl)propionylamino)acetyl)-*O*²-[5']cytidylyl-ξ-neuraminic acid (2.31 mg, 212 nmol) in a buffer consisting of 25 mM HEPES, 25 mM NaCl pH 7.0 (0.37 ml) was added. The mixture was gently shaken at 20 °C for 5 min. A 1 M solution of sodium cyanoborohydride in water (0.0045 ml) was added. The reaction mixture was shaken at 20 °C for 1 h. A 1 M solution of sodium cyanoborohydride in water (0.0045 ml) was added. The reaction mixture was shaken at 20 °C for 1 h. A 1 M solution of sodium cyanoborohydride in water (0.0045 ml) was added. The reaction mixture was shaken at 20 °C for 18 h. The reaction mixture was placed in an Amicon ultracentrifugation device with a cut off of 10 kDa. A buffer consisting of 25 mM TRIS, 25 mM NaCl pH 8.00 (2.5 ml) was added. The mixture was subjected to an ultracentrifugation at 4000 rpm at 20 °C for 4 min. A buffer consisting of 25 mM TRIS, 25 mM NaCl pH 8.00 (2.2 ml) was added. The mixture was subjected to an ultracentrifugation at 4000 rpm at 20 °C for 22 min. A buffer consisting of 25 mM TRIS, 25 mM NaCl pH 8.00 (3.2 ml) was added. The mixture was subjected to an ultracentrifugation at 4000 rpm at 20 °C for 12 min. Buffer consisting of 25 mM TRIS, 25 mM NaCl pH 8.00 (2.2 ml) was added to the mixture of approximately 0.120 ml. This mixture was divided into two equal parts. Each of which were added to a spin Q column (VIVAPURE Q MINI M, Sartorius, product no.: VS-1X01QM24) which had been equilibrated with a buffer consisting of 25 mM TRIS, 25 mM NaCl pH 8.00. Each column was subjected to an ultracentrifugation at 2000 rpm at room temperature for 5 min. Buffer consisting of 25 mM TRIS, 25 mM NaCl pH 8.00 (0.400 ml) was added to each of the spin columns. Each column was subjected to an ultracentrifugation at 2000 rpm at room temperature for 5 min. Buffer consisting of 25 mM TRIS, 500 mM NaCl pH 8.00 (0.400 ml) was added to each of the spin columns. Each column was subjected to an ultracentrifugation at 2000 rpm at room temperature for 5 min. The filtrates were combined and placed in an Amicon ultracentrifugation device with a cut off of 10 kDa. They were subjected to an ultracentrifugation at 4000 rpm at 18 °C for 8 min. The obtained solution was kept at -80 °C until purification.

The solution was thawed and subjected to a size exclusion chromatography on a Superdex 75 column with a bed size of 016 mm x 600 mm at a flow of 0.80 ml/min, utilizing a buffer of 25 mM TRIS, 150 mM NaCl at pH 8.00. The fractions containing the desired product - as judged by SDS-PAGE - were pooled and placed in an Amicoun ultracentrifugation device with a cut off of 10 kDa. They were subjected to an ultracentrifugation at 4000 rpm at 18 °C for 18 min. The solution of 0.280 ml was frozen to -80 °C as soon as possible. Quantification on a Nanodrop® apparatus using a molar absorbance of 13.05 showed a concentration of 0.23 mg/ml. The product showed the expected bands in a SDS-PAGE by silver staining. No unreacted PEG was identified on a SDS-PAGE using a staining procedure as described in Kurfürst, M. M. Analytical Biochemistry 200, 244-248. 1992.

### EXAMPLE 14

### Conjugation of AP3 scFv LC-HC C39S S248C to FVIII

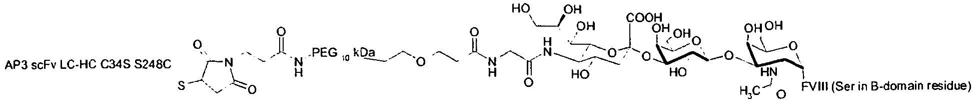

A solution of B-domain deleted FVIII which has a residual B-domain sequence of SFSQNSRHPSQNPPVLKRHQR (SEQ ID NO 4) at the C-terminus of the heavy chain (1 mg, 5.64 mmol) in a buffer consisting of 20 mM imidazole, 10 mM CaCl₂, 150 mM NaCl, 0.02% Tween80 and 1 M glycerol, which had been adjusted to pH 7.35 (0.018 ml) was placed in an Amicon ultracentrifugation device with a cut off of 10 kDa. A solution of the attachment product of *N*-((3-((ω-(3-(2,5-dioxo-2,5-dihydropyrrol-1-yl)propionylamino)10 kDa PEGyl)propionylamino)acetyl)-*O*²-[5']cytidylyl-ξ-neuraminic acid to Cys 248 of AP3 scFV C34S S248C (Intermediate example 3/EXAMPLE 13, 1.29 mg, 34 nmol) in a solution of 25 mM TRIS and 150 mM NaCl which had been adjusted to pH 8.0 (0.680 ml) was added and a buffer consisting of 20 mM histidine, 10 mM CaCl₂, 10% glycerol, 0.02% Tween80, 500 mM NaCl which had been adjusted to pH 6.07 (3 ml) were added subsequently. The mixture was subjected to an ultracentrifugation at 4000 rpm at 10 °C for 20 min. The remaining volume was 0.800 ml or 1.25 mg/ml for FVIII. A solution of Sialidase from A. Urifaciens (0.43 mg/ml, 302 U/mg, 0.0049 ml, 0.645 U) and a solution of ST3-Gal-I (2.5 mg/ml, 0.105 mg, 0.042 ml) were added subsequently. The reaction mixture was gently shaken at 32 °C for 1 min and thereafter left at 32 °C for 18 h. It was kept in the freezer until purification.

The reaction mixture was thawed. It was divided into two parts, each of which were subjected to a size exclusion chromatography using a Superose 6 gel with a bed size of 10 mm in diameter and 300 mm in length at a flow of 0.30 ml/min and a buffer consisting of 20 mM imidazole, 10 mM CaCl₂, 0.02% Tween80, 150 mM NaCl, and 1 M glycerol, which had been adjusted to pH 7.35 as eluent. All fractions of both runs containing the desired product were pooled. They were placed in an Amicon ultracentrifugation device with a cut off of 10 kDa and subjected to an ultracentrifugation at 4000 rpm at 10 °C for 18 min.

A solution of CMP-*N*-acetylneuraminic acid (CMP NeuNac, 1.5 mg, 2597 nmol) in a buffer consisting of 20 mM imidazole, 10 mM CaCl₂, 0.02% Tween80, 150 mM NaCl, and 1 M glycerol, which had been adjusted to pH 7.35 (0.100 ml) and a 0.33 mg/ml solution of ST3Gal-III (0.10 ml, 0.033 mg) were added subsequently. The reaction mixture was gently shaken at 300 rpm and thereafter kept in the freezer until purification.

The reaction mixture was divided into two parts. Each of those was filtered through a 0.00045 mm filter. They were applicated to a sepharose column with a bed size of 5 mm in diameter and 5 cm in length to which a F25 antibody had been attached after activation with CNBr. F25 is a known antibody for FVIII. After application, the column was washed for 3 CV with a buffer consisting of 20 mM imidazole, 10 mM CaCl₂, 0.02% Tween80, 150 mM NaCl, and 1 M glycerol, which had been adjusted to pH 7.35 at a flow of 0.6 ml/min. Thereafter it was washed for 3 CV with a buffer consisting of 20 mM imidazole, 10 mM CaCl₂, 0.02% Tween80, and 650 mM NaCl, which had been adjusted to pH 7.35 at a flow of 0.6 ml/min. Finally, the compound was eluted within 6 CVwith a buffer consisting of 20 mM imidazole, 10 mM CaCl₂, 0.02% Tween80, 2.5 M NaCl in a 50%v/v ethylene glycol/water solution, which had been adjusted to pH 7.35 at a flow of 0.1 ml/min. The fractions of both runs containing the desired product were pooled. They were placed in an Amicon ultracentrifugation device with a cut off of 10 kDa. They were subjected to an ultracentrifugation at 4000 rpm at 10 °C for 14 min. The remaining solution was subjected to a size exclusion chromatography on a Superose 6 material with a bed size of 10 mm in diameter and 30 cm in length with a flow of 0.50 ml/min utilizing a buffer consisting of 10 mM Histidine, 1.7 mM CaCl₂, 0.01% Tween80, 0.3 M NaCl, 8.8 mM sucrose which had been adjusted to pH 7. The fractions containing the desired compound in a suitable purity were pooled and place in an Amicon ultracentrifugation device with a cut off of 10 kDa. They were subjected to an ultracentrifugation at 4000 rpm at 9 °C for 12 min. Using a molar absorbance of 14.46, the yield was found to be 0.0176 mg of a conjugate of AP3 scFv LC-HC C39S S248C to FVIII. The analyses by SDS-PAGE gel under non-reduced conditions were in accordance with the expectation. From the form of the peaks in the chromatograpies it was concluded that the material contained a lot of aggregated protein.

### EXAMPLE 15

### Conjugation of Abciximab to FVIII at the O-glycan

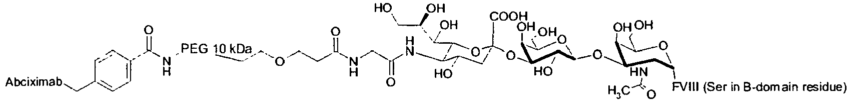

A buffer consisting of 20 mM histidine, 10 mM CaCl₂, 150 mM NaCl, 0.02% Tween80 and 1 M glycerol which had been adjusted to pH 7.35 (2.5 ml) was added to a solution of of B-domain deleted FVIII which has a residual B-domain sequence of SFSQNSRHPSQNPPVLKRHQR (SEQ ID NO 4) at the C-terminus of the heavy chain (5.7 mg/ml, 1 mg, 5.6 nmol) in a buffer consisting of 20 mM imidazole, 10 mM CaCl₂, 150 mM NaCl, 0.02% Tween80 and 1 M glycerol which had been adjusted to pH 7.35. A solution of the conjugate of of *N*-((3-(ω-(4-formylbenzoylamino)10 kDa PEGyl)propionylamino)acetyl)-*O*²-[5']cytidylyl-ξ-neuraminic acid to one N-terminus of Abxicimab (Intermediate example 4, EXAMPLE 13, 2.3 mg, 39.5 nmol) in a buffer consisting of 25 mM TRIS, 150 mM NaCl, which had been adjusted to pH 8.0 (0.323 ml) was added. The solution was placed in an Amicon ultracentrifugation device with a cut off of 10 kDa. It was subjected to a ultracentrifugation at 4000 rpm at 10 °C for 20 min. Buffer consisting of 20 mM histidine, 10 mM CaCl₂, 150 mM NaCl, 0.02% Tween80 and 1 M glycerol which had been adjusted to pH 7.35 (2 ml) was added. The solution was subjected to an ultracentrifugation at 4000 rpm at 10 °C for 30 min, leaving a solution with a volume of 1.4 ml. A solution of sialidase of A. Urifaciens (0.4 mg/ml, 242 U/mg, 0.0066 ml) and a solution of ST3Gal-I (2.5 mg/ml, 0.042 ml) were added subsequently. The reaction mixture was gently shaken at 32 °C for 15 min. After that the reaction mixture was left standing at 32 °C for 20.5 h. The reaction mixture was placed in an Amicon ultracentrifugation device with a cut off of 10 kDa. It was subjected to an ultracentrifugation at 4000 rpm at 10 °C for 15 min. The remaining solution of 0.300 ml was subjected to a size exclusion chromatography, using Superose 6 material with a bed size of Ø10 mm x 300 mm at a flow of 0.5 ml/min and using a buffer consisting of 10 mM Histidine, 1.7 mM CaCl₂, 0.01% Tween80, 0.3 M NaCl, 8.8 mM sucrose which had been adjusted to pH 7 as eluent. The fractions, containing the desired product were pooled and placed in an Amicon ultracentrifugation device with a cut off of 10 kDa. Buffer, consisting of 20 mM histidine, 10 mM CaCl2, 10% glycerol, 0.02% Tween80, 500 mM NaCl which had been adjusted to pH 6.07 (2.5 ml) was added. The solution was subjected to an ultracentrifugation at 4000 rpm at 10 °C for 15 min. Buffer, consisting of 20 mM histidine, 10 mM CaCl2, 10% glycerol, 0.02% Tween80, 500 mM NaCl which had been adjusted to pH 6.07 (1.5 ml) was added. The solution was subjected to an ultracentrifugation at 4000 rpm at 10 °C for 15 min. The remaining solution of 0.220 ml was placed in a plactic reactor. A solution of commercially available CMP NeuNAc (1.73 mg, 2800 nmol) in buffer consisting of 20 mM histidine, 10 mM CaCl2, 10% glycerol, 0.02% Tween80, 500 mM NaCl which had been adjusted to pH 6.07 (0.173 ml) was added. The reaction mixture was gently shaken at 300 rpm at 32 °C for 1 h. It was subjected to a size exclusion chromatography using using Superose 6 material with a bed size of Ø10 mm x 300 mm at a flow of 0.5 ml/min and using a buffer consisting of 10 mM Histidine, 1.7 mM CaCl₂, 0.01% Tween80, 0.3 M NaCl, 8.8 mM sucrose which had been adjusted to pH 7 as eluent. The fractions, containing the desired product were pooled an placed in an Amicon ultracentrifugation device with a cut off of 10 kDa. The pool was subjected to an ultracentrifugation at 4000 rpm at 10 °C for 5 min to give a solution of 0.275 ml of 0.0358 mg of a conjugation product of Abciximab to FVIII at the O-glycan. For quantification a molar absorbance of 13.15 was used on an Nanodrop ® spectrofphotometer. The SDS-PAGE analysis was in accordance with the expectation for a SDS-PAGE of a conjugation product of Abciximab to FVIII at the O-glycan.

### EXAMPLE 16

### Conjugation of AP3-FAB fragment to BDD-FVIII

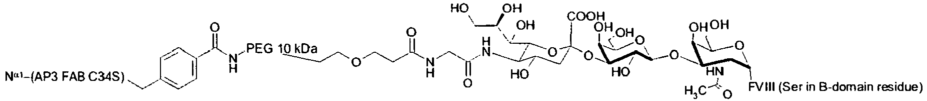

The product of the reaction of intermediate example 5 was placed in an Amicon ultracentrifugation device with a cut off of 10 kDa. A buffer consisting of 20 mM histidine, 10 mM CaCl2, 10% glycerol, 0.02% Tween80, 500 mM NaCl at pH 6.07 (4 ml) was added. The solution was subjected to an ultracentrifugation at 4000 rpm, at 20 °C for 12 min. A solution of B-domain deleted FVIII which has a residual B-domain sequence of SFSQNSRHPSQNPPVLKRHQR (SEQ ID NO 4) at the C-terminus of the heavy chain (0.5 mg, 2.8 nmol) in a buffer consisting of 20 mM imidazole, 10 mM CaCl₂, 150 mM NaCl, 0.02% Tween80 and 1 M glycerol, which had been adjusted to pH 7.35 (0.088 ml) was added to the remaining 0.600 ml. The solution was subjected to an ultracentrifugation at 4000 rpm at 20 °C for 30 min. The remaining 0.125 ml were placed in an Eppendorf vial. A solution of sialidase of A. Urifaciens with His6 TAG (0.4 mg/ml, 0.0033 ml) and a solution of ST3Gal-I (2.5 mg/ml, 0.021 ml) were added subsequently. The reaction mixture was gently shaken at 300 rpm at 32 °C. After 20 min, the shaking was stopped, and the reaction mixture was left at 32 °C for 19 h. It was filtered and subjected to a size exclusion chromatography using Superose 6 material with a bed size of Ø10 mm x 300 mm, using a buffer consisting of 10 mM Histidine, 1.7 mM CaCl₂, 0.01% Tween80, 0.3 M NaCl, 8.8 mM sucrose which had been adjusted to pH 7 as eluent at a flow of 0.500 ml/min. The fractions containing the desired compound - as judged by SDS-PAGE - were pooled. The pool was transferred into an Amicon ultracentrifugation device with a cut off of 10 kDa. It was subjected to an ultracentrifugation at 4000 rpm at 20 °C. The concentration of the desired product in the remaining 0.270 ml was determined to be 0.29 mg/ml on a Nanodrop apparatus, using a molar absorption of 13.61. The results of SDS-PAGE under both reducing and non-reducing conditions of the isolated product were in accordance with the presence of a conjugate of the FAB-fragment of AP3 with FVIII.

### EXAMPLE 17

### Coupling of N8-(O)-PEG10kD-CHO to AP3-ONH2 to obtain AP3-N8 conjugate, compound MZ1

### Intermediate example A: Introduction of a hydroxylamine handle on the N-glycans of AP3 antibody

### 1st step: Galactosidation of AP3 antibody N-glycans:

The N-glycans of AP3 FL mIgG are mostly of the complex, biantennary type. The majority of the biantennary N-glycans carry two N-acetyl glucosamine moieties, or one N-acetyl glucosamine moiety and one galactose moiety as the ultimate monosaccharide moiety (G0F, G1F). The light chain has a biantennary glycan with one sialic acid and one galactose moiety as the ultimate monosaccharides. The G0F and G1F glycans were converted to the G2F form by (bovine) β1,4-galactosyltransferase catalyzed transfer of galactose from galactose -UDP. Briefly, to the AP3 FL mIgG antibody in solution in 50mM MES buffer pH6.4 (3mg, 800µl) was added 50mM MES buffer pH6.4 (500µl), galactose-UDP (500µl of a 12.2mg/ml solution in 10mM phosphate buffer pH7.4 containing 140mM NaCl, 3mM KCl) and manganese chloride (100µl of a 12.6mg/ml solution in water). The reaction was started by addition of β1,4-galactosyltransferase (100µl of a 10U/ml enzyme solution in 100mM HEPES pH7.5 buffer). The reaction mixture was incubated for overnight at 25°C.

### 2nd step: Introduction of a hydroxylamine handle on the N-glycans of AP3 antibody

To the galactosylated antibody obtained in the first step (3mg, 2ml) was added GSC-ONH2 (5.8mg in 190µl 10mM phosphate buffer pH7.4 containing 140mM NaCl, 3mM KCI). The reaction was started by addition of ST3Gal III (80µl, 0.4mg, 480mU).
The reaction mixture was incubated at 32°C for 21h.

### GSC-ONH2: 5'-(2-(12-((aminoxymethylcarbonyl)amino)-4-7-10-trioxadodecanoyl)-aminoethanoyl)-neuraminic acid cytidine monophosphate

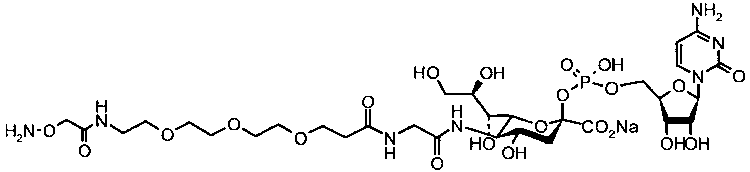

Any unmodified galactose moiety was subsequently capped by adding NAN-CMP in solution in PBS buffer (4.5mg, 100µl). The reaction mixture was incubated for 1h at 25°C.
The reaction mixture was then filtered (0.45µ filter, Gelman GHP) and buffer shifted to 20mM phosphate buffer pH7.2 containing 150mM NaCl (by ultra filtration, Millipore Centrifugal Filter Units Amicon Ultra, 0.5ml device, 10kD cut off).
The purification was run on a HiTrap Protein A HP column (1ml) using an Äkta Purifier 10 system (GE Healthcare). The loading and washing buffer was 20mM phosphate buffer pH7.2 containing 150mM NaCl, the elution buffer was 10mM formic acid adjusted to pH3.5 with 10M sodium hydroxide. The flow was 0.5ml/min, the fraction volume 0.5ml (fractionation in a deep well microtiter plate). Prior to fractionation, 0.1M Tris buffer pH9 (15µl) was added in the microtiter plate wells to avoid keeping the product at acidic pH. Relevant fractions were pooled, upconcentrated and buffer shifted to buffer A (by ultra filtration, Millipore Centrifugal Filter Units Amicon Ultra, 0.5ml device, 10kD cut off), giving the product ("AP3-ONH2") with an overall protein recovery of 50% (1.5mg, 0.91mg/ml).

In order to find out if the hydroxylamine moiety had been successfully introduced, an aliquot of the product was pegylated, using PEG20kD-CHO (60 molar equivalents of PEG20kD-CHO in solution in buffer A). An SDS polyacrylamide gel electrophoresis analysis using NuPage 4-12% Bis-Tris gel (Invitrogen) under reducing conditions (200V, 40min) was done. The gel was coomassie blue stained using SimplyBlue SafeStain (Invitrogen). Standard proteins were from Invitrogen (Novex Sharp Unstained Stds (3.5-260kD). The gel showed the disappearance of the light chain (the light chain of the unpegylated, N-glycan modified AP3 appearing at about 32kD), and only a faint band corresponding to the remaining heavy chain at about 55kD) was present. Four new bands had appeared at about 70, 100, 130, 170kD, assumed to correspond to respectively pegylated LC glycan, monopegylated Fc glycans, di-pegylated Fc glycans and an undefined product.
Thus, hydroxylamine moieties have been introduced on AP3.

### Intermediate example B: Introduction of an aldehyde handle on the N8 O-glycans:

### 1st step: desialylation of N8 and ST3Gal1 catalyzed transfer of GSC-PEG10kD-CHO on O-glycans (1pot):

### GSC-PEG10kD-CHO: N-((3-(ω-(4-formylbenzoylamino)10 kDa PE-Gyl)propionylamino)acetyl)-O²-[5']cytidylyl-ξ-neuraminic acid.

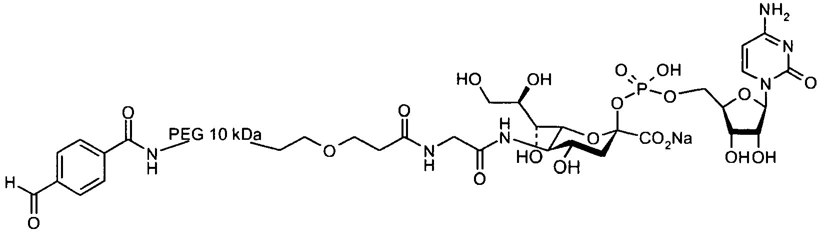

To a solution of N8 in 20mM imidazole buffer pH7.3, 10mM CaCl2, 0.02% Tween 80, 1M glycerol, 0.5M NaCl (263µl, 1.5mg) was added a solution of sialidase (sialidase from *Arthrobacter ureafaciens*) (7µl, 680mU), followed by addition of a solution of GSC-PEG10kD-CHO in PBS buffer (68µl, 1.85mg) and a solution of His-ST3Gal I in 50mM Tris, 100mM NaCl, pH8 (108µl, 0.27mg). The reaction mixture was incubated at 23°C for 24h.

After dilution in 20mM imidazole buffer pH7.3, 10mM CaCl2, 0.02% Tween 80, 1M glycerol to lower the ion concentration, the mixture was purified by anion exchange (MonoQ 5/50GL column, GE Healthcare), using an Äkta purifier 10 system (GE Healthcare): The mixture was loaded on the MonoQ 5/50 GL column equilibrated with 20 mM Imidazol pH7.3, 10 mM CaCl₂, 0.02% Tween 80, 1 M glycerol. The elution buffer B was 20 mM Imidazol pH7.3, 10 mM CaCl₂, 0.02% Tween 80, 1.5M NaCl, 1 M glycerol. The elution program was: 0 to 20%B over 5CV, 20%B over 10CV, 100%B over 10CV, with a flow of 0.5ml/min. The product was eluted with 100% elution buffer. The relevant fractions were pooled. Yield 1.2 mg, 80% protein recovery.

### 2nd step: Capping:

To a solution of the product obtained in the first step was upconcentrated, (1.13mg protein, 400µl), a solution of NAN-CMP in PBS buffer (40µl, 1.05mg) was added. The reaction was started by addition of ST3Gal III (110µl, 110µg, 0.13U). The reaction mixture was incubated at 32°C for 1h.
The reaction mixture was then purified on MonoQ, using a slightly modified elution program (compared to purification method iin step1): The elution program was: 0 to 13%B over 5CV, 13%B over 10CV, 100%B over 10CV, with a flow of 0.5ml/min. The product ("N8-(O)-PEG10kD-CHO" (NB: this product is a mixture of des-O-glycan and (O)-PEG-CHO-N8) was eluted with 100% elution buffer. The relevant fractions were pooled. Yield 0.96mg, 80% protein recovery, ie overall protein recovery from N8: 64%.
An SDS polyacrylamide gel electrophoresis analysis using NuPage 7% Tris acetate gel (Invitrogen) under reducing conditions (150V, 70min) was run. The gel was coomassie blue stained using SimplyBlue SafeStain (Invitrogen). Standard proteins were from Invitrogen (HiMark Unstained HMW). The pattern obtained was as expected: 3 bands of MW about 84kD, 93kD and 120kD, which could correspond to respectively the light chain, the heavy chain, and the pegylated heavy chain. The bands at 93 and 120kD have about the same intensity: this was expected, since only about 50% of N8 carries an O-glycan on its B domain.
Thus, the aldehyde handle has been successfully introduced on N8.

### Coupling of N8-(O)-PEG10kD-CHO to AP3-ONH2

N8-(O)-PEG10kD-CHO prepared according to intermediate example B was buffer shifted to 50mM imidazol buffer pH6.2 containing 10mM CaCl2, 0.02% Tween 80, 0.5M NaCl and upconcentrated by ultrafiltration (Millipore Centrifugal Filter Units Amicon Ultra, 0.5ml device, 50kD cut off) to a concentration of about 16mg/ml. Likewise, AP3-ONH2 prepared according to intermediate example A was buffer shifted to 50mM imidazol buffer pH6.2 containing 10mM CaCl2, 0.02% Tween 80, 0.5M NaCl and upconcentrated by ultrafiltration (Millipore Centrifugal Filter Units Amicon Ultra, 0.5ml device, 10kD cut off) to a concentration of about 34.7mg/ml.

To the upconcentrated AP3-ONH2 solution above (45µl, 1.56mg) was added the upconcentrated solution of N8-(O)-PEG10kD-CHO (21.5µl, 244µg), followed by the addition of an aqueous solution of aniline (5µl, 180µg). The mixture was incubated at 25°C overnight. The unreacted hydroxylamine moieties were quenched by addition of acetone (1.8µl, 1.4mg, about 600 molar equivalents). The reaction mixture was incubated 30min at 25°C.

The reaction mixture was diluted to 3ml with 20mM imidazole buffer pH7.3, 10mM CaCl2, 0.02% Tween 80, 1M glycerol, 25mM NaCl, and filtered (0.45µ Gelman GHP filter), and purified by ion exchange on MonoQ column 5/50 GL(GE Healthcare). The loading and washout buffer (buffer A) was 20mM imidazole buffer pH7.3, 10mM CaCl2, 0.02% Tween 80, 1M glycerol, 25mM NaCl, the elution buffer (buffer B) was 20mM imidazole buffer pH7.3, 10mM CaCl2, 0.02% Tween 80, 1M glycerol, 1M NaCl. The elution program consisted in 4 steps: 0 to 20%B over 4CV, 20%B over 10CV, 20 to 100%B over 16CV, 100% B over 5CV. The flow was 0.5ml/min, the temperature was 15C. 1ml fractions were recovered in the first two steps, 0.5ml fractions in the last two steps.

AP3-ONH2 eluted first at roughly 20%B, the remaining material eluted between 30 and 65%B as one major peak and several minor, not fully resolved peaks. After upconcentration (by ultrafiltration, 50kD cut off), the fractions were analyzed by gel electrophoresis using NuPage 7% Tris acetate gel (Invitrogen) (150V, 70min). The gel was coomassie blue stained using SimplyBlue SafeStain (Invitrogen). Standard proteins were from Invitrogen (HiMark Unstained HMW). The main peak from the material eluting between 30 and 65%B (peak top elutes at 52%B) corresponds to the conjugated material.

The unreduced conjugated material shows three bands on the gel at MW 84kD, 285kD, and >500kD (very faint band). They are assumed to correspond respectively to the (N8) light chain of N8-(O)-PEG10kD-AP3, the (N8) heavy chain of N8-(O)-PEG10kD-AP3 (for the control samples of unreduced N8-(O)-PEG10kD-CHO: the heavy chain appears at about 110kD, and the control samples of the unreduced AP3-ONH2 appears at about 176kD), and to an unidentified compound.

The reduced conjugated material shows three bands on the gel, at MW 84kD, 145kD and 170kD, assumed to correspond to respectively (N8) light chain of N8-(O)-PEG10kD-AP3 and N8 pegylated heavy chain conjugated to the light chain of AP3, and N8 pegylated heavy chain conjugated to the heavy chain of AP3. The amount of recovered conjugated material was 129µg, 53% yield from N8-PEG10kD-CHO.

### EXAMPLE 18

### Disodium (5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[3-(2-pyridyldisulfanyl)propanoylamino]acetyl]amino]-6-[(2R)-1,2,3-trihydroxypropyl]tetrahydropyran-2-carboxylate, compound 1M

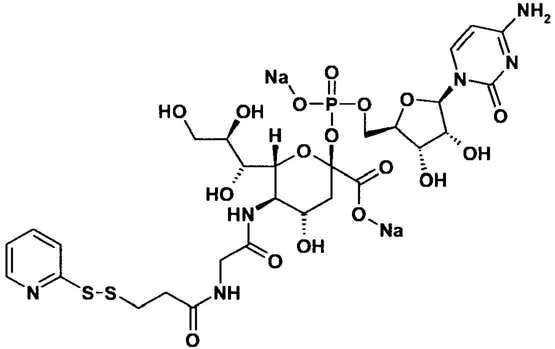

S,S'-2,2'-Dithiodipyridine (1.59 mmol, 350 mg) and 3-mercaptopropionic acid (0.477 mmol, 50.1 mg, 41.5 microliter) were mixed in tetrahydrofuran (2 ml). The mixture was stirred for 45 minutes. N-Hydroxysuccinimide (0.477 mmol, 54.9 mg) and N,N'-diisopropylcarbodimide (0.954 mmol, 148 microliter) were added to the mixture which was subsequently stirred for 3 h.

Disodium (5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[2-aminoacetylamino]-6-[(2R)-1,2,3-trihydroxypropyl]tetrahydropyran-2-carboxylate (0.159 mmol, 100 mg) was dissolved in water (500 microliter). pH was adjusted to 8.5 by addition of 500 mM aq. Na₂HPO₄ / NaH₂PO₄ (aq., sat.). The final volume was adjusted to 2 ml by addition of water.

The two solutions (NHS ester in tetrahydrofuran and nucleotide in water) were mixed. The resulting mixture was gently agitated for 3 h. The volume was adjusted to 20 ml by addition of water. The crude compound was purified using reversed phase HPLC (0-30 vol% acetonitrile in water, 10 % 500 mM aq. NH₄HCO₃, C₁₈ column). The selected fractions were pooled. Sodium hydroxide (1 M, 156 microliter) was added. The resulting mixture was lyophilised.
Purity and identity were determined by analytical HPLC and LCMS ([M+H]⁺: 827.4)

### EXAMPLE 19

### 2-[2-[4-[2-(3-hydroxy-6-oxo-xanthen-9-yl)benzoyl]piperazin-1-yl]-2-oxo-ethoxy]acetic acid, compound 2M

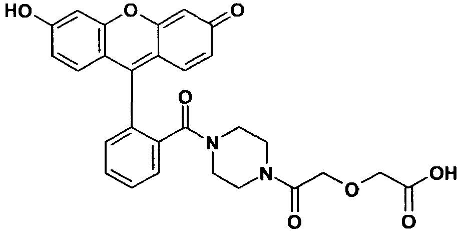

6-hydroxy-9-[2-(piperazine-1-carbonyl)phenyl]xanthen-3-one hydrochloride (5.72 mmol, 2.5 g) (prepared as described in J. Am. Chem. Soc., 2007, 129, 8400-8401) was suspended in a mixture of sat. aq. sodium bicarbonate (50 ml) and tetrahydrofuran (50 ml). The mixture was stirred for 10 minutes. Diglycolic anhydride (8.58 mmol, 1.0 g) was added. After 3 h, additional diglycolic anhydride (500 mg) was added. The mixture was stirred for 20 h. The mixture was acidified with fuming hydrochloric acid to pH 1. Dichloromethane (100 ml) and hydrochloric acid (1 M, 100 ml) were added. Brine (100 ml) and solid sodium chloride were added. A solid was observed in between the two phases. The phases were separated. The aq. phase was extracted with DCM (3x100 ml). The combined org. phases were dried (Na₂SO₄), filtered, and concentrated *in vacuo.*
The solid formed in between the phases was isolated by filtration.
The solid was suspended in hydrochloric acid (1 M) on a filter. The solids were continuously extracted with hydrochloric acid (1 M). The filtered extract was combined with the aqueous extracts and the resulting solution was purified using reversed phase HPLC (C₁₈ column, 10-35 vol% aceonitrile in water, 0.1 % trifluoroacetic acid). The selected fractions were pooled and lyophilised.
LCMS: 517.2776 [M+H]⁺

### EXAMPLE 20

### 2-[[2-[2-[4-[2-(3-hydroxy-6-oxo-xanthen-9-yl)benzoyl]piperazin-1-yl]-2-oxo-ethoxy]acetyl]-[2-[2-[2-[3-(2-pyridyldisulfanyl)propanoylamino]ethoxy]ethoxy]ethyl]amino]acetic acid, compound 3M

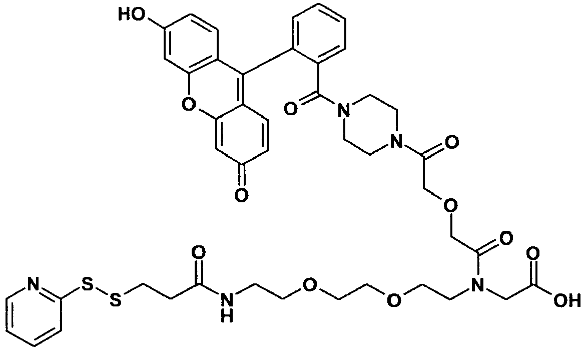

Trityl polystyrene resin (200 mg, 1.5 mmol/g, Pepchem) was placed in a fritted syringe. Thionyl chloride / dichloromethane (1:1, 5 ml) was added. The mixture was shaken for ½ h. The resin was washed with dichloromethane.
A mixture of bromoacetic acid (0.9 mmol, 125 mg) and *N,N'*-diisopropylethylamine (0.9 mmol, 156 microliter) in dichloromethane (3 ml) was added. After 5 minutes, more *N,N'*-diisopropyl-ethylamine (0.9 mmol, 156 microliter) was added. The mixture was shaken for ½ h. The resin was drained and an identical mixture of bromoacetic acid and *N,N'*-diisopropyl-ethylamine in dichloromethane was added to the resin. The mixture was shaken for 1½ h. The resin was washed with dichloromethane (5x), methanol, and *N-*methylpyrrolidone. During the methanol-wash vigorous shaking was done in order to suppress any formation of lumps.

A solution of 1,8-diamino-3,6-dioxaoctane (7.5 mmol, 1100 microliter) in N-methylpyrrolidone (3.75 ml) was added. The mixture was shaken for 3 h. The resin was washed. A solution of 2-acetyldimedone (3.0 mmol, 546 mg) in N-methylpyrrolidone (3 ml) was added to the resin. The mixture was shaken over night. The resin was washed with N-methylpyrrolidone. A mixture of 2-[2-[4-[2-(3-hydroxy-6-oxo-xanthen-9-yl)benzoyl]piperazin-1-yl]-2-oxo-ethoxy]acetic acid, compound 2M (155 mg, 0.3 mmol), 1-hydroxy-7-azabenzotriazole (0.9 mmol) and N,N'-diisopropylcarbodiimide (0.9 mmol, 140 microliter) in N-methylpyrrolidone (1.8 ml) was added to the resin. The mixture was shaken over night. The resin was washed with N-methylpyrrolidone (3-4 times). The resin was shaken in a mixture of hydrazine and N-methylpyrrolidone (5 vol% hydrazine mono hydrate) for 2x5 minutes. The syringe was drained. The resin was washed with N-methylpyrrolidone.

A mixture of S,S'-2,2'-dithiodipyridine (7.5 mmol, 1650 mg) and 3-mercaptopropionic acid (3.0 mmol, 261 microliter) in N-methylpyrrolidone (3 ml) was incubated for ½ h. N,N'-diisopropylcarbodiimide (1.5 mmol, 233 microliter) was added. The resulting mixture was added to the resin. The mixture was shaken for 1½ h. The resin was washed with N-methylpyrrolidone and dichloromethane.

The resin was treated with a mixture of trifluoroacetic acid and water (95:5) for 15 min. The filtrate was triturated with diethyl ether/n-heptane. The crude compound was dissolved in acetic acid (2½ ml). Water (15 ml) and a little acetonitrile were added. The mixture was filtered and purified using reversed phase HPLC (C₁₈ column, 20-50 % acetonitrile in water, 0.1 % TFA).
LCMS: 902.2807 [M+H]⁺

### EXAMPLE 21

### Diammonium (4S,5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[[2-[[2-[2-[4-[2-(3-hydroxy-6-oxo-xanthen-9-yl)benzoyl]piperazin-1-yl]-2-oxo-ethoxy]acetyl]-[2-[2-[2-[3-(2-pyridyldisul-fanyl)propanoylamino]ethoxy]ethoxy]ethyl]amino]acetyl]amino]acetyl] amino]-6-[(1R,2R)-1,2,3-trihydroxypropyl]tetrahydropyran-2-carboxylate, compound 4M

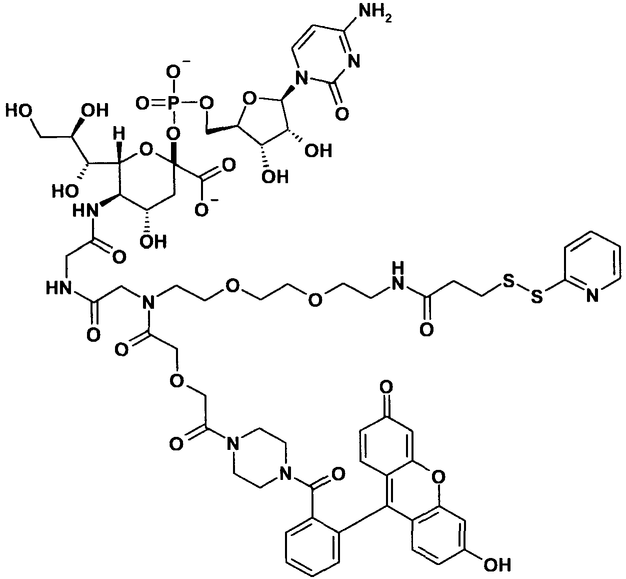

2-[[2-[2-[4-[2-(3-hydroxy-6-oxo-xanthen-9-yl)benzoyl]piperazin-1-yl]-2-oxo-ethoxy]acetyl]-[2-[2-[2-[3-(2-pyridyldisulfanyl)propanoylamino]ethoxy]ethoxy]ethyl]amino]acetic acid, compound 3M (0.3 mmol, 270 mg) was dissolved in tetrahydrofuran (2 ml). N-Hydroxysuccinimide (0.9 mmol, 104 mg) and N,N'-diisopropylcarbodiimide (1.5 mmol, 233 microliter) were added to the mixture which was subsequently stirred for1½ h.

Disodium (5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[2-aminoacetylamino]-6-[(2R)-1,2,3-trihydroxypropyl]tetrahydropyran-2-carboxylate (0.45 mmol, 303 mg) was dissolved in water (500 microliter). pH was adjusted to 8.5 by addition of 500 mM aq. Na₂HPO₄ / NaH₂PO₄ (aq., sat.). The final volume was adjusted to 2 ml by addition of water.

The two solutions were mixed. The resulting pH was 7.5-8.0. The resulting mixture was stirred for 20 h. The mixture was diluted to 15 ml with water, filtered, and purified using reversed phase HPLC (0-35 % acetonitrile in water, 10 % 500 mM NH₄HCO₃ aq.). The selected fractions were pooled and lyophilised. LCMS: 1513.9 [M+H]⁺, 757.5 [M+H]²⁺

### EXAMPLE 22

### Oregon Green 488 labelling of F8-500 AP3-LC-HC scFv -Δa3 via N-glycan, compound 5M

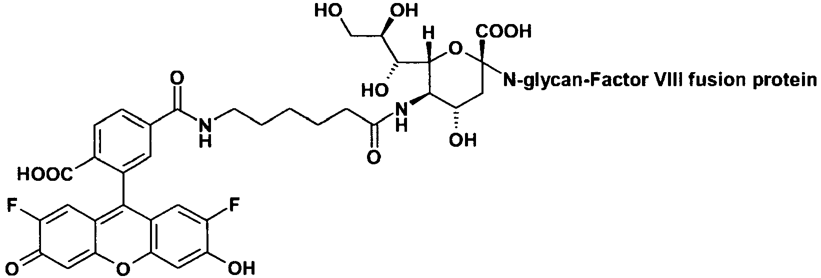

Fusion protein (F8-500 AP3-LC-HC scFv -Δa3 (SEQ ID NO 39), 11 microgram) in aqueous buffer (20 mM Imidazol, 10 mM CaCl₂, 0.02% Tween 80, 1 M glycerol, 500 mM NaCl, pH 7.3; 1.2 ml) was mixed with from recombinant sialidase from *Clostridium perfringens* (0.1 U). The final volume was 1.2 ml. The mixture was left at 25 degrees Celsius for 30 minutes. The mixture was diluted to 10 ml with buffer (20 mM Imidazol, 10 mM CaCl₂, 0.02% Tween 80, 1 M glycerol, pH 7.3). The mixture was loaded onto a pre-conditioned (with buffer A) MonoQ 5/50 GL column (GE Healthcare) and eluted using the following program: 10 CV eq 0 % Buffer B, 2 CV wash out unbound sample (0 % Buffer B), 10 CV 0 - 100 % (Buffer B), 5 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

The selected fractions were pooled and mixed with disodium (2R,5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-5-[6-[[4-carboxy-3-(2,7-difluoro-3-hydroxy-6-oxo-xanthen-9-yl)benzoyl]amino]hexanoylamino]-4-hydroxy-6-[(2R)-1,2,3-trihydroxypropyl]tetrahydropyran-2-carboxylate and sialyl transferase (rat rec. ST3GalIII). The final concentrations were: Factor VIII: 0.026 micromolar, fluorescence labelling reagent: 39 micromolar, ST3GalIII: 177 U/l. Final volume: 2.1 ml. The mixture was incubated at 32 degrees Celsius for 4 hours. Disodium (2R,5R,6R)-5-acetamido-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(2R)-1,2,3-trihydroxypropyl]tetrahydropyran-2-carboxylate was added to the mixture (final concentration: 54 micromolar). The mixture was incubated at 32 degrees Celsius for 3 hours. The mixture was diluted to 25 ml with buffer (20 mM Imidazol, 10 mM CaCl₂, 0.02% Tween 80, 1 M glycerol, no NaCl, pH 7.3). The mixture was loaded onto a pre-conditioned (with buffer A) MonoQ 5/50 GL column (GE Healthcare) and eluted using the following program: 10 CV eq , 5 CV wash out unbound sample, 10 CV 0 - 25 % buffer B, 10 CV 25-28 % (buffer B), 25 CV 28 - 100 % buffer B, and 10 CV 100 % buffer B. Imidazol Buffer A: 20 mM Imidazol, 10 mM CaCl₂, 0.02% Tween 80, 1 M glycerol, pH 7.3. Imidazol Buffer B: 20 mM Imidazol, 10 mM CaCl₂, 0.02% Tween 80, 1 M NaCl, 1 M glycerol, pH 7.3.

SDS-PAGE analysis (reduced, 7 % NuPAGE Tris acetate, 1.0 mm, Invitrogen, 150 V, 70 min) confirmed the incorporation of the fluorophor in the protein.

### EXAMPLE 23

### N,O-glycan-asialo-BDD Factor VIII, compound 6M

BDD factor VIII (1 mg) in aqueous buffer (20 mM Imidazol, 10 mM CaCl2, 0.02% Tween 80, 1 M glycerol, 500 mM NaCl, pH 7.3) was mixed with recombinant sialidase from *Arthrobacter Ureafaciens* (Biotechnol. Appl. Biochem., 2005, 41, 225-231). The final concentrations were: factor VIII: 5.7 mg/ml and sialidase: 1.5 U/ml. The mixture was left at 25 degrees Celsius for 30 minutes. The mixture was diluted to 20 ml with buffer (20 mM Imidazol, 10 mM CaCl₂, 0.02% Tween 80, 1 M glycerol, 25 mM NaCl, pH 7.3). The mixture was loaded onto a pre-conditioned (with buffer A) MonoQ 5/50 GL column (GE Healthcare) and eluted using the following program: 0.1 CV eq 5 % Buffer B, 2 CV wash out unbound sample (5 % Buffer B), 10 CV 5 - 100 % (Buffer B), 5 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

### EXAMPLE 24

### 3-(2-pyridyldisulfanyl)propanoylamino handle conjugated to BDD FVIII via O-glycan, compound 7M

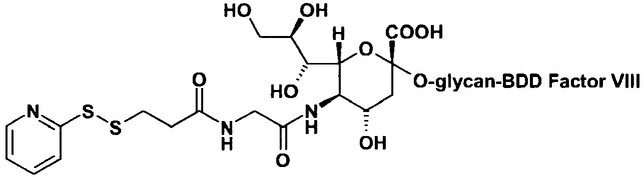

Asialo BDD factor VIII, compound 6 (3.95 mg) in buffer (20 mM Imidazol, 10 mM CaCl₂, 0.02% Tween 80, 1 M glycerol, 500 mM NaCl, pH 7.3) was mixed with disodium (5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[3-(2-pyridyldisulfanyl) propanoylamino]acetyl]amino]-6-[(2R)-1,2,3-trihydroxypropyl]tetrahydropyran-2-carboxylate and sialyl transferase (ST3GalI). The final concentrations were: factor VIII: 1.16 mg/ml, sialyltranferase: 0.22 mg/ml, nucleotide: 250 micromolar. Final volume: 3.4 ml. The mixture was left at 32 degrees Celsius for 20 hours. Disodium (2R,5R,6R)-5-acetamido-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(2R)-1,2,3-trihydroxypropyl]tetrahydropyran-2-carboxylate was added to the mixture (final concentration: 54 micromolar). The mixture was incubated at 32 degrees Celsius for 30 minutes. The mixture was diluted to 25 ml with buffer (20 mM Imidazol, 10 mM CaCl₂, 0.02% Tween 80, 1 M glycerol, 25 mM NaCl, pH 7.3).

The mixture was loaded onto a pre-conditioned (with buffer A) MonoQ 5/50 GL column (GE Healthcare) and eluted using the following program: 1 CV eq 0 % Buffer B, 2 CV wash out unbound sample (0 % Buffer B), 10 CV 0 - 20 % Buffer B, 10 CV 20 % Buffer B, 10 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

The isolated protein was mixed with sialyl tranferase (ST3GalIII) and disodium (2R,5R,6R)-5-acetamido-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(2R)-1,2,3-trihydroxypropyl]tetrahydropyran-2-carboxylate. The final concentrations were: factor VIII: 1.12 mg/ml, sialyltranferase: 0.13 mg/ml, nucleotide: 54 micromolar. Final volume: 3.2 ml. The mixture was left at 32 degrees Celsius for 1 hour.

The mixture was diluted to 40 ml with buffer (20 mM Imidazol, 10 mM CaCl₂, 0.02% Tween 80, 1 M glycerol, 25 mM NaCl, pH 7.3). The mixture was loaded onto a pre-conditioned (with buffer A) MonoQ 5/50 GL column (GE Healthcare) and eluted using the following program: 5 CV wash out unbound sample (0 % Buffer B), 5 CV 0 - 20 % Buffer B, 15 CV 20 % Buffer B, 10 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

Incubation of the product with PEG 30 kDa thiol prior to SDS-PAGE analysis resulted in presence of a band with MW higher that FVIII under non-reduced conditions but not under reduced conditions.

### EXAMPLE 25

### Fluorescent 2-pyridyldisulfanyl handle conjugated to AP3 full length antibody via N-glycan, compound 8M

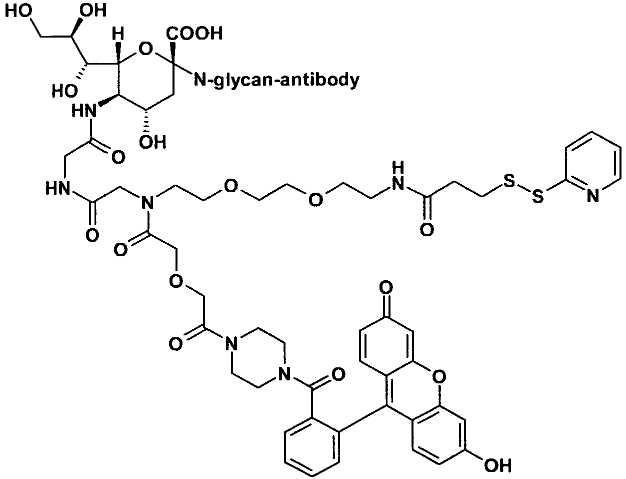

AP3 mlgG1 wt HC LC C39S full length antibody (1.5 mg) in aqueous buffer (50 % 100 mM HEPES.HCl, 150 mM NaCl, pH 7.5, 33 % 100 mM Glycine.HCl, pH 3.5, 17 % 20 mM HEPES.HCl, 150 mM NaCl, 0,01 % Tween80, pH 7.5 - final pH 7.2) was mixed with diammonium (4S,5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[[2-[[2-[2-[4-[2-(3-hydroxy-6-oxo-xanthen-9-yl)benzoyl]piperazin-1-yl]-2-oxo-ethoxy]acetyl]-[2-[2-[2-[3-(2-pyridyldisulfanyl)propanoylamino]ethoxy] ethoxy] ethyl]amino]acetyl]amino]acetyl]amino]-6-[(1R,2R)-1,2,3-trihydroxypropyl] tetrahydropyran-2-carboxylate, compound 4M and sialyl tranferase (ST3GalIII). The final concentrations were: Antibody: 1.4 mg/ml, sialyltranferase: 0.12 mg/ml, nucleotide: 190 micromolar. Final volume: 650 microliter. The mixture was left at 32 degrees Celsius for 19 hours.
The sample was transferred to a Millipore Amicon Ultra Centrifugal device, MWCO 10.000 Da. The protein was washed several times with buffer (Histidine, 1.5 mg/ml, CaCl₂, 0.25 mg/ml, Tween 80, 0.1 mg/ml, NaCl, 18 mg/ml, Sucrose 3 mg/ml, pH 7.0. The solution was concentrated to 100 microliter in said buffer. The absorbance at 280 nm and 500 nm were determined to be SDS-PAGE analysis (reduced and non-reduced, 7 % NuPAGE Tris acetate, 1.0 mm, Invitrogen, 150 V, 70 min) confirmed the incorporation of the fluorophor in antibody light chain.

### EXAMPLE 26

### Fluorescent 2-pyridyldisulfanyl handle conjugated to BDD FVIII via O-glycan, compound 9M

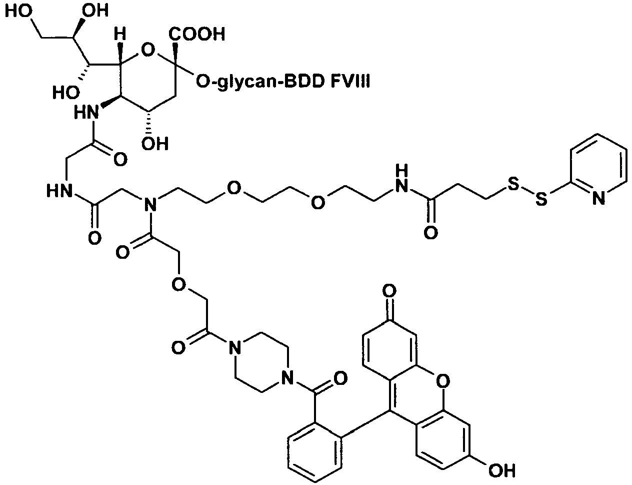

Asialo BDD factor VIII, compound 6M (1.86 mg) in buffer (20 mM Imidazol, 10 mM CaCl2, 0.02% Tween 80, 1 M glycerol, 500 mM NaCl, pH 7.3) was mixed with diammonium (4S,5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[[2-[[2-[2-[4-[2-(3-hydroxy-6-oxo-xanthen-9-yl)benzoyl]piperazin-1-yl]-2-oxo-ethoxy]acetyl]-[2-[2-[2-[3-(2-pyridyldisulfanyl)propanoylamino]ethoxy]ethoxy]ethyl] amino]acetyl]amino] acetyl]amino]-6-[(1R,2R)-1,2,3-trihydroxypropyl]tetrahydropyran-2-carboxylate, compound 4M and sialyl transferase (ST3GalI). The final concentrations were: factor VIII: 1.5 mg/ml, sialyltranferase: 0.3 mg/ml, nucleotide: 95 micromolar. Final volume: 1.25 ml. The mixture was left at 32 degrees Celsius for 15 hours. Disodium (2R,5R,6R)-5-acetamido-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(2R)-1,2,3-trihydroxypropyl]tetrahydropyran-2-carboxylate (final concentration: 2.5 mM) and ST3GalIII were added to the mixture. The mixture was incubated at 32 degrees Celsius for 30 minutes. The mixture was diluted to 25 ml with buffer (20 mM Imidazol, 10 mM CaCl₂, 0.02% Tween 80, 1 M glycerol, 150 mM NaCl, pH 7.3).

The mixture was loaded onto a pre-conditioned (with buffer A) MonoQ 5/50 GL column (GE Healthcare) and eluted using the following program: 1 CV eq 0 % Buffer B, 2 CV wash out unbound sample (0 % Buffer B), 10 CV 0 - 20 % Buffer B, 10 CV 20 % Buffer B, 10 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

SDS-PAGE analysis (reduced and non-reduced, 7 % NuPAGE Tris acetate, 1.0 mm, Invitrogen, 150 V, 70 min) confirmed the incorporation of the fluorophor in BDD FVIII heavy chain.

### EXAMPLE 27

### 3-[2-[2-[2-(2-acetylsulfanylethoxy)ethoxy]ethoxy]ethoxy]propanoyl handle conjugated to AP3 full length antibody, random positions, compound 10 M

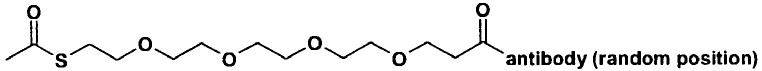

AP3 mlgG1 wt Hc Lc C39S full length antibody (1.3 mg) in buffer (50 % 100 mM HEPES.HCl, 150 mM NaCl, pH 7.5, 33 % 100 mM Glycine.HCl, pH 3.5, 17 % 20 mM HEPES.HCl, 150 mM NaCl, 0,01 % Tween80, pH 7.5 - final pH 7.2) was placed in a Millipore Amicon Ultra Centrifugal device, MWCO 10.000 Da. The protein was washed several times with buffer (20 mM HEPES, pH 7.3). The solution was concentrated to 65 microliter in said buffer.

(2,5-dioxopyrrolidin-1-yl) 3-[2-[2-[2-(2-acetylsulfanylethoxy)ethoxy]ethoxy] ethoxy]propanoate (Pierce/Thermo Scientific, 2.2 mg, 5.2 micromol) was dissolved in buffer (20 mM HEPES, pH 7.3, 100 microliter). 4 microliter of this solution (209 nmol) was added to the protein solution. The resulting mixture was incubated for 1 hour. The solution was placed in a Millipore Amicon Ultra Centrifugal device, MWCO 10.000 Da. The protein was washed several times with buffer (20 mM HEPES, pH 7.3).

### EXAMPLE 28

### N-glycan-asialo-BDD Factor VIII, compound 11M

BDD factor VIII (7 mg) in aqueous buffer (20 mM Imidazol, 10 mM CaCl2, 0.02% Tween 80, 1 M glycerol, 500 mM NaCl, pH 7.3) was mixed with recombinant sialidase from recombinant sialidase from *Clostridium perfringens* (4 U) . The final volume was 4 ml. The mixture was left at 25 degrees Celsius for 30 minutes. The mixture was diluted to 5 ml with buffer (20 mM Imidazol, 10 mM CaCl₂, 0.02% Tween 80, 1 M glycerol, 25 mM NaCl, pH 7.3). The mixture was loaded onto a pre-conditioned (with buffer A) MonoQ 5/50 GL column (GE Healthcare) and eluted using the following program: 5 CV eq 0 % Buffer B, 2 CV wash out unbound sample (0 % Buffer B), 25 CV 0 - 70 % (Buffer B), 10 CV 70-100 % (Buffer B), 5 CV 100 % Buffer B. Buffer A: Histidine (1.5 mg/ml), CaCl₂ (0.25 mg/ml), Tween 80 (0.1 mg/ml), NaCl (2.9 mg/ml), and sucrose (3 mg/ml), pH 7.0. Buffer B: Histidine (1.5 mg/ml), CaCl₂ (0.25 mg/ml), Tween 80 (0.1 mg/ml), NaCl (58 mg/ml), and sucrose (3 mg/ml), pH 7.0

### EXAMPLE 29

### 3-(2-pyridyldisulfanyl)propanoylamino handle conjugated to fluorescence labelled BDD FVIII via N-glycan, compound 12M

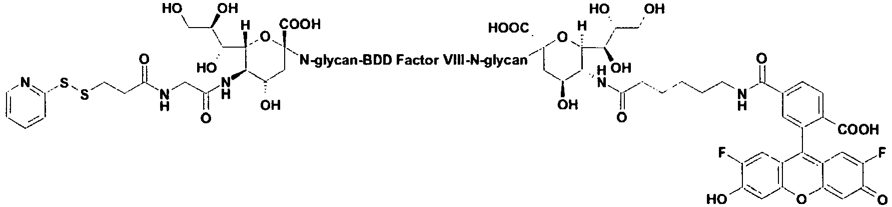

N-glycan-asialo-BDD Factor VIII, compound 11M (3 mg) in buffer (Histidine (1.5 mg/ml), CaCl2 (0.25 mg/ml), Tween 80 (0.1 mg/ml), NaCl (30 mg/ml), and sucrose (3 mg/ml), pH 7.0) was mixed with disodium (5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-5-[[2-[3-(2-pyridyldisulfanyl)propanoylamino]acetyl]amino]-6-[(2R)-1,2,3-trihydroxypropyl]tetrahydropyran-2-carboxylate, compound 1M, disodium (2R,5R,6R)-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-5-[6-[[4-carboxy-3-(2,7-difluoro-3-hydroxy-6-oxo-xanthen-9-yl)benzoyl]amino]hexanoylamino]-4-hydroxy-6-[(2R)-1,2,3-trihydroxypropyl]tetrahydropyran-2-carboxylate and sialyl transferase (rat rec. ST3GalIII). The final concentrations were: factor VIII: 0.85 mg/ml, sialyltranferase: 271 U/l, Oregon Green 488 nucleotide: 14 micromolar, compound 1M: 15 micromolar. Final volume: 3.5 ml. The mixture was left at 32 degrees Celsius for 18 hours. Disodium (2R,5R,6R)-5-acetamido-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(2R)-1,2,3-trihydroxypropyl]tetrahydropyran-2-carboxylate was added to the mixture (final concentration: 54 micromolar). The mixture was incubated at 32 degrees Celsius for 30 minutes.

The mixture was diluted to 14 ml with buffer (Histidine (1.5 mg/ml), CaCl₂ (0.25 mg/ml), Tween 80 (0.1 mg/ml), NaCl (2.9 mg/ml), and sucrose (3 mg/ml), pH 7.0).
The mixture was loaded onto a pre-conditioned (with buffer A) MonoQ 5/50 GL column (GE Healthcare) and eluted using the following program: 5 CV eq, 2 wash out unbound sample, 25 CV 0 - 70 %, 10 CV 70-100 % buffer B, and 5 CV 100 % B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

SDS-PAGE analysis (reduced and non-reduced, 7 % NuPAGE Tris acetate, 1.0 mm, Invitrogen, 150 V, 70 min) confirmed the incorporation of the fluorophor in BDD FVIII heavy and light chains. Incubation of the product with PEG 30 kDa thiol prior to SDS-PAGE analysis resulted in presence of a fluorescent band with MW higher that FVIII under non-reduced conditions but not under reduced conditions.

### EXAMPLE 30

### Conjugate between 3-(2-pyridyldisulfanyl) propanoylamino handle conjugated to fluorescence labelled BDD FVIII via N-glycan, compound 12M and AP3 scFv LC-HC, compound 13M

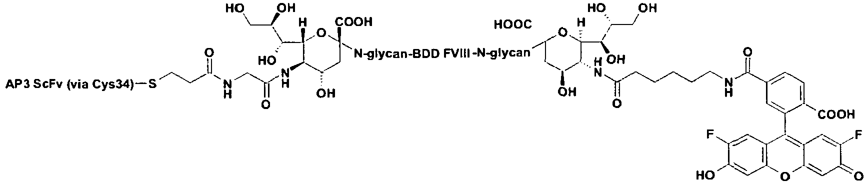

AP3 scFv in a 1:1 mixture of glycine buffer (100 mM, pH 3.5) and HEPES buffer (100 mM, pH 7.5) was mixed with 3-(2-pyridyldisulfanyl) propanoylamino handle conjugated to fluorescence labelled BDD FVIII via N-glycan, compound 12M in aqueous buffer (20 mM Imidazol, 10 mM CaCl2, 0.02% Tween 80, 1 M glycerol, 500 mM NaCl, pH 7.3). The final concentrations were: compound 12M: 0.25 mg/ml, ScFv: 2 micromolar, 0.06 mg/ml. Final volume: 2.1 ml. The mixture was left at 23 degrees Celsius for 3 hours. Solutions of Cysteine (1 mg/ml) in buffer (2.3 microliter) and Cystine (1 mg/ml) in buffer (3.5 microliter) were added. The mixture was left at 23 degrees Celsius for 15 minutes.
The mixture was diluted to 40 ml with buffer (20 mM Imidazol, 10 mM CaCl₂, 0.02% Tween 80, 1 M glycerol, 25 mM NaCl, pH 7.3). The mixture was loaded onto a pre-conditioned (with buffer A) MonoQ 5/50 GL column (GE Healthcare) and eluted using the following program: 5 CV eq 0 % Buffer B, 2 CV wash out unbound sample (0 % Buffer B), 25 CV 0 - 70 % (Buffer B), 50 CV 70-100 % (Buffer B), 5 CV 100 % Buffer B. Imidazol Buffer A: 20 mM Imidazol, 10 mM CaCl₂, 0.02% Tween 80, 25 mM NaCl, 1 M glycerol, pH 7.3. Imidazol Buffer B: 20 mM Imidazol, 10 mM CaCl₂, 0.02% Tween 80, 1 M NaCl, 1 M glycerol, pH 7.3.

The selected fractions were transferred to a Millipore Amicon Ultra Centrifugal device, MWCO 50.000 Da and the total volume was reduced to 0.5 ml. The mixture was loaded onto onto a pre-conditioned (with buffer) Superose 6 10/300 GL column (GE Healthcare) and eluted using the following buffer: Histidine (1.5 mg/ml), CaCl₂ (0.25 mg/ml), Tween 80 (0.1 mg/ml), NaCl (18 mg/ml), and sucrose (3 mg/ml), pH 7.0.

SDS-PAGE analysis (non-reduced as well as thrombin digested/non-reduced, 7 % NuPAGE Tris acetate, 1.0 mm, Invitrogen, 150 V, 70 min) showed the presence of fluorescent bands with higher MW than the starting material. The protein was shown not bind to platelets which could be rationalised by the fact that Cys-34 is found in a CDR region, hence effecting binding affinity.

### EXAMPLE 31

### Conjugate between fluorescent 3-(2-pyridyldisulfanyl) handle conjugated AP3 mlgG1 wt HC LC C39S full length antibody via N-glycan, compound 8M and BDD-FVIII via handle on O-glycan, compound 7M

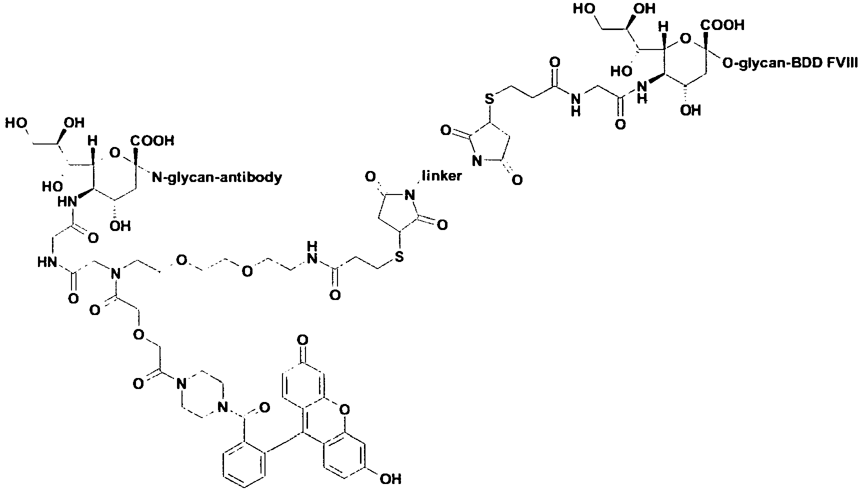

AP3 mlgG1 wt Hc Lc C39S full length antibody conjugated to a handle, e.g., compound 8M in buffer is mixed with a solution of tris(carboxyethyl)phosphine in buffer. The buffer is exchanged in order to remove excess trialkylphosphine after selective reduction of the disulfide bond. A solution of a bis-maleimide, e.g., bis-maleimide PEG 6000 from Rapp Polymere Gmbh, cat. No.: 11 6000-45, in buffer is added. The buffer is exchanged after completion of the reaction.

FVIII conjugated to a 3-(2-pyridyldisulfanyl)propanoylamino handle, e.g., compound 7M, in buffer is mixed with a solution of tris(carboxyethyl)phosphine in buffer. The buffer is exchanged in order to remove excess trialkylphosphine after selective reduction of the disulfide bond.

The two solutions of modified proteins (FVIII and AP3 Ab) are mixed. The formation of the desired conjugate is monitored by SDS-PAGE analysis. Purification is accomplished using a suitable type of chromatography, e.g., ion-exchange or anti-FVIII affinity chromatography.

### EXAMPLE 32

### Conjugate between fluorescent 3-(2-pyridyldisulfanyl) handle conjugated AP3 mlgG1 wt HC LC C39S full length antibody via N-glycan, compound 8M and BDD-FVIII via handle on O-glycan, compound 7M

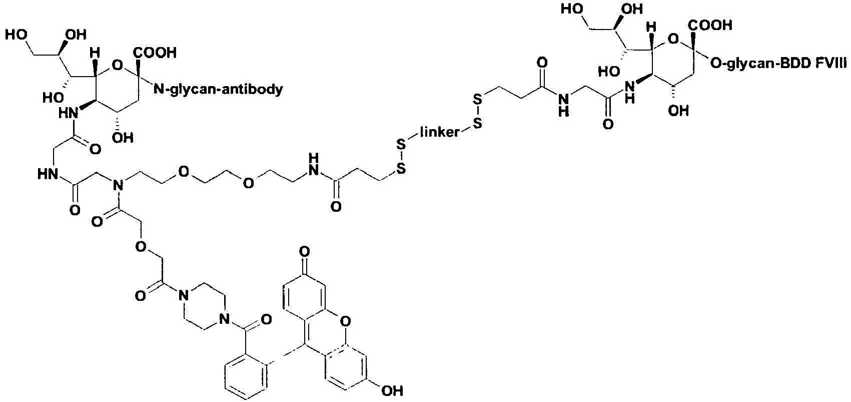

AP3 mlgG1 wt Hc Lc C39S full length antibody conjugated to a handle, e.g., compound 8M in buffer is mixed with a solution of a dithiol, e.g., 3,6-dioxa-1,8-octanedithiol, in buffer. The buffer is exchanged after completion of the reaction. A solution of 3-(2-pyridyldisulfanyl)propanoylamino handle conjugated to FVIII, e.g., compound 7M, is added. The formation of the desired conjugate is monitored by SDS-PAGE analysis. Purification is accomplished using a suitable type of chromatography, e.g., ion-exchange or anti-FVIII affinity chromatography.

### EXAMPLE 33

### Conjugate between fluorescent 3-(2-pyridyldisulfanyl) handle conjugated AP3 mlgG1 wt HC LC C39S full length antibody via N-glycan, compound 8M and Cys mutant of FVIII

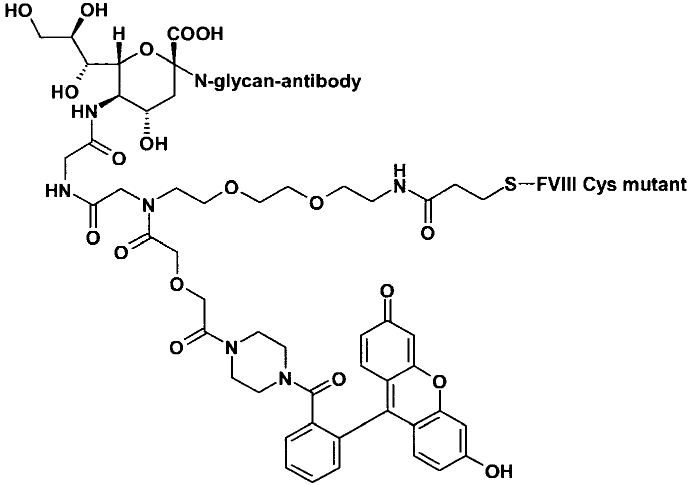

A surface accessible Cys mutant of FVIII is conjugated to AP3 antibody or similar (or fragment thereof) using the same methods as described in above. The Cys-mutant of FVIII is, if necessary, treated with reduction agent in order to remove substituents covalently bound to the mutated Cystein, thus resulting in the generation / ensuring the presence of a thiol. The formed protein is conjugated to a suitable reagent of the type described, e.g., Fluorescent **2-pyridyldisulfanyl** handle conjugated to AP3 full length antibody via N-glycan, compound 8M. The formation of the desired conjugate is monitored by SDS-PAGE analysis. Purification is accomplished using a suitable type of chromatography, e.g., ion-exchange or anti-FVIII affinity chromatography.

### Example 34

### Preparation of FVIII modified with Oregon Green 488 on the N-glycans and dPEG12-SH on the O-glycan

### Step 1: Preparation of PySS-dPEG₁₂-GSC (compound J1)

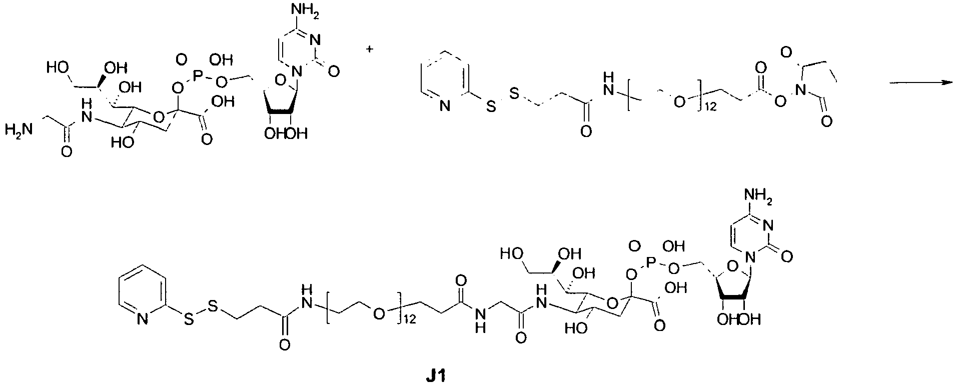

Glycyl sialic acid cytosine 5'-monophosphate ester (GSC, dimethylamine salt, MW 673, 85% pure, 96 mg, 153 µmol) was dissolved in 100 mM TRIS buffer pH 8.4 (650 µL) followed by acetonitrile (650µL) and stirred to observe a two-phase system. SPDP-dPEG₁₂-NHS-ester (Quanta Biodesign, prod no 10378, 1.2 eq, 183 µmol, 170 mg) was dissolved in 650 µL of THF, followed by 100 mM TRIS, pH 8.4, (650 µL) mild white hazy precipitate was observed and this NHS-ester solution was added to the GSC solution. The solution was slightly hazy at pH 8.4. The solution was stirred at room temperature for 7 h and the resulting mixture was frozen and lyophilized to get the crude compound. The white powder obtained after lyophilisation was purified by preparative HPLC using neat water as equilibration buffer and a linear gradient of neat acetonitrile for elution. Fractions containing the desired product were pooled and lyophilized to yield 100 mg of the target compound, which was homogeneous by analytical HPLC and identified by mass spectrometry.

### Step 2: Coupling of PySS-dPEG₁₂-GSC to the O-glycan of wt BDD FVIII (compound J2)

To wt BDD FVIII ("N8", 5.7 mg/ml; 5 mg, 875µl) was added sialidase *A. urifaciens* (0.44 mg/ml, 50 µl) and PySS-dPEG₁₂-GSC (**J1**), 1 mg/ml in buffer 20 mM imidazol, 10 mM CaCl₂, 0.02% Tween 80, 200 mM NaCl, 1 M glycerol, pH 7.3, 100 ul was used) and His-ST3Gal-I (2.5 mg/ml, 375 µl). The reaction mixture was incubated at 32°C overnight for a period of 17 h. It was then diluted with a 25 ml buffer 20 mM imidazol, 10 mM CaCl₂, 0.02% Tween 80, 25 mM NaCl, 1 M glycerol, pH 7.3 to lower the conductivity. The mixture was then loaded to a MonoQ 5/50 GL column equilibrated with 20 mM imidazol, 10 mM CaCl₂, 0.02% Tween 80, 25 mM NaCl, 1 M glycerol, pH 7.3. The product was eluted using a two-step-gradient with elution buffer 20 mM imidazol, 10 mM CaCl₂, 0.02% Tween 80, 1 M NaCl, 1 M glycerol, pH 7.3. FVIII-unrelated material was eluted with 20% elution buffer, while the product was eluted with 100% elution-buffer. Fractions containing the desired product (**J2**) were pooled. Yield 4.1 mg, 1.17 mg/ml.

### Step 3: Labelling with Oregon Green and capping of the N-glycans (J3)

The modified protein prepared according to Step 2 (**J2**) was mixed with MBP-ST3Gal-III (1.2 U/ml, 300 µl) and Oregon Green 488-GSC (2 mg was dissolved in 800 µl buffer 20 mM imidazol, 10 mM CaCl₂, 0.02% Tween 80, 200 mM NaCl, 1 M glycerol, pH 7.3; 200 µl, 0.5 mg, 20 eq. was used) and incubated in the dark at 32 C overnight. Subsequently, CMP-NAN (9 mg in 250 µl buffer 20 mM Imidazol, 10 mM CaCl₂, 0.02% Tween 80, 200 mM NaCl, 1 M glycerol, pH 7.3) was added to the reaction mixture and incubated for further 1 hour at 32°C. It was then diluted with 30 ml 20 mM imidazol, 10 mM CaCl₂, 0.02% Tween 80, 25 mM NaCl, 1 M glycerol, pH 7.3 to lower the conductivity. Purification was carried out by AIEX as outlined in Step 2. Yield: 1.54 mg, 0.51 mg/ml.

### Step 4: Reduction with TCEP

The modified protein prepared according to Step 3 (**J3**) was mixed with triscarboxylethyl phosphine (TCEP, 700 eq., 1.69 mg; 6 mg TCEP was dissolved in buffer 1 ml, 20 mM imidazol, 10 mM CaCl₂, 0.02% Tween 80, 200 mM NaCl, 1 M glycerol, pH 7.3, 282 µl was used) and incubated for 30 min at 5 C in the dark. It was then diluted with 30 ml 20 mM imidazol, 10 mM CaCl₂, 0.02% Tween 80, 25 mM NaCl, 1 M glycerol, pH 7.3 to lower the conductivity. Purification was carried out by AIEX as outlined in Step 2. Yield: 610 µg, 610 µg/ml. The product was further purified by SEC (column: Superdex 200 10/300 GL) using buffer histidine (1.5 mg/ml), CaCl₂ (0.25 mg/ml), Tween 80 (0.1 mg/ml), NaCl (18 mg/ml), sucrose (3 mg/ml) as mobile phase. Fractions containing the desired product (**J4**) were pooled. Yield: 420 µg, 120 µg/ml. Data from SDS PAGE gel analysis of the product were essentially identical to N8 and gel fluorescence imaging showed that the HC and LC both contained fluorescence label. The product was subjected to a test reaction using a 30 kDa PEG-maleimide. SDS PAGE analysis of this reaction showed bands with increased MW and disappearance of the FVIII HC band, thus indicating the presence of of the free thiol group.

This example shows that FVIII can be modified on the O-glycan with a thiol group.

### EXAMPLE 35

### Preparation of full length AP3 mIgG antibody modfied with -SSPy groups (J5)

Full length AP3 mlgG1 wt LC C39S (3.0 mg/ml, 400 µl, 1200 µg, 8 nmol, pH 7,2, in a mixture of buffers 50 % 100 mM HEPES HCl, 150 mM NaCl, pH 7.5, 33 % 100 mM Glycine HCl, pH 3.5, and 17 % 20 mM HEPES HCl, 150 mM NaCl, 0,01 % Tween80, pH 7.5) was buffer exchanged to HEPES 100 mM, pH 7.5 by concentrating to 100 µl (15 min, 12.000 g, Millipore Amicon Ultra 10 kDa), diluting to 900 µl and then concentrate to 100 ul (12 min, 12.000 g) x 6. Final volume was ca. 80 µl. It was then diluted to ca. 260 µl using HEPES 100 mM pH 7.5. Subsequently, SPDP-dPEG₁₂-NHS-ester (Quanta Biodesign, prod no. 10378, 1.8 mg was dissolved in 400 µl ml HEPES 100 mM, pH 7.5; from this, 20 eq., 144 µg, 32 µl was used) was added to the buffer exchanged AP3 FL mIgG. The reaction mixture was incubated at r.t. overnight. Reduced and un-reduced SDS PAGE gel analysis showed that the bands corresponding to FL antibody and the individual heavy and light chains were increased in molecular weight, as well as being broader in appearence, thus showing that the desired modication had taken place. The protein was buffer exchanged to HEPES 100 mM, pH 7.5 as described above. Yield 765 µg, 8.5 mg/ml.

This example shows that AP3 FL mIgG can be modified with an acylation agent containing thiol sensitive -SSPy conjugation group.

### EXAMPLE 36

### Preparation of a conjugate between FVIII and AP3 FL mIgG

AP3 mIgG1 dPEG₁₂-SSPy (**J5**, 8.5 µg/µl, 0.057 nmol/µl, 31 µl, 1.76 nmol) was mixed with 2500 µl of the FVIII derivative **J4** (300 µg, 1.76 nmol, 120 µg/ml). The mixture was sterile-filtered, wrapped in alu foil and left at r.t. overnight after which SDS PAGE gel analysis showed that products with MW corresponding to the desired adduct(s) between wt BDD FVIII and AP3 FL mIgG had formed. Gel fluorescence imaging showed that these bands contained fluorescence label, showing that the originated from the FVIII HC or LC chains. Gel analysis was also performed in the presence of excess N-ethylmaleimide to verify that the conjugation did not happen during SDS sample preparation. The mixture was allowed to react for an additional 3 days, after which it was diluted with 30 ml 20 mM imidazol, 10 mM CaCl2, 0.02% Tween 80, 25 mM NaCl, 1 M glycerol, pH 7.3 and loaded to a MonoQ 5/50 GL column equilibrated with 20 mM Imidazol, 10 mM CaCl2, 0.02% Tween 80, 25 mM NaCl, 1 M glycerol, pH 7.3. The product was eluted using a linear gradient with elution buffer 20 mM Imidazol, 10 mM CaCl2, 0.02% Tween 80, 1 M NaCl, 1 M glycerol, pH 7.3. In this manner, the free AP3 FL mIgG was separated from free BDD FVIII and the conjugate. Reduced and non-reduced SDS PAGE analysis showed that a few fractions contained the desired product. The non-reduced gel indicated a disappearance of the FVIII HC band while showing high MW bands corresponding to conjugates between AP3 FL mIgG and FVIII. Western blot with mIgG specific Ab showed that these bands contained the AP3 FL antibody. The reduced gel showed the presence of both FVIII and AP3 heavy and light chains.

This example shows that AP3 FL mIgG and BDD FVIII can be conjugated via a disulfide linkage formed by the reaction between a thiol group on the AP3 FL mIgG and an -SSPy group on the BDD FVIII.

### EXAMPLE 37

### FVIII:C in cell culture harvests measured by chromogenic assay

The FVIII activity (FVIII:C) of the rFVIII compound in cell culture harvest (supernatant fraction) was evaluated in a chromogenic FVIII assay using Coatest SP reagents (Chromogenix) as follows: rFVIII samples and a FVIII standard (Coagulation reference, Technoclone) were diluted in Coatest assay buffer (50 mM Tris, 150 mM NaCl, 1 % BSA, pH 7.3, with preservative). Fifty µl of samples, standards, and buffer negative control were added to 96-well microtiter plates (Spectraplates MB, Perkin Elmer). All samples were tested diluted 1:100, 1:400, 1:1600, and 1:6400. The factor IXa/factor X reagent, the phospholipid reagent and CaCl₂ from the Coatest SP kit were mixed 5:1:3 (vol:vol:vol) and 75 µl of this added to the wells. After 15 min incubation at room temperature, 50 µl of the factor Xa substrate S-2765/thrombin inhibitor I-2581 mix was added and the reactions were incubated 5 min at room temperature before 25 µl 1 M citric acid, pH 3, was added. The absorbance at 405 nm was measured on an Envision microtiter plate reader (Perkin Elmer) with absorbance at 620 nm used as reference wavelength. The value for the negative control was subtracted from all samples and a calibration curve prepared by linear regression of the absorbance values plotted vs. FVIII concentration. The specific activity was calculated by dividing the activity of the samples with the protein concentration determined by ELISA.

### EXAMPLE 38

### Specific activity of AP3-FVIII fusion-proteins in clinically relevant FVIII activity assays

FVIII activity of purified proteins and conjugates (N8 control, F8-500 AP3-LC-HC scFV-Δa3 (SEQ ID NO 39), F8-500 AP3-LC-HC scFV (SEQ ID NO 42) and F8-500 AP3-HC-LC scFV (SEQ ID NO 41)) was evaluated in standard FVIII:C assays, either chromogenic assays or in a one-stage clotting assay.
Chromogenic FVIII activity of purified proteins was assessed using the the Coatest SP assay (Chromogenix, Lexington, MA, USA). The assay was preformed according to manufacturer's instructions with a few minor modifications and run in a 96-well plate format. Briefly, diluted FVIII samples and FVIII reference material were incubated with a mixture of factor IXa/factor X reagent, phospholipid reagent and CaCl₂ from the Coatest SP kit. After 15 min incubation at room temperature the factor Xa substrate S-2765/thrombin inhibitor I-2581 mix was added and the reactions incubated 10 min at room temperature before the reaction was stopped with 20% citric acid. The absorbance at 415 nm was measured on a Spectramax microtiter plate reader (Molecular Devices) with absorbance at 620 nm used as reference wavelength. A recombinant FVIII calibrated against the 7th WHO/NIBSC recombinant FVIII standard was used as reference material. See table 4

### EXAMPLE 39

### FVIII:C in purified samples measured by one-stage clot assay

The FVIII clotting activity of the FVIII compounds was determined by diluting the concentrated FVIII samples and reference material first in buffer and then in FVIII-deficient plasma with von Willebrand Factor (Siemens, Deerfield, IL, USA) according to SSC recommendations. Each FVIII sample was measured in 4 different concentrations. The FVIII clotting activity was measured on an ACL 9000 instrument using the single factor program (FVIII:Cd) where FVIII samples were mixed with APTT reagent (Synthasil, ILS, Bedford, MA, USA), 25 mM CaCl2 and FVIII-deficient plasma. A recombinant FVIII calibrated against the 7th WHO/NIBSC recombinant FVIII standard was used as reference material. S

**Table 4**

| **Compound** | **Specific Clot Activity (IU/mg)** | **Specific Chromogenic Activity (IU/mg)** |
|---|---|---|
| N8-AP3 IgG conjugate Compound MZ1 | n.d. | **2687 ± 177** |
| F8-500 AP3 HC-LC | 6867 ± 101 (n=2) | 6837 ± 1241 (n=2) |
| F8-500 AP3-LC-HC scFV | 5565 ± 69 (n=2) | 5677 ± 1049 (n=2) |
| F8-500 AP3-LC-HC scFV-Δa3 | 13528 ± 2205 (n=3) | 10554 ± 1410 (n=3) |
| N8 control | 8533 ± 638 (n=5) | 7769 ± 692 (n=5) |

As show in table 4 AP3-FVIII fusion proteins and conjugates have similar FVIII activities in FVIII Chromogenic- and Clotting activity assays as the N8 starting material.

### EXAMPLE 40

### Thrombin generation supported by AP3-N8 fusion-proteins specifically bound to platelets

Global Coagulation tests such as Thrombin Generation Test (TGT) have been suggested to be more physiologically relevant measurements of factor VIII (FVIII) activity than clotting and chromogenic assays especially when platelets are included in the assays. In order to assess the effect of AP3-N8 on platelets the FVIII compounds (F8-500 AP3-LC-HC scFV-Δa3 and N8 control) were tested in a system that mimic of human hemophilia A. Mimics of hemophilia A were prepared by substituting FVIII-deficient plasma (>1% FVIII activity) with washed platelets from individual normal blood donors. The coagulation process is initiated with tissue factor and calcium and the thrombin generation can be followed by including fluorogenic thrombin substrate in the assay that can be detected even as the fibrin clot forms. Thus in mimic of hemophilia A the amount of thrombin generated, and the speed of thrombin generation will be dependent on the amount of FVIII present in the TGT. The TGT method used here is modified from a Platelet Rich Plasma (PRP) assay. Briefly, Platelets isolated from citrated blood collected from individual normal healthy donors and purified through a series of wash steps according to the procedure of Mustard *Washed platelets were added to FVIII-deficient plasma (George King, Overland Park, KA, USA) to a concentration of 150,000 platelets/µl. 80µL of this FVIII-deficient PRP (100,000 platelets/µl final) was mixed with 10µl of Innovin (final dilution 1:200,000) in a microtitter plate and pre-incubated for 10 min at 37°C before 10 µl FVIII compound or buffer, and 20 µl fluorogenic substrate (Z-Gly-Gly-Arg-AMC, Bachem, final concentration 417 nM) mixed with CaCl2 (final concentration 16.7 mM) was added. Emission at 460 nm after excitation at 390nm was measured continuously for 2 hours in Fluoroskan Ascent plate reader (Thermo Electron Corporation). The fluorescence signal was corrected for α2-macroglobulin bound thrombin activity and converted to thrombin concentration using a calibrator and Trombinoscope software (Synapse BV).

In order to demonstrate that AP3 convey a specific platelet binding to N8 that would allow the FVIII molecule to be carried with the platelet in circulation and not just localize to the platelet at the time of activation a the TGT assay described above was performed with the following additional steps: *once the washed platelets had been obtained the platelets were incubated for 30 min at room temperature with either AP3-N8-fusion protein or N8 (20 U/ml) in order to allow the N8 compounds to bind to the platelets. This incubation was follow by two additional wash steps to remove all N8 not specifically bound to the resting platelets. Following these two additional wash steps the platelets were added to FVIII deficient plasma, calcium and fluorogenic substrate and FVIII activity in the form of thrombin generation was measured as described. Parameters obtained from the software are recorded as "Lag time" which describes the time to start of thrombin generation, "Peak" which described the maximum rate of thrombin generation in nM and "Time to peak". A strong clot is characterized by fast early onset and high rates of thrombin formation.

**Table 5**

| Compound | Lagtime (min) | Peak (nM Thrombin) | Time To Peak (min) |
|---|---|---|---|
| Buffer | 18,2 | 9,9 | 97,9 |
| N8 control | 17,1 | 16,5 | 99,6 |
| F8-500 AP3-LC-HC scFV-Δa3 | 8,2 | 68,2 | 27,4 |

The results show strong thrombin generation in the experiment where platelets were incubated with the AP3-N8-fusion protein whereas the platelets incubated with N8 showed barely more than background (hemophilia A) levels of thrombin generation. Thus, while the N8 control could be washed away from the platelets the AP3-N8-fusion protein stayed bound to the platelets thereby directly demonstrating a FVIII activity carried by the platelets.

### EXAMPLE 41

### Analysis of fusion protein binding to purified GPIIIa

Binding interaction analysis was obtained by Surface Plasmon Resonance in a Biacore T-100 instrument. Capture of purified GPIIbIIIa (Enzyme Research Laboratories) at a fixed concentration was obtained by direct immobilization to a CM5 chip to a level of 1000-4000 RU in 10 mM sodium acetate pH 4.5-5.0. Two-fold dilutions of the FVIII derivatives from 5-0.31 nM were tested for binding to the immobilized GPIIbIIIa. Running and dilution buffer: 10 mM HEPES, 150 mM NaCl, 5 mM CaCl2, 0.005% p20, pH 7.4. All FVIII derivatives were dialysed and diluted in running buffer. Regeneration was obtained by 10 mM Glycine, pH 1.7. Determination of kinetic and binding constants (kₒₙ, k_{off}) was obtained assuming a 1:1 interaction of the FVIII derivative and GPIIbIIIa using the Biacore T100 evaluation software. Results are shown in the table below.

**Table 6: Surface Plasmon Resonance (SPR) analysis. Binding to GPIIbIIIa. Kinetic constants.**

| **FVIII derivative ID** | **kₒₙ (1/Ms)** | **k_{off} (1/s)** | **KD (nM)** |
|---|---|---|---|
| F8-500 AP3-HC-LC scFV | 1.3E+05 | 1.5E-04 | 1.2 |
| F8-500 AP3-LC-HC scFV | 1.4E+06 | n.d. | n.d. |
| F8-500 AP3-LC-HC scFV-Δa3 | 2.2E+06 | 5.0E-05 | 0.02 |
| N8-AP3 conjugate Compund MZ 1 | 2.5E+05 | 6.1E-06 | 0.02 |

| | | | |
|---|---|---|---|
| n.d. observed dissociation rate too low for accurate determination of k_{off} | | | |

### EXAMPLE 42

### Analysis of FVIII fusions/conjugates binding to platelets

Platelet-binding of a fusion protein can be tested by flow cytometry. Peripheral blood platelets may be purified, or whole blood can be used. The platelets may be activated or resting. The platelets are incubated with fusion protein for 15-30 min. The fusion protein may be directly labelled with a fluorophore or detected using a fluorescently labelled secondary antibody. A fluorescently labelled platelet specific antibody not interfering with binding of the fusion protein can be added to assess whether the particles binding the fusion protein are indeed platelets. After incubation, the cells are washed to remove fusion protein, and the samples are analyzed on a flow cytometer. The flow cytometer detects unlabelled cells and fluorescently labelled molecules binding to cells and thus can be used to specifically analyze to which extent fusion protein is bound to platelets (or other cells).

The specificity of binding can be assessed e.g. by adding a surplus of unlabelled antibody (when using directly labelled fusion protein). Binding of the FVIII moiety to the platelets can be assessed e.g. by adding a surplus of annexin V or FVIII. Internalization of the fusion protein by the resting platelet may be assessed e.g. by incubating platelets with directly labelled fusion protein followed by incubation with an antibody, which quenches the signal from surface-bound (i.e. not internalized) fusion protein. Only the internalized fusion protein will then be detected by flow cytometry. It may hypothesized that activated platelets will release internalized fusion-protein at the site of clot formation.

### EXAMPLE 43

### Test of FVIII-AP3 fusions and conjugates binding to platelets

Platelet binding of F8-500 AP3-LC-HC scFV-Δa3 (SEQ ID NO 39) (AP3-N8 2097) and full length AP3 IgG chemically coupled to N8 (MZ1 in example 18) were tested by flow cytometry. Washed platelets from human peripheral blood were prepared and incubated with AP3-N8 in dark at room temperature for 30 min (- 300.000 platelets per sample). As a platelet marker, peridinin chlorophyll protein (PerCP)-labelled anti CD42a was added to the samples along with the AP3-N8 constructs. After incubation, the cells were washed with buffer (20 mM Hepes, 150 mM NaCl, 1 mg/ml BSA, 5 mM CaCl₂) to remove unbound antibody, and a phycoerythrin-Cy7 (PE-Cy7) anti-FVIII monoclonal antibody was added (10 µg/ml). After 30 min incubation, the cells were washed with buffer, and the samples were analyzed on a BD LSRFortessa™ flow cytometer with the forward and side scatter detectors in log mode ( at least 5.000 events per sample were analyzed). Doses of AP3-N8 from ∼ 0.025 - 51.2 nM (25.6 nM for MZ1) were analyzed to assess dose-dependency of the binding. Three donors were tested in individual experiments for AP3-N8 2097 and one donor for the MZ1 construct. The specificity of the AP3-N8 binding was assessed by adding excess unlabelled AP3-LC-HC scFV-FLAG (SEQ ID NO 21) to the samples (up to 50- fold excess). Thus, the data show AP3-specific binding of the constructs to the GPIIb/IIIa receptor on platelets. Experiments using a similar AP3-N8 construct, where the N8 moiety was directly labelled with Oregon Green gave comparable results, supporting specific binding of the AP3-N8 construct to the platelets (n = 5).

Internalization of the AP3-N8 can be assessed by first incubating platelets with fluorescently labelled AP3-N8 and measuring the fluorescence by flow cytometry and thereafter adding an antibody, which quenches the signal from surface-bound (i.e. not internalized) AP3-N8. When re-analyzing the samples by flow cytometry, only the internalized AP3-N8 will be detected.

### EXAMPLE 44

### Testing of anti-aggregatory effect of F8-500 AP3-LC-HC scFV-Δa3 (SEQ ID NO 39 on platelets

The possible anti-aggregatory effect of F8-500 AP3-LC-HC scFV-Δa3 (SEQ ID NO 39) was measured by monitoring the change in light transmission through a suspension of isolated platelets. This method was first described essentially by Gustav von Born in the 1960s (Born, Nature, 194:927-29 1962) and is today one of the most used methods for evaluation of platelet function. In brief, the method measures the capability of light to transverse through a suspension of platelets. This suspension of platelets might either be platelet rich plasma or isolated platelets. Upon activation the GPIIb/IIIa changes its conformation to a fibrinogen high-binding state and in the presence of fibrinogen the platelets will start to form aggregates. This is registered as an increase in light transmission since more light will go through a sample with few large aggregates than many single platelets. The inhibitory effect of an antibody is generally examined by the ability of the antibody to decrease platelet aggregatory response to an activator (e.g. ADP or thrombin) measured for example by change in light transmission (Coller et al. JCI 72(1):325-38, 1983).

The term "isolated human platelets" in this example refer to platelets derived from human whole blood kept in an isotonic buffer. The platelets were isolated from heparinized human venous human blood from healthy volunteers that was mixed with acid-citrate-dextrose (ACD) solution (6:1, v/v) containing 85 mM Na-citrat, 71 mM citric acid and 111 mM glucose. The blood was centrifuged at 220g for 20 minutes to obtain platelet-rich plasma (PRP). Acetylsalicylic acid (100 µM) and apyrase 0.5 U/ml were added to prevent activation of the platelets by eicosanoids and adenine nucleotides during the preparation procedure. The platelets in the PRP were spun down by a 20 minutes centrifugation at 480g and the supernatant was removed. The platelets were gently resuspended in calcium free Hepes solution (pH 7.4) composed of 145 mM NaCl, 5 mM KCl, 1 mM MgSO4, 10 mM glucose, 10 mM Hepes and 1 U/ml apyrase. The platelet suspensions were kept in plastic tubes at room temperature and [Ca²⁺] was adjusted to 1 mM and the temperature was adjusted to 37°C in each experiment.

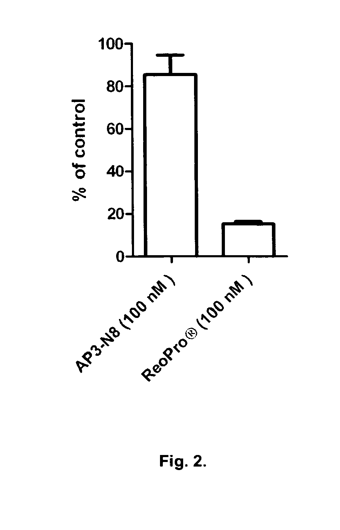

The platelet samples were kept in siliconized glass cyvettes with continuous stirring (1200 rpm) at 37°C while measuring light transmission using the Platelet Aggregation Profiler® (PAP)-8E instrument (Bio/Data Corporation, Horsham, PA). The samples were incubated with either F8-500 AP3-LC-HC scFV-Δa3 (SEQ ID NO 39) (30 nM), AP3-LC-HC scFV-FLAG (SEQ ID NO 21) (30 nM) or abciximab (ReoPro®) (30 nM) for 3 minutes before activated with a protease activated receptor-1 (PAR-1) activating peptide (amino acid sequence SFLLRN) (10 µM). The results show no significant difference in platelet aggregation in AP3-N8 treated platelets compared to control (Table 7). Furthermore, AP3-LC-HC scFV-FLAG was tested for anti-aggregatory properties at 30 nM showing no indication for an inhibitory effect. However, the anti-aggregatory abciximab (ReoPro®) (30 nM) effectively inhibited platelet aggregation by almost 40 % in SFLLRN activated platelets (Table 7). The difference between F8-500 AP3-LC-HC scFV-Δa3 and ReoPro® in respect to inhibition of platelet aggregation was even more pronounced when increasing the concentration. Figure 2 show platelet aggregation when using F8-500 AP3-LC-HC scFV-Δa3 (100 nM) or ReoPro® (100 nM). This higher concentration results in an augmented inhibition with ReoPro® whereas the increased concentration F8-500 AP3-LC-HC scFV-Δa3 do not influence on platelet aggregation.

**Table 7. Platelet aggregation**

| **F8-500 AP3-LC-HC scFV-Δa3 (30 nM)** | **AP3-LC-HC scFV-FLAG (30 nM)** | **ReoPro® (30 nM)** | **Control** |
|---|---|---|---|
| 86.02±5.8 | 95.09±3.5 | 60.72±22.1 | 100 |

| | | | |
|---|---|---|---|
| *Data shown as % of control (SFLLRN 10 µM)* ±SD *and n=3 in all groups.* | | | |

### EXAMPLE 45

### Preparation and use of a mouse strain with a humanised IgB3 receptor

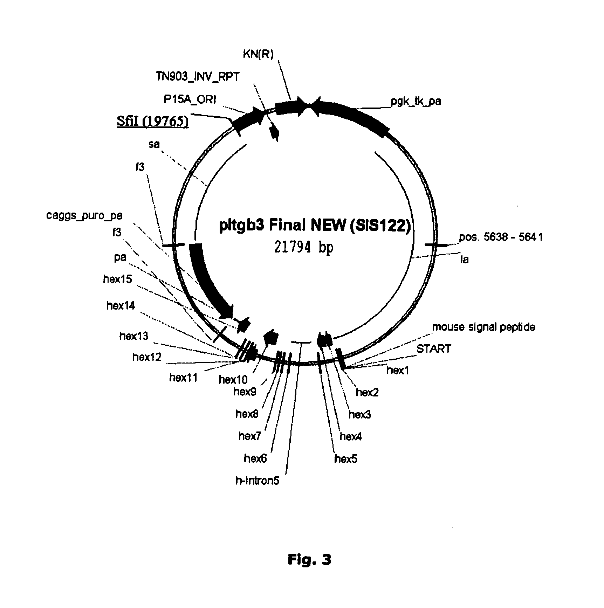

A vector containing the Human ITGB3 cDNA {from exon 2 (G26) to exon 15 (including partial 3'UTR from cDNA provided)} is engineered by inserting human ITGB3 intron 5 (647 bp) between sequences corresponding to human exons 5 and 6. A transcriptional STOP cassette is inserted immediately downstream of the human partial 3'UTR. The engineered human ITGB3 cDNA is inserted at its corresponding position in mouse exon 1. In order to optimize processing of the human ITGB3 protein in mouse cells, the humanized allele will express a fusion protein between mouse Itgb3 signal peptide (encoded within exon 1) and human ITGB3 mature protein. Selection marker (Puro) is flanked by F3 sites and inserted downstream of human ITGB3 3'UTR. Humanized allele after Flp-mediated removal of selection marker. The human Itgb3 protein will be expressed under the control of the mouse Itgb3 promoter. The insertion of the engineered human ITGB3 cDNA, including its 3'UTR region, into mouse exon 1 should lead to the inactivation of the mouse Itgb3 gene. The confirmed sequence of the final targeting vector is shown in figure 3.

### Generation of heterozygous targeted C57BL/6 ES cells

The C57BL/6N ES cell line is grown on a mitotically inactivated feeder layer comprised of mouse embryonic fibroblasts in DMEM High Glucose medium containing 20% FBS and 1200 u/mL Leukemia Inhibitory Factor. 1×10⁷ cells and 30 µg of linearized DNA vector are electroporated at 240 V and 500 µF. Clone selection is based on Puromycin selection (1 µg/mL) started on d2 and counterselection with Gancyclovir (2 µM) started on d5 after electroporation. ES clones are isolated on d8 and analyzed by Southern Blotting after expansion.

### Generation of hererozygous animals:

After administration of hormones, superovulated Balb/c females are mated with Balb/c males. Blastocysts is isolated from the uterus at dpc 3.5. For microinjection, blastocysts are placed in a drop of DMEM with 15% FCS under mineral oil. A flat tip, piezo actuated microinjection-pipette with an internal diameter of 12 - 15 micrometer is used to inject 10-15 targeted C57BL/6 N.tac ES cells into each blastocyst. After recovery, 8 injected blastocysts are transferred to each uterine horn of 2.5 days post coitum, pseudopregnant NMRI females. Chimerism is measured in chimeras (G0) by coat colour contribution of ES cells to the Balb/c host (black/white). Highly chimeric mice are bred to strain C57BL/6 females. Germline transmission was identified by the presence of black, strain C57BL/6, offspring (G1).

### Generation of homozygous animals

Offspring G1 was propagated either by breeding of heterozygous mice or by in vitro fertilization. From the initial attemps at mating and IVF, genotypic distributions was determined as outlined in table 8.

**Table 8. Genotyping of humanized GPIIIb mice.**

| **Genotype** | **IVF** | | **Mating** |
|---|---|---|---|
| *Human itgB3* | *Males* | *Females* | |
| Homozygous | none | none | 3 |
| Heterozygous | 10 | 12 | 7 |
| Wildtype | 8 | 11 | 4 |

### EXAMPLE 46

### Pharmacokinetics of GPIIIa-targeted fusion proteins in GPIIIa transgenic mice

The AP3 antibody binds to the human GPIIb/IIIa (integrin αIIβ3) receptor on platelets, but it does not recognize murine GPIIb/IIIa, preventing the use of wild type mice for pharmacokinetic (PK) analyses. The PK profile of an AP3-FVIII fusion or conjugate can be analyzed in transgenic mice expressing human GPIIIa (bred at Taconic M&B), which associates with murine GPIIb enabling the binding of AP3 to the receptor. The GPIIIa transgenic mice will receive a single i.v. injection of AP3-FVIII fusion or conjugate and blood will be collected from the pre-orbital plexus at time-points up to 288 hours after injection. About three samples will be collected from each mouse during the study and 2-4 samples collected for each time point. The blood is stabilized and diluted in appropriate buffer. The injected AP3-FVIII fusion or conjugate (free and/or plate bound) can be quantified by means of ELISA or flow cytometry either using antibodies against N8 or directly labelled AP3-FVIII fusion or conjugate.

### EXAMPLE 47

### Duration of effect of GPIIIa-targeted fusion proteins in GPIIIa transgenic mice

In order to evaluate efficacy and duration of action of effect of a GPIIIa-targeted fusion protein in an animal disease model a mouse strain with a humanised IgB3 receptor is generated. The generated mouse strain may be cross breed with other (transgenic) mice strains this includes but are not limited to mice lacking the coagulation factor VIII or IX. The mice may be breed by natural mating or using IVF transfer (e.g. G.Vergara Theri-ogenology 1997; 47, 1245-1252). Alternative the humanized platelets may be transferred to other mice by the means of bone marrow transplantation. The bone marrow cells are isolated from the humanised mice for example by the method described in Shi et al. (Blood. 2008;112, 2713-2721) and injected into appropriated prepared recipient mice ie. mice lacking factor VIII or IX. The efficacy can be tested in the above mentioned animals models by measuring the ability to reduce the bleeding in a tail bleeding test (Holmberg et al., J Thromb Haemost 2009; 7: 1517-22) or the ability to form a clot in FeCl₃ injury model (Moller & Tranholm, Haemophilia 2010; 16, e216-e222). The duration of action can be tested by the efficacy of the drug after prolonged in the above mentioned models.

## Claims

1. A FVIII molecule comprising an amino acid sequence that is at least 95% identical to the mature portion of an amino acid sequence selected from the list consisting of SEQ ID NO:1 and SEQ ID NO: 3, which molecule is covalently attached to a platelet-specific molecule, wherein said platelet-specific molecule is a GPIIb/IIIa antibody that does not inhibit platelet aggregation.

2. A FVIII molecule according to claim 1, wherein said molecule has reduced vWF binding capacity.

3. A FVIII molecule according to any one of claims 1-2, wherein the molecule is a fusion protein.

4. A FVIII molecule according to any one of claims 1-2, wherein the GPIIb/IIIa antibody that does not inhibit platelet aggregation is covalently attached to FVIII via a linker.

5. A FVIII molecule according to claim 4, wherein the linker comprises an N-linked or an O-linked glycan on the FVIII molecule.

6. A FVIII molecule according to claim 5, wherein the glycan is placed in the B domain.

7. A FVIII molecule according to claim 3, wherein the GPIIb/IIIa antibodythat does not inhibit platelet aggregation, is fused to the B-domain of a B domain truncated Factor VIII molecule.

8. A FVIII molecule according to claim 3, wherein the a3 domain of the FVIII molecule is replaced with the GPIIb/IIIa antibody that does not inhibit platelet aggregation.

9. A factor FVIII molecule according to claim 6, wherein the FVIII molecule comprises the sequence as set forth in SEQ ID NO 3, and wherein the linker comprises an O-linked glycan placed in the B domain.

10. A nucleic acid encoding a FVIII molecule according to any one of claims 7 or 8.

11. A host cell comprising a nucleic acid according to claim 10.

12. A method of producing a FVIII molecule said method comprising expressing the nucleic acid according to claim 10 in a host cell according to claim 11.

13. A method of producing a FVIII molecule according to any one of claims 4, 5, 6 and 9, wherein said method comprises conjugation of the FVIII molecule with the GPIIb/IIIa antibody that does not inhibit platelet aggregation.

14. A pharmaceutical composition comprising a FVIII molecule according to any one of claims 1-9.

15. A FVIII molecule according to any one of claims 1-9 for use in a method for the treatment of haemophilia A.

## Patentansprüche

1. FVIII-Molekül, umfassend eine Aminosäuresequenz, die mit dem reifen Anteil einer Aminosäuresequenz, ausgewählt aus der Liste, bestehend aus SEQ ID NO. 1 und SEQ ID NO: 3, zu mindestens 95% identisch ist; wobei das Molekül an ein blutplättchenspezifisches Molekül kovalent angelagert ist, wobei das blutplättchenspezifische Molekül ein eine Blutplättchenaggregation nicht hemmender GPIIb/IIIa-Antikörper ist.

2. FVIII-Molekül nach Anspruch 1, wobei das Molekül ein reduziertes vWF-Bindungsvermögen aufweist.

3. FVIII-Molekül nach einem der Ansprüche 1-2, wobei das Molekül ein Fusionsprotein ist.

4. FVIII-Molekül nach einem der Ansprüche 1-2, wobei der eine Blutplättchenaggregation nicht hemmende GPIIb/IIIa-Antikörper über eine Verbindungsgruppe an FVIII kovalent angelagert ist.

5. FVIII-Molekül nach Anspruch 4, wobei die Verbindungsgruppe ein N-gebundenes oder ein O-gebundenes Glycan am FVIII-Molekül umfasst.

6. FVIII-Molekül nach Anspruch 5, wobei das Glycan in der B-Domäne angeordnet ist.

7. FVIII-Molekül nach Anspruch 3, wobei der eine Blutplättchenaggregation nicht hemmende GPIIb/IIIa-Antikörper an die B-Domäne eines B-Domänen-trunkierten Faktor-VIII-Moleküls kondensiert ist.

8. FVIII-Molekül nach Anspruch 3, wobei die a3-Domäne des FVIII-Moleküls durch den eine Blutplättchenaggregation nicht hemmenden GPIIb/IIIa-Antikörper ersetzt ist.

9. FVIII-Molekül nach Anspruch 6, wobei das FVIII-Molekül die wie in SEQ ID NO. 3 dargestellte Sequenz umfasst und wobei die Verbindungsgruppe ein in der B-Domäne angeordnetes O-gebundenes Glycan umfasst.

10. Nukleinsäure, die ein FVIII-Molekül nach einem der Ansprüche 7 oder 8 kodiert.

11. Wirtszelle, die eine Nukleinsäure nach Anspruch 10 umfasst.

12. Verfahren zur Herstellung eines FVIII-Moleküls, wobei das Verfahren das Exprimieren der Nukleinsäure nach Anspruch 10 in einer Wirtszelle nach Anspruch 11 umfasst.

13. Verfahren zur Herstellung eines FVIII-Moleküls nach einem der Ansprüche 4, 5, 6 und 9, wobei das Verfahren die Konjugation des FVIII-Moleküls mit dem eine Blutplättchenaggregation nicht hemmenden GPIIb/IIIa-Antikörper umfasst.

14. Arzneimittel, das ein FVIII-Molekül nach einem der Ansprüche 1-9 umfasst.

15. FVIII-Molekül nach einem der Ansprüche 1-9 zur Verwendung in einem Verfahren zur Behandlung von Hämophilie A.

## Revendications

1. Molécule de FVIII comprenant une séquence d'acides aminés qui a une identité d'au moins 95 % avec la portion mature d'une séquence d'acides aminés choisie dans la liste consistant en SEQ ID NO:1 et SEQ ID NO:3, laquelle molécule étant fixée par liaison covalente à une molécule spécifique de plaquette, ladite molécule spécifique de plaquette étant un anticorps anti-GPIIb/IIIa qui n'inhibe pas l'agrégation plaquettaire.

2. Molécule de FVIII selon la revendication 1, ladite molécule présentant une capacité réduite de liaison au vWF.

3. Molécule de FVIII selon l'une quelconque des revendications 1-2, la molécule étant une protéine de fusion.

4. Molécule de FVIII selon l'une quelconque des revendications 1-2, dans laquelle l'anticorps anti-GPIIb/IIIa qui n'inhibe pas l'agrégation plaquettaire est fixé par liaison covalente au FVIII par l'intermédiaire d'un lieur.

5. Molécule de FVIII selon la revendication 4, dans laquelle le lieur comprend un glycanne N-lié ou O-lié sur la molécule de FVIII.

6. Molécule de FVIII selon la revendication 5, dans laquelle le glycanne est placé est dans le domaine B.

7. Molécule de FVIII selon la revendication 3, dans laquelle l'anticorps anti-GPIIb/IIIa qui n'inhibe pas l'agrégation plaquettaire est fusionné au domaine B d'une molécule de facteur VIII à domaine B tronqué.

8. Molécule de FVIII selon la revendication 3, dans laquelle le domaine a3 de la molécule de FVIII est remplacé par un anticorps anti-GBIIb/IIIa qui n'inhibe pas l'agrégation plaquettaire.

9. Molécule de facteur FVIII selon la revendication 6, la molécule de FVIII comprenant la séquence telle que présentée dans SEQ ID NO:3, le lieur comprenant un glycanne O-lié placé dans le domaine B.

10. Acide nucléique codant pour une molécule de FVIII selon l'une quelconque des revendications 7 ou 8.

11. Cellule hôte comprenant un acide nucléique selon la revendication 10.

12. Procédé de production d'une molécule de FVIII, ledit procédé comprenant l'expression de l'acide nucléique selon la revendication 10 dans une cellule hôte selon la revendication 11.

13. Procédé de production d'une molécule de FVIII selon l'une quelconque des revendications 4, 5, 6 et 9, ledit procédé comprenant la conjugaison de la molécule de FVIII avec l'anticorps anti-GPIIb/IIIa qui n'inhibe pas l'agrégation plaquettaire.

14. Composition pharmaceutique comprenant une molécule de FVIII selon l'une quelconque des revendications 1 à 9.

15. Molécule de FVIII selon l'une quelconque des revendications 1 à 9 pour une utilisation dans un procédé destiné au traitement de l'hémophilie A.


### SEQUENCE LISTING

<110> Novo Nordisk A/S
   <120> TARGETED DELIVERY OF FACTOR VIII PROTEINS TO PLATELETS
   <130> 8044.504-WO
   <160> 42
   <170> PatentIn version 3.5
<210> 1
   <211> 2351
   <212> PRT
   <213> homo sapiens
<400> 1
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
   450 455 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
   500 505 510
   Gly Val Lys His Leu Lys Asp Phe Pro Ile Leu Pro Gly Glu Ile Phe
   515 520 525
   Lys Tyr Lys Trp Thr Val Thr Val Glu Asp Gly Pro Thr Lys Ser Asp
   530 535 540
   Pro Arg Cys Leu Thr Arg Tyr Tyr Ser Ser Phe Val Asn Met Glu Arg
   545 550 555 560
   Asp Leu Ala Ser Gly Leu Ile Gly Pro Leu Leu Ile Cys Tyr Lys Glu
   565 570 575
   Ser Val Asp Gln Arg Gly Asn Gln Ile Met Ser Asp Lys Arg Asn Val
   580 585 590
   Ile Leu Phe Ser Val Phe Asp Glu Asn Arg Ser Trp Tyr Leu Thr Glu
   595 600 605
   Asn Ile Gln Arg Phe Leu Pro Asn Pro Ala Gly Val Gln Leu Glu Asp
   610 615 620
   Pro Glu Phe Gln Ala Ser Asn Ile Met His Ser Ile Asn Gly Tyr Val
   625 630 635 640
   Phe Asp Ser Leu Gln Leu Ser Val Cys Leu His Glu Val Ala Tyr Trp
   645 650 655
   Tyr Ile Leu Ser Ile Gly Ala Gln Thr Asp Phe Leu Ser Val Phe Phe
   660 665 670
   Ser Gly Tyr Thr Phe Lys His Lys Met Val Tyr Glu Asp Thr Leu Thr
   675 680 685
   Leu Phe Pro Phe Ser Gly Glu Thr Val Phe Met Ser Met Glu Asn Pro
   690 695 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Ser Arg His Pro
           755                 760                 765
   Ser Thr Arg Gln Lys Gln Phe Asn Ala Thr Thr Ile Pro Glu Asn Asp
       770                 775                 780
   Ile Glu Lys Thr Asp Pro Trp Phe Ala His Arg Thr Pro Met Pro Lys
   785                 790                 795                 800
   Ile Gln Asn Val Ser Ser Ser Asp Leu Leu Met Leu Leu Arg Gln Ser
                   805                 810                 815
   Pro Thr Pro His Gly Leu Ser Leu Ser Asp Leu Gln Glu Ala Lys Tyr
               820                 825                 830
   Glu Thr Phe Ser Asp Asp Pro Ser Pro Gly Ala Ile Asp Ser Asn Asn
           835                 840                 845
   Ser Leu Ser Glu Met Thr His Phe Arg Pro Gln Leu His His Ser Gly
       850                 855                 860
   Asp Met Val Phe Thr Pro Glu Ser Gly Leu Gln Leu Arg Leu Asn Glu
   865                 870                 875                 880
   Lys Leu Gly Thr Thr Ala Ala Thr Glu Leu Lys Lys Leu Asp Phe Lys
                   885                 890                 895
   Val Ser Ser Thr Ser Asn Asn Leu Ile Ser Thr Ile Pro Ser Asp Asn
               900                 905                 910
   Leu Ala Ala Gly Thr Asp Asn Thr Ser Ser Leu Gly Pro Pro Ser Met
           915                 920                 925
   Pro Val His Tyr Asp Ser Gln Leu Asp Thr Thr Leu Phe Gly Lys Lys
       930                 935                 940
   Ser Ser Pro Leu Thr Glu Ser Gly Gly Pro Leu Ser Leu Ser Glu Glu
   945                 950                 955                 960
   Asn Asn Asp Ser Lys Leu Leu Glu Ser Gly Leu Met Asn Ser Gln Glu
   965 970 975
   Ser Ser Trp Gly Lys Asn Val Ser Ser Thr Glu Ser Gly Arg Leu Phe
   980 985 990
   Lys Gly Lys Arg Ala His Gly Pro Ala Leu Leu Thr Lys Asp Asn Ala
   995 1000 1005
   Leu Phe Lys Val Ser Ile Ser Leu Leu Lys Thr Asn Lys Thr Ser
   1010 1015 1020
   Asn Asn Ser Ala Thr Asn Arg Lys Thr His Ile Asp Gly Pro Ser
   1025 1030 1035
   Leu Leu Ile Glu Asn Ser Pro Ser Val Trp Gln Asn Ile Leu Glu
   1040 1045 1050
   Ser Asp Thr Glu Phe Lys Lys Val Thr Pro Leu Ile His Asp Arg
   1055 1060 1065
   Met Leu Met Asp Lys Asn Ala Thr Ala Leu Arg Leu Asn His Met
   1070 1075 1080
   Ser Asn Lys Thr Thr Ser Ser Lys Asn Met Glu Met Val Gln Gln
   1085 1090 1095
   Lys Lys Glu Gly Pro Ile Pro Pro Asp Ala Gln Asn Pro Asp Met
   1100 1105 1110
   Ser Phe Phe Lys Met Leu Phe Leu Pro Glu Ser Ala Arg Trp Ile
   1115 1120 1125
   Gln Arg Thr His Gly Lys Asn Ser Leu Asn Ser Gly Gln Gly Pro
   1130 1135 1140
   Ser Pro Lys Gln Leu Val Ser Leu Gly Pro Glu Lys Ser Val Glu
   1145 1150 1155
   Gly Gln Asn Phe Leu Ser Glu Lys Asn Lys Val Val Val Gly Lys
   1160 1165 1170
   Gly Glu Phe Thr Lys Asp Val Gly Leu Lys Glu Met Val Phe Pro
   1175 1180 1185
   Ser Ser Arg Asn Leu Phe Leu Thr Asn Leu Asp Asn Leu His Glu
   1190 1195 1200
   Asn Asn Thr His Asn Gln Glu Lys Lys Ile Gln Glu Glu Ile Glu
   1205 1210 1215
   Lys Lys Glu Thr Leu Ile Gln Glu Asn Val Val Leu Pro Gln Ile
   1220 1225 1230
   His Thr Val Thr Gly Thr Lys Asn Phe Met Lys Asn Leu Phe Leu
   1235 1240 1245
   Leu Ser Thr Arg Gln Asn Val Glu Gly Ser Tyr Asp Gly Ala Tyr
   1250 1255 1260
   Ala Pro Val Leu Gln Asp Phe Arg Ser Leu Asn Asp Ser Thr Asn
   1265 1270 1275
   Arg Thr Lys Lys His Thr Ala His Phe Ser Lys Lys Gly Glu Glu
   1280 1285 1290
   Glu Asn Leu Glu Gly Leu Gly Asn Gln Thr Lys Gln Ile Val Glu
   1295 1300 1305
   Lys Tyr Ala Cys Thr Thr Arg Ile Ser Pro Asn Thr Ser Gln Gln
   1310 1315 1320
   Asn Phe Val Thr Gln Arg Ser Lys Arg Ala Leu Lys Gln Phe Arg
   1325 1330 1335
   Leu Pro Leu Glu Glu Thr Glu Leu Glu Lys Arg Ile Ile Val Asp
   1340 1345 1350
   Asp Thr Ser Thr Gln Trp Ser Lys Asn Met Lys His Leu Thr Pro
   1355 1360 1365
   Ser Thr Leu Thr Gln Ile Asp Tyr Asn Glu Lys Glu Lys Gly Ala
   1370 1375 1380
   Ile Thr Gln Ser Pro Leu Ser Asp Cys Leu Thr Arg Ser His Ser
   1385 1390 1395
   Ile Pro Gln Ala Asn Arg Ser Pro Leu Pro Ile Ala Lys Val Ser
   1400 1405 1410
   Ser Phe Pro Ser Ile Arg Pro Ile Tyr Leu Thr Arg Val Leu Phe
   1415 1420 1425
   Gln Asp Asn Ser Ser His Leu Pro Ala Ala Ser Tyr Arg Lys Lys
   1430 1435 1440
   Asp Ser Gly Val Gln Glu Ser Ser His Phe Leu Gln Gly Ala Lys
   1445 1450 1455
   Lys Asn Asn Leu Ser Leu Ala Ile Leu Thr Leu Glu Met Thr Gly
   1460 1465 1470
   Asp Gln Arg Glu Val Gly Ser Leu Gly Thr Ser Ala Thr Asn Ser
   1475 1480 1485
   Val Thr Tyr Lys Lys Val Glu Asn Thr Val Leu Pro Lys Pro Asp
   1490 1495 1500
   Leu Pro Lys Thr Ser Gly Lys Val Glu Leu Leu Pro Lys Val His
   1505 1510 1515
   Ile Tyr Gln Lys Asp Leu Phe Pro Thr Glu Thr Ser Asn Gly Ser
   1520 1525 1530
   Pro Gly His Leu Asp Leu Val Glu Gly Ser Leu Leu Gln Gly Thr
   1535 1540 1545
   Glu Gly Ala Ile Lys Trp Asn Glu Ala Asn Arg Pro Gly Lys Val
   1550 1555 1560
   Pro Phe Leu Arg Val Ala Thr Glu Ser Ser Ala Lys Thr Pro Ser
   1565 1570 1575
   Lys Leu Leu Asp Pro Leu Ala Trp Asp Asn His Tyr Gly Thr Gln
   1580 1585 1590
   Ile Pro Lys Glu Glu Trp Lys Ser Gln Glu Lys Ser Pro Glu Lys
   1595 1600 1605
   Thr Ala Phe Lys Lys Lys Asp Thr Ile Leu Ser Leu Asn Ala Cys
   1610 1615 1620
   Glu Ser Asn His Ala Ile Ala Ala Ile Asn Glu Gly Gln Asn Lys
   1625 1630 1635
   Pro Glu Ile Glu Val Thr Trp Ala Lys Gln Gly Arg Thr Glu Arg
   1640 1645 1650
   Leu Cys Ser Gln Asn Pro Pro Val Leu Lys Arg His Gln Arg Glu
   1655 1660 1665
   Ile Thr Arg Thr Thr Leu Gln Ser Asp Gln Glu Glu Ile Asp Tyr
   1670 1675 1680
   Asp Asp Thr Ile Ser Val Glu Met Lys Lys Glu Asp Phe Asp Ile
   1685 1690 1695
   Tyr Asp Glu Asp Glu Asn Gln Ser Pro Arg Ser Phe Gln Lys Lys
   1700 1705 1710
   Thr Arg His Tyr Phe Ile Ala Ala Val Glu Arg Leu Trp Asp Tyr
   1715 1720 1725
   Gly Met Ser Ser Ser Pro His Val Leu Arg Asn Arg Ala Gln Ser
   1730 1735 1740
   Gly Ser Val Pro Gln Phe Lys Lys Val Val Phe Gln Glu Phe Thr
   1745 1750 1755
   Asp Gly Ser Phe Thr Gln Pro Leu Tyr Arg Gly Glu Leu Asn Glu
   1760 1765 1770
   His Leu Gly Leu Leu Gly Pro Tyr Ile Arg Ala Glu Val Glu Asp
   1775 1780 1785
   Asn Ile Met Val Thr Phe Arg Asn Gln Ala Ser Arg Pro Tyr Ser
   1790 1795 1800
   Phe Tyr Ser Ser Leu Ile Ser Tyr Glu Glu Asp Gln Arg Gln Gly
   1805 1810 1815
   Ala Glu Pro Arg Lys Asn Phe Val Lys Pro Asn Glu Thr Lys Thr
   1820 1825 1830
   Tyr Phe Trp Lys Val Gln His His Met Ala Pro Thr Lys Asp Glu
   1835 1840 1845
   Phe Asp Cys Lys Ala Trp Ala Tyr Phe Ser Asp Val Asp Leu Glu
   1850 1855 1860
   Lys Asp Val His Ser Gly Leu Ile Gly Pro Leu Leu Val Cys His
   1865 1870 1875
   Thr Asn Thr Leu Asn Pro Ala His Gly Arg Gln Val Thr Val Gln
   1880 1885 1890
   Glu Phe Ala Leu Phe Phe Thr Ile Phe Asp Glu Thr Lys Ser Trp
   1895 1900 1905
   Tyr Phe Thr Glu Asn Met Glu Arg Asn Cys Arg Ala Pro Cys Asn
   1910 1915 1920
   Ile Gln Met Glu Asp Pro Thr Phe Lys Glu Asn Tyr Arg Phe His
   1925 1930 1935
   Ala Ile Asn Gly Tyr Ile Met Asp Thr Leu Pro Gly Leu Val Met
   1940 1945 1950
   Ala Gln Asp Gln Arg Ile Arg Trp Tyr Leu Leu Ser Met Gly Ser
   1955 1960 1965
   Asn Glu Asn Ile His Ser Ile His Phe Ser Gly His Val Phe Thr
   1970 1975 1980
   Val Arg Lys Lys Glu Glu Tyr Lys Met Ala Leu Tyr Asn Leu Tyr
   1985 1990 1995
   Pro Gly Val Phe Glu Thr Val Glu Met Leu Pro Ser Lys Ala Gly
   2000 2005 2010
   Ile Trp Arg Val Glu Cys Leu Ile Gly Glu His Leu His Ala Gly
   2015 2020 2025
   Met Ser Thr Leu Phe Leu Val Tyr Ser Asn Lys Cys Gln Thr Pro
   2030 2035 2040
   Leu Gly Met Ala Ser Gly His Ile Arg Asp Phe Gln Ile Thr Ala
   2045 2050 2055
   Ser Gly Gln Tyr Gly Gln Trp Ala Pro Lys Leu Ala Arg Leu His
   2060 2065 2070
   Tyr Ser Gly Ser Ile Asn Ala Trp Ser Thr Lys Glu Pro Phe Ser
   2075 2080 2085
   Trp Ile Lys Val Asp Leu Leu Ala Pro Met Ile Ile His Gly Ile
   2090 2095 2100
   Lys Thr Gln Gly Ala Arg Gln Lys Phe Ser Ser Leu Tyr Ile Ser
   2105 2110 2115
   Gln Phe Ile Ile Met Tyr Ser Leu Asp Gly Lys Lys Trp Gln Thr
   2120 2125 2130
   Tyr Arg Gly Asn Ser Thr Gly Thr Leu Met Val Phe Phe Gly Asn
   2135 2140 2145
   Val Asp Ser Ser Gly Ile Lys His Asn Ile Phe Asn Pro Pro Ile
   2150 2155 2160
   Ile Ala Arg Tyr Ile Arg Leu His Pro Thr His Tyr Ser Ile Arg
   2165 2170 2175
   Ser Thr Leu Arg Met Glu Leu Met Gly Cys Asp Leu Asn Ser Cys
   2180 2185 2190
   Ser Met Pro Leu Gly Met Glu Ser Lys Ala Ile Ser Asp Ala Gln
   2195 2200 2205
   Ile Thr Ala Ser Ser Tyr Phe Thr Asn Met Phe Ala Thr Trp Ser
   2210 2215 2220
   Pro Ser Lys Ala Arg Leu His Leu Gln Gly Arg Ser Asn Ala Trp
   2225 2230 2235
   Arg Pro Gln Val Asn Asn Pro Lys Glu Trp Leu Gln Val Asp Phe
   2240 2245 2250
   Gln Lys Thr Met Lys Val Thr Gly Val Thr Thr Gln Gly Val Lys
   2255 2260 2265
   Ser Leu Leu Thr Ser Met Tyr Val Lys Glu Phe Leu Ile Ser Ser
   2270 2275 2280
   Ser Gln Asp Gly His Gln Trp Thr Leu Phe Phe Gln Asn Gly Lys
   2285 2290 2295
   Val Lys Val Phe Gln Gly Asn Gln Asp Ser Phe Thr Pro Val Val
   2300 2305 2310
   Asn Ser Leu Asp Pro Pro Leu Leu Thr Arg Tyr Leu Arg Ile His
   2315 2320 2325
   Pro Gln Ser Trp Val His Gln Ile Ala Leu Arg Met Glu Val Leu
   2330 2335 2340
   Gly Cys Glu Ala Gln Asp Leu Tyr
   2345 2350
<210> 2
   <211> 1457
   <212> PRT
   <213> homo sapiens
<400> 2
   Met Gln Ile Glu Leu Ser Thr Cys Phe Phe Leu Cys Leu Leu Arg Phe
   1 5 10 15
   Cys Phe Ser Ala Thr Arg Arg Tyr Tyr Leu Gly Ala Val Glu Leu Ser
   20 25 30
   Trp Asp Tyr Met Gln Ser Asp Leu Gly Glu Leu Pro Val Asp Ala Arg
   35 40 45
   Phe Pro Pro Arg Val Pro Lys Ser Phe Pro Phe Asn Thr Ser Val Val
   50 55 60
   Tyr Lys Lys Thr Leu Phe Val Glu Phe Thr Asp His Leu Phe Asn Ile
   65 70 75 80
   Ala Lys Pro Arg Pro Pro Trp Met Gly Leu Leu Gly Pro Thr Ile Gln
   85 90 95
   Ala Glu Val Tyr Asp Thr Val Val Ile Thr Leu Lys Asn Met Ala Ser
   100 105 110
   His Pro Val Ser Leu His Ala Val Gly Val Ser Tyr Trp Lys Ala Ser
   115 120 125
   Glu Gly Ala Glu Tyr Asp Asp Gln Thr Ser Gln Arg Glu Lys Glu Asp
   130 135 140
   Asp Lys Val Phe Pro Gly Gly Ser His Thr Tyr Val Trp Gln Val Leu
   145 150 155 160
   Lys Glu Asn Gly Pro Met Ala Ser Asp Pro Leu Cys Leu Thr Tyr Ser
   165 170 175
   Tyr Leu Ser His Val Asp Leu Val Lys Asp Leu Asn Ser Gly Leu Ile
   180 185 190
   Gly Ala Leu Leu Val Cys Arg Glu Gly Ser Leu Ala Lys Glu Lys Thr
   195 200 205
   Gln Thr Leu His Lys Phe Ile Leu Leu Phe Ala Val Phe Asp Glu Gly
   210 215 220
   Lys Ser Trp His Ser Glu Thr Lys Asn Ser Leu Met Gln Asp Arg Asp
   225 230 235 240
   Ala Ala Ser Ala Arg Ala Trp Pro Lys Met His Thr Val Asn Gly Tyr
   245 250 255
   Val Asn Arg Ser Leu Pro Gly Leu Ile Gly Cys His Arg Lys Ser Val
   260 265 270
   Tyr Trp His Val Ile Gly Met Gly Thr Thr Pro Glu Val His Ser Ile
   275 280 285
   Phe Leu Glu Gly His Thr Phe Leu Val Arg Asn His Arg Gln Ala Ser
   290 295 300
   Leu Glu Ile Ser Pro Ile Thr Phe Leu Thr Ala Gln Thr Leu Leu Met
   305 310 315 320
   Asp Leu Gly Gln Phe Leu Leu Phe Cys His Ile Ser Ser His Gln His
   325 330 335
   Asp Gly Met Glu Ala Tyr Val Lys Val Asp Ser Cys Pro Glu Glu Pro
   340 345 350
   Gln Leu Arg Met Lys Asn Asn Glu Glu Ala Glu Asp Tyr Asp Asp Asp
   355 360 365
   Leu Thr Asp Ser Glu Met Asp Val Val Arg Phe Asp Asp Asp Asn Ser
   370 375 380
   Pro Ser Phe Ile Gln Ile Arg Ser Val Ala Lys Lys His Pro Lys Thr
   385 390 395 400
   Trp Val His Tyr Ile Ala Ala Glu Glu Glu Asp Trp Asp Tyr Ala Pro
   405 410 415
   Leu Val Leu Ala Pro Asp Asp Arg Ser Tyr Lys Ser Gln Tyr Leu Asn
   420 425 430
   Asn Gly Pro Gln Arg Ile Gly Arg Lys Tyr Lys Lys Val Arg Phe Met
   435 440 445
   Ala Tyr Thr Asp Glu Thr Phe Lys Thr Arg Glu Ala Ile Gln His Glu
   450 455 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
   500 505 510
   Gly Val Lys His Leu Lys Asp Phe Pro Ile Leu Pro Gly Glu Ile Phe
   515 520 525
   Lys Tyr Lys Trp Thr Val Thr Val Glu Asp Gly Pro Thr Lys Ser Asp
   530 535 540
   Pro Arg Cys Leu Thr Arg Tyr Tyr Ser Ser Phe Val Asn Met Glu Arg
   545 550 555 560
   Asp Leu Ala Ser Gly Leu Ile Gly Pro Leu Leu Ile Cys Tyr Lys Glu
   565 570 575
   Ser Val Asp Gln Arg Gly Asn Gln Ile Met Ser Asp Lys Arg Asn Val
   580 585 590
   Ile Leu Phe Ser Val Phe Asp Glu Asn Arg Ser Trp Tyr Leu Thr Glu
   595 600 605
   Asn Ile Gln Arg Phe Leu Pro Asn Pro Ala Gly Val Gln Leu Glu Asp
   610 615 620
   Pro Glu Phe Gln Ala Ser Asn Ile Met His Ser Ile Asn Gly Tyr Val
   625 630 635 640
   Phe Asp Ser Leu Gln Leu Ser Val Cys Leu His Glu Val Ala Tyr Trp
   645 650 655
   Tyr Ile Leu Ser Ile Gly Ala Gln Thr Asp Phe Leu Ser Val Phe Phe
   660 665 670
   Ser Gly Tyr Thr Phe Lys His Lys Met Val Tyr Glu Asp Thr Leu Thr
   675 680 685
   Leu Phe Pro Phe Ser Gly Glu Thr Val Phe Met Ser Met Glu Asn Pro
   690 695 700
   Gly Leu Trp Ile Leu Gly Cys His Asn Ser Asp Phe Arg Asn Arg Gly
   705 710 715 720
   Met Thr Ala Leu Leu Lys Val Ser Ser Cys Asp Lys Asn Thr Gly Asp
   725 730 735
   Tyr Tyr Glu Asp Ser Tyr Glu Asp Ile Ser Ala Tyr Leu Leu Ser Lys
   740 745 750
   Asn Asn Ala Ile Glu Pro Arg Ser Phe Ser Gln Asn Pro Pro Val Leu
   755 760 765
   Lys Arg His Gln Arg Glu Ile Thr Arg Thr Thr Leu Gln Ser Asp Gln
   770 775 780
   Glu Glu Ile Asp Tyr Asp Asp Thr Ile Ser Val Glu Met Lys Lys Glu
   785 790 795 800
   Asp Phe Asp Ile Tyr Asp Glu Asp Glu Asn Gln Ser Pro Arg Ser Phe
   805 810 815
   Gln Lys Lys Thr Arg His Tyr Phe Ile Ala Ala Val Glu Arg Leu Trp
   820 825 830
   Asp Tyr Gly Met Ser Ser Ser Pro His Val Leu Arg Asn Arg Ala Gln
   835 840 845
   Ser Gly Ser Val Pro Gln Phe Lys Lys Val Val Phe Gln Glu Phe Thr
   850 855 860
   Asp Gly Ser Phe Thr Gln Pro Leu Tyr Arg Gly Glu Leu Asn Glu His
   865 870 875 880
   Leu Gly Leu Leu Gly Pro Tyr Ile Arg Ala Glu Val Glu Asp Asn Ile
   885 890 895
   Met Val Thr Phe Arg Asn Gln Ala Ser Arg Pro Tyr Ser Phe Tyr Ser
   900 905 910
   Ser Leu Ile Ser Tyr Glu Glu Asp Gln Arg Gln Gly Ala Glu Pro Arg
   915 920 925
   Lys Asn Phe Val Lys Pro Asn Glu Thr Lys Thr Tyr Phe Trp Lys Val
   930 935 940
   Gln His His Met Ala Pro Thr Lys Asp Glu Phe Asp Cys Lys Ala Trp
   945 950 955 960
   Ala Tyr Phe Ser Asp Val Asp Leu Glu Lys Asp Val His Ser Gly Leu
   965 970 975
   Ile Gly Pro Leu Leu Val Cys His Thr Asn Thr Leu Asn Pro Ala His
   980 985 990
   Gly Arg Gln Val Thr Val Gln Glu Phe Ala Leu Phe Phe Thr Ile Phe
   995 1000 1005
   Asp Glu Thr Lys Ser Trp Tyr Phe Thr Glu Asn Met Glu Arg Asn
   1010 1015 1020
   Cys Arg Ala Pro Cys Asn Ile Gln Met Glu Asp Pro Thr Phe Lys
   1025 1030 1035
   Glu Asn Tyr Arg Phe His Ala Ile Asn Gly Tyr Ile Met Asp Thr
   1040 1045 1050
   Leu Pro Gly Leu Val Met Ala Gln Asp Gln Arg Ile Arg Trp Tyr
   1055 1060 1065
   Leu Leu Ser Met Gly Ser Asn Glu Asn Ile His Ser Ile His Phe
   1070 1075 1080
   Ser Gly His Val Phe Thr Val Arg Lys Lys Glu Glu Tyr Lys Met
   1085 1090 1095
   Ala Leu Tyr Asn Leu Tyr Pro Gly Val Phe Glu Thr Val Glu Met
   1100 1105 1110
   Leu Pro Ser Lys Ala Gly Ile Trp Arg Val Glu Cys Leu Ile Gly
   1115 1120 1125
   Glu His Leu His Ala Gly Met Ser Thr Leu Phe Leu Val Tyr Ser
   1130 1135 1140
   Asn Lys Cys Gln Thr Pro Leu Gly Met Ala Ser Gly His Ile Arg
   1145 1150 1155
   Asp Phe Gln Ile Thr Ala Ser Gly Gln Tyr Gly Gln Trp Ala Pro
   1160 1165 1170
   Lys Leu Ala Arg Leu His Tyr Ser Gly Ser Ile Asn Ala Trp Ser
   1175 1180 1185
   Thr Lys Glu Pro Phe Ser Trp Ile Lys Val Asp Leu Leu Ala Pro
   1190 1195 1200
   Met Ile Ile His Gly Ile Lys Thr Gln Gly Ala Arg Gln Lys Phe
   1205 1210 1215
   Ser Ser Leu Tyr Ile Ser Gln Phe Ile Ile Met Tyr Ser Leu Asp
   1220 1225 1230
   Gly Lys Lys Trp Gln Thr Tyr Arg Gly Asn Ser Thr Gly Thr Leu
   1235 1240 1245
   Met Val Phe Phe Gly Asn Val Asp Ser Ser Gly Ile Lys His Asn
   1250 1255 1260
   Ile Phe Asn Pro Pro Ile Ile Ala Arg Tyr Ile Arg Leu His Pro
   1265 1270 1275
   Thr His Tyr Ser Ile Arg Ser Thr Leu Arg Met Glu Leu Met Gly
   1280 1285 1290
   Cys Asp Leu Asn Ser Cys Ser Met Pro Leu Gly Met Glu Ser Lys
   1295 1300 1305
   Ala Ile Ser Asp Ala Gln Ile Thr Ala Ser Ser Tyr Phe Thr Asn
   1310 1315 1320
   Met Phe Ala Thr Trp Ser Pro Ser Lys Ala Arg Leu His Leu Gln
       1325                    1330                    1335
   Gly Arg  Ser Asn Ala Trp Arg  Pro Gln Val Asn Asn  Pro Lys Glu
       1340                    1345                    1350
   Trp Leu  Gln Val Asp Phe Gln  Lys Thr Met Lys Val  Thr Gly Val
       1355                    1360                    1365
   Thr Thr  Gln Gly Val Lys Ser  Leu Leu Thr Ser Met  Tyr Val Lys
       1370                    1375                    1380
   Glu Phe  Leu Ile Ser Ser Ser  Gln Asp Gly His Gln  Trp Thr Leu
       1385                    1390                    1395
   Phe Phe  Gln Asn Gly Lys Val  Lys Val Phe Gln Gly  Asn Gln Asp
       1400                    1405                    1410
   Ser Phe  Thr Pro Val Val Asn  Ser Leu Asp Pro Pro  Leu Leu Thr
       1415                    1420                    1425
   Arg Tyr  Leu Arg Ile His Pro  Gln Ser Trp Val His  Gln Ile Ala
       1430                    1435                    1440
   Leu Arg  Met Glu Val Leu Gly  Cys Glu Ala Gln Asp  Leu Tyr
       1445                    1450                    1455
<210> 3
   <211> 1464
   <212> PRT
   <213> homo sapiens
<400> 3
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                      80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
   100 105 110
   His Pro Val Ser Leu His Ala Val Gly Val Ser Tyr Trp Lys Ala Ser
   115 120 125
   Glu Gly Ala Glu Tyr Asp Asp Gln Thr Ser Gln Arg Glu Lys Glu Asp
   130 135 140
   Asp Lys Val Phe Pro Gly Gly Ser His Thr Tyr Val Trp Gln Val Leu
   145 150 155 160
   Lys Glu Asn Gly Pro Met Ala Ser Asp Pro Leu Cys Leu Thr Tyr Ser
   165 170 175
   Tyr Leu Ser His Val Asp Leu Val Lys Asp Leu Asn Ser Gly Leu Ile
   180 185 190
   Gly Ala Leu Leu Val Cys Arg Glu Gly Ser Leu Ala Lys Glu Lys Thr
   195 200 205
   Gln Thr Leu His Lys Phe Ile Leu Leu Phe Ala Val Phe Asp Glu Gly
   210 215 220
   Lys Ser Trp His Ser Glu Thr Lys Asn Ser Leu Met Gln Asp Arg Asp
   225 230 235 240
   Ala Ala Ser Ala Arg Ala Trp Pro Lys Met His Thr Val Asn Gly Tyr
   245 250 255
   Val Asn Arg Ser Leu Pro Gly Leu Ile Gly Cys His Arg Lys Ser Val
   260 265 270
   Tyr Trp His Val Ile Gly Met Gly Thr Thr Pro Glu Val His Ser Ile
   275 280 285
   Phe Leu Glu Gly His Thr Phe Leu Val Arg Asn His Arg Gln Ala Ser
   290 295 300
   Leu Glu Ile Ser Pro Ile Thr Phe Leu Thr Ala Gln Thr Leu Leu Met
   305 310 315 320
   Asp Leu Gly Gln Phe Leu Leu Phe Cys His Ile Ser Ser His Gln His
   325 330 335
   Asp Gly Met Glu Ala Tyr Val Lys Val Asp Ser Cys Pro Glu Glu Pro
   340 345 350
   Gln Leu Arg Met Lys Asn Asn Glu Glu Ala Glu Asp Tyr Asp Asp Asp
   355 360 365
   Leu Thr Asp Ser Glu Met Asp Val Val Arg Phe Asp Asp Asp Asn Ser
   370 375 380
   Pro Ser Phe Ile Gln Ile Arg Ser Val Ala Lys Lys His Pro Lys Thr
   385 390 395 400
   Trp Val His Tyr Ile Ala Ala Glu Glu Glu Asp Trp Asp Tyr Ala Pro
   405 410 415
   Leu Val Leu Ala Pro Asp Asp Arg Ser Tyr Lys Ser Gln Tyr Leu Asn
   420 425 430
   Asn Gly Pro Gln Arg Ile Gly Arg Lys Tyr Lys Lys Val Arg Phe Met
   435 440 445
   Ala Tyr Thr Asp Glu Thr Phe Lys Thr Arg Glu Ala Ile Gln His Glu
   450 455 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
   500 505 510
   Gly Val Lys His Leu Lys Asp Phe Pro Ile Leu Pro Gly Glu Ile Phe
   515 520 525
   Lys Tyr Lys Trp Thr Val Thr Val Glu Asp Gly Pro Thr Lys Ser Asp
   530 535 540
   Pro Arg Cys Leu Thr Arg Tyr Tyr Ser Ser Phe Val Asn Met Glu Arg
   545 550 555 560
   Asp Leu Ala Ser Gly Leu Ile Gly Pro Leu Leu Ile Cys Tyr Lys Glu
   565 570 575
   Ser Val Asp Gln Arg Gly Asn Gln Ile Met Ser Asp Lys Arg Asn Val
   580 585 590
   Ile Leu Phe Ser Val Phe Asp Glu Asn Arg Ser Trp Tyr Leu Thr Glu
   595 600 605
   Asn Ile Gln Arg Phe Leu Pro Asn Pro Ala Gly Val Gln Leu Glu Asp
   610 615 620
   Pro Glu Phe Gln Ala Ser Asn Ile Met His Ser Ile Asn Gly Tyr Val
   625 630 635 640
   Phe Asp Ser Leu Gln Leu Ser Val Cys Leu His Glu Val Ala Tyr Trp
   645 650 655
   Tyr Ile Leu Ser Ile Gly Ala Gln Thr Asp Phe Leu Ser Val Phe Phe
   660 665 670
   Ser Gly Tyr Thr Phe Lys His Lys Met Val Tyr Glu Asp Thr Leu Thr
   675 680 685
   Leu Phe Pro Phe Ser Gly Glu Thr Val Phe Met Ser Met Glu Asn Pro
   690 695 700
   Gly Leu Trp Ile Leu Gly Cys His Asn Ser Asp Phe Arg Asn Arg Gly
   705 710 715 720
   Met Thr Ala Leu Leu Lys Val Ser Ser Cys Asp Lys Asn Thr Gly Asp
   725 730 735
   Tyr Tyr Glu Asp Ser Tyr Glu Asp Ile Ser Ala Tyr Leu Leu Ser Lys
   740 745 750
   Asn Asn Ala Ile Glu Pro Arg Ser Phe Ser Gln Asn Ser Arg His Pro
   755 760 765
   Ser Gln Asn Pro Pro Val Leu Lys Arg His Gln Arg Glu Ile Thr Arg
   770 775 780
   Thr Thr Leu Gln Ser Asp Gln Glu Glu Ile Asp Tyr Asp Asp Thr Ile
   785 790 795 800
   Ser Val Glu Met Lys Lys Glu Asp Phe Asp Ile Tyr Asp Glu Asp Glu
   805 810 815
   Asn Gln Ser Pro Arg Ser Phe Gln Lys Lys Thr Arg His Tyr Phe Ile
   820 825 830
   Ala Ala Val Glu Arg Leu Trp Asp Tyr Gly Met Ser Ser Ser Pro His
   835 840 845
   Val Leu Arg Asn Arg Ala Gln Ser Gly Ser Val Pro Gln Phe Lys Lys
   850 855 860
   Val Val Phe Gln Glu Phe Thr Asp Gly Ser Phe Thr Gln Pro Leu Tyr
   865 870 875 880
   Arg Gly Glu Leu Asn Glu His Leu Gly Leu Leu Gly Pro Tyr Ile Arg
   885 890 895
   Ala Glu Val Glu Asp Asn Ile Met Val Thr Phe Arg Asn Gln Ala Ser
   900 905 910
   Arg Pro Tyr Ser Phe Tyr Ser Ser Leu Ile Ser Tyr Glu Glu Asp Gln
   915 920 925
   Arg Gln Gly Ala Glu Pro Arg Lys Asn Phe Val Lys Pro Asn Glu Thr
   930 935 940
   Lys Thr Tyr Phe Trp Lys Val Gln His His Met Ala Pro Thr Lys Asp
   945 950 955 960
   Glu Phe Asp Cys Lys Ala Trp Ala Tyr Phe Ser Asp Val Asp Leu Glu
   965 970 975
   Lys Asp Val His Ser Gly Leu Ile Gly Pro Leu Leu Val Cys His Thr
   980 985 990
   Asn Thr Leu Asn Pro Ala His Gly Arg Gln Val Thr Val Gln Glu Phe
   995 1000 1005
   Ala Leu Phe Phe Thr Ile Phe Asp Glu Thr Lys Ser Trp Tyr Phe
   1010 1015 1020
   Thr Glu Asn Met Glu Arg Asn Cys Arg Ala Pro Cys Asn Ile Gln
   1025 1030 1035
   Met Glu Asp Pro Thr Phe Lys Glu Asn Tyr Arg Phe His Ala Ile
   1040 1045 1050
   Asn Gly Tyr Ile Met Asp Thr Leu Pro Gly Leu Val Met Ala Gln
   1055 1060 1065
   Asp Gln Arg Ile Arg Trp Tyr Leu Leu Ser Met Gly Ser Asn Glu
   1070 1075 1080
   Asn Ile His Ser Ile His Phe Ser Gly His Val Phe Thr Val Arg
   1085 1090 1095
   Lys Lys Glu Glu Tyr Lys Met Ala Leu Tyr Asn Leu Tyr Pro Gly
   1100 1105 1110
   Val Phe Glu Thr Val Glu Met Leu Pro Ser Lys Ala Gly Ile Trp
   1115 1120 1125
   Arg Val Glu Cys Leu Ile Gly Glu His Leu His Ala Gly Met Ser
   1130 1135 1140
   Thr Leu Phe Leu Val Tyr Ser Asn Lys Cys Gln Thr Pro Leu Gly
   1145 1150 1155
   Met Ala Ser Gly His Ile Arg Asp Phe Gln Ile Thr Ala Ser Gly
   1160 1165 1170
   Gln Tyr Gly Gln Trp Ala Pro Lys Leu Ala Arg Leu His Tyr Ser
   1175 1180 1185
   Gly Ser Ile Asn Ala Trp Ser Thr Lys Glu Pro Phe Ser Trp Ile
   1190 1195 1200
   Lys Val Asp Leu Leu Ala Pro Met Ile Ile His Gly Ile Lys Thr
   1205 1210 1215
   Gln Gly Ala Arg Gln Lys Phe Ser Ser Leu Tyr Ile Ser Gln Phe
   1220 1225 1230
   Ile Ile Met Tyr Ser Leu Asp Gly Lys Lys Trp Gln Thr Tyr Arg
   1235 1240 1245
   Gly Asn Ser Thr Gly Thr Leu Met Val Phe Phe Gly Asn Val Asp
   1250 1255 1260
   Ser Ser Gly Ile Lys His Asn Ile Phe Asn Pro Pro Ile Ile Ala
   1265 1270 1275
   Arg Tyr Ile Arg Leu His Pro Thr His Tyr Ser Ile Arg Ser Thr
   1280 1285 1290
   Leu Arg Met Glu Leu Met Gly Cys Asp Leu Asn Ser Cys Ser Met
   1295 1300 1305
   Pro Leu Gly Met Glu Ser Lys Ala Ile Ser Asp Ala Gln Ile Thr
   1310 1315 1320
   Ala Ser Ser Tyr Phe Thr Asn Met Phe Ala Thr Trp Ser Pro Ser
   1325 1330 1335
   Lys Ala Arg Leu His Leu Gln Gly Arg Ser Asn Ala Trp Arg Pro
   1340 1345 1350
   Gln Val Asn Asn Pro Lys Glu Trp Leu Gln Val Asp Phe Gln Lys
   1355 1360 1365
   Thr Met Lys Val Thr Gly Val Thr Thr Gln Gly Val Lys Ser Leu
   1370 1375 1380
   Leu Thr Ser Met Tyr Val Lys Glu Phe Leu Ile Ser Ser Ser Gln
   1385 1390 1395
   Asp Gly His Gln Trp Thr Leu Phe Phe Gln Asn Gly Lys Val Lys
   1400 1405 1410
   Val Phe Gln Gly Asn Gln Asp Ser Phe Thr Pro Val Val Asn Ser
   1415 1420 1425
   Leu Asp Pro Pro Leu Leu Thr Arg Tyr Leu Arg Ile His Pro Gln
   1430 1435 1440
   Ser Trp Val His Gln Ile Ala Leu Arg Met Glu Val Leu Gly Cys
   1445 1450 1455
   Glu Ala Gln Asp Leu Tyr
   1460
<210> 4
   <211> 21
   <212> PRT
   <213> homo sapiens
<400> 4
   Ser Phe Ser Gln Asn Ser Arg His Pro Ser Gln Asn Pro Pro Val Leu
   1 5 10 15
   Lys Arg His Gln Arg
   20
<210> 5
   <211> 28
   <212> PRT
   <213> artificial
<220>
   <223> HIS tagged FVIII B domain linker
<400> 5
   Ser Phe Ser Gln Asn Ser Arg His Pro Ser His His His His His His
   1 5 10 15
   Ser Gln Asn Pro Pro Val Leu Lys Arg His Gln Arg
   20 25
<210> 6
   <211> 28
   <212> PRT
   <213> artificial
<220>
   <223> HIS tagged FVIII B domain linker
<400> 6
   Ser Phe Ser Gln Asn Ser Arg His Pro Ser His His His His His His
   1               5                   10                  15
   Ser Gln Asn Pro Pro Val Leu Lys Arg His Gln Arg
               20                  25
<210> 7
   <211> 44
   <212> DNA
   <213> artificial
<220>
   <223> Primer
<400> 7
   ctaatacgac tcactatagg gcaagcagtg gtatcacgca gagt 44
<210> 8
   <211> 22
   <212> DNA
   <213> artificial
<220>
   <223> Primer
<400> 8
   ctaatacgac tcactatagg gc 22
<210> 9
   <211> 36
   <212> DNA
   <213> artificial
<220>
   <223> Primer
<400> 9
   gctctagact aacactcatt cctgttgaag ctcttg 36
<210> 10
   <211> 44
   <212> DNA
   <213> artificial
<220>
   <223> Primer
<400> 10
   ctaatacgac tcactatagg gcaagcagtg gtatcacgca gagt 44
<210> 11
   <211> 22
   <212> DNA
   <213> artificial
<220>
   <223> Primer
<400> 11
   ctaatacgac tcactatagg gc 22
<210> 12
   <211> 21
   <212> DNA
   <213> artificial
<220>
   <223> Primer
<400> 12
   gtctaccaca acacacgtga c 21
<210> 13
   <211> 33
   <212> DNA
   <213> artificial
<220>
   <223> Primer
<400> 13
   gactttttgt atgaattcct caccatgagg tgc 33
<210> 14
   <211> 28
   <212> DNA
   <213> artificial
<220>
   <223> Primer
<400> 14
   caacacttac ttgtcctggt tcctgcag 28
<210> 15
   <211> 28
   <212> DNA
   <213> artificial
<220>
   <223> Primer
<400> 15
   ctgcaggaac caggacaagt aagtgttg 28
<210> 16
   <211> 444
   <212> PRT
   <213> mus musculus
<400> 16
   Gln Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Asn Lys Tyr Asn Glu Asn Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Glu Tyr Gly Asn Tyr Asp Tyr Ala Met Asp Ser Trp Gly Gln
               100                 105                 110
   Gly Thr Ser Val Thr Val Ser Ser Ala Lys Thr Thr Pro Pro Ser Val
           115                 120                 125
   Tyr Pro Leu Ala Pro Gly Ser Ala Ala Gln Thr Asn Ser Met Val Thr
       130                 135                 140
   Leu Gly Cys Leu Val Lys Gly Tyr Phe Pro Glu Pro Val Thr Val Thr
   145                 150                 155                 160
   Trp Asn Ser Gly Ser Leu Ser Ser Gly Val His Thr Phe Pro Ala Val
                   165                 170                 175
   Leu Gln Ser Asp Leu Tyr Thr Leu Ser Ser Ser Val Thr Val Pro Ser
               180                 185                 190
   Ser Thr Trp Pro Ser Glu Thr Val Thr Cys Asn Val Ala His Pro Ala
           195                 200                 205
   Ser Ser Thr Lys Val Asp Lys Lys Ile Val Pro Arg Asp Cys Gly Cys
       210                 215                 220
   Lys Pro Cys Ile Cys Thr Val Pro Glu Val Ser Ser Val Phe Ile Phe
   225                 230                 235                 240
   Pro Pro Lys Pro Lys Asp Val Leu Thr Ile Thr Leu Thr Pro Lys Val
                   245                 250                 255
   Thr Cys Val Val Val Asp Ile Ser Lys Asp Asp Pro Glu Val Gln Phe
               260                 265                 270
   Ser Trp Phe Val Asp Asp Val Glu Val His Thr Ala Gln Thr Gln Pro
           275                 280                 285
   Arg Glu Glu Gln Phe Asn Ser Thr Phe Arg Ser Val Ser Glu Leu Pro
       290                 295                 300
   Ile Met His Gln Asp Trp Leu Asn Gly Lys Glu Phe Lys Cys Arg Val
   305                 310                 315                 320
   Asn Ser Ala Ala Phe Pro Ala Pro Ile Glu Lys Thr Ile Ser Lys Thr
                   325                 330                 335
   Lys Gly Arg Pro Lys Ala Pro Gln Val Tyr Thr Ile Pro Pro Pro Lys
               340                 345                 350
   Glu Gln Met Ala Lys Asp Lys Val Ser Leu Thr Cys Met Ile Thr Asp
           355                 360                 365
   Phe Phe Pro Glu Asp Ile Thr Val Glu Trp Gln Trp Asn Gly Gln Pro
       370                 375                 380
   Ala Glu Asn Tyr Lys Asn Thr Gln Pro Ile Met Asp Thr Asp Gly Ser
   385                 390                 395                 400
   Tyr Phe Val Tyr Ser Lys Leu Asn Val Gln Lys Ser Asn Trp Glu Ala
                   405                 410                 415
   Gly Asn Thr Phe Thr Cys Ser Val Leu His Glu Gly Leu His Asn His
               420                 425                 430
   His Thr Glu Lys Ser Leu Ser His Ser Pro Gly Lys
           435                 440
<210> 17
   <211> 219
   <212> PRT
   <213> mus musculus
<400> 17
   Asp Ile Val Met Thr Gln Ala Ala Pro Ser Val Pro Val Thr Pro Gly
   1               5                   10                  15
   Glu Ser Val Ser Ile Ser Cys Arg Ser Ser Arg Ser Leu Leu His Ser
               20                  25                  30
   Asn Gly Asn Thr Tyr Leu Cys Trp Phe Leu Gln Arg Pro Gly Gln Ser
           35                  40                  45
   Pro Gln Leu Leu Ile Tyr Arg Met Ser Asn Leu Ala Ser Gly Val Pro
   50 55 60
   Asp Arg Phe Ser Gly Ser Gly Ser Gly Thr Ala Phe Thr Leu Arg Ile
   65 70 75 80
   Ser Arg Val Glu Ala Glu Asp Val Gly Val Tyr Tyr Cys Met Gln His
   85 90 95
   Leu Glu Tyr Pro Phe Thr Phe Gly Ser Gly Thr Lys Leu Glu Ile Lys
   100 105 110
   Arg Ala Asp Ala Ala Pro Thr Val Ser Ile Phe Pro Pro Ser Ser Glu
   115 120 125
   Gln Leu Thr Ser Gly Gly Ala Ser Val Val Cys Phe Leu Asn Asn Phe
   130 135 140
   Tyr Pro Lys Asp Ile Asn Val Lys Trp Lys Ile Asp Gly Ser Glu Arg
   145 150 155 160
   Gln Asn Gly Val Leu Asn Ser Trp Thr Asp Gln Asp Ser Lys Asp Ser
   165 170 175
   Thr Tyr Ser Met Ser Ser Thr Leu Thr Leu Thr Lys Asp Glu Tyr Glu
   180 185 190
   Arg His Asn Asn Tyr Thr Cys Glu Ala Thr His Lys Thr Ser Thr Ser
   195 200 205
   Pro Ile Val Lys Ser Phe Asn Arg Asn Glu Cys
   210 215
<210> 18
   <211> 219
   <212> PRT
   <213> mus musculus
<400> 18
   Asp Ile Val Met Thr Gln Ala Ala Pro Ser Val Pro Val Thr Pro Gly
   1               5                   10                  15
   Glu Ser Val Ser Ile Ser Cys Arg Ser Ser Arg Ser Leu Leu His Ser
               20                  25                  30
   Asn Gly Asn Thr Tyr Leu Ser Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Phe Thr Phe Gly Ser Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Asn Tyr Thr Cys Glu Ala Thr His Lys Thr Ser Thr Ser
           195                 200                 205
   Pro Ile Val Lys Ser Phe Asn Arg Asn Glu Cys
       210                 215
<210> 19
   <211> 28
   <212> DNA
   <213> artificial
<220>
   <223> Primer
<400> 19
   caacacttac ttgtcctggt tcctgcag 28
<210> 20
   <211> 28
   <212> DNA
   <213> artificial
<220>
   <223> Primer
<400> 20
   ctgcaggaac caggacaagt aagtgttg 28
<210> 21
   <211> 256
   <212> PRT
   <213> mus musculus
<400> 21
   Asp Ile Val Met Thr Gln Ala Ala Pro Ser Val Pro Val Thr Pro Gly
   1 5 10 15
   Glu Ser Val Ser Ile Ser Cys Arg Ser Ser Arg Ser Leu Leu His Ser
   20 25 30
   Asn Gly Asn Thr Tyr Leu Cys Trp Phe Leu Gln Arg Pro Gly Gln Ser
   35 40 45
   Pro Gln Leu Leu Ile Tyr Arg Met Ser Asn Leu Ala Ser Gly Val Pro
   50 55 60
   Asp Arg Phe Ser Gly Ser Gly Ser Gly Thr Ala Phe Thr Leu Arg Ile
   65 70 75 80
   Ser Arg Val Glu Ala Glu Asp Val Gly Val Tyr Tyr Cys Met Gln His
   85 90 95
   Leu Glu Tyr Pro Phe Thr Phe Gly Ser Gly Thr Lys Leu Glu Ile Lys
   100 105 110
   Arg Gly Gly Gly Gly Ser Gly Gly Gly Gly Ser Gly Gly Gly Gly Ser
   115 120 125
   Gln Val Gln Leu Gln Gln Ser Gly Ala Glu Leu Val Arg Pro Gly Thr
   130 135 140
   Ser Val Lys Ile Ser Cys Lys Ala Ser Gly Tyr Thr Phe Thr Asn Tyr
   145 150 155 160
   Trp Leu Gly Trp Val Lys Gln Arg Pro Gly His Gly Leu Glu Trp Ile
   165 170 175
   Gly Asp Ile Tyr Pro Gly Gly Gly Tyr Asn Lys Tyr Asn Glu Asn Phe
   180 185 190
   Lys Gly Lys Ala Thr Leu Thr Ala Asp Thr Ser Ser Ser Thr Ala Tyr
   195 200 205
   Met Gln Leu Ser Ser Leu Thr Ser Glu Asp Ser Ala Val Tyr Phe Cys
   210 215 220
   Ala Arg Glu Tyr Gly Asn Tyr Asp Tyr Ala Met Asp Ser Trp Gly Gln
   225 230 235 240
   Gly Thr Ser Val Thr Val Ser Ser Asp Tyr Lys Asp Asp Asp Asp Lys
   245 250 255
<210> 22
   <211> 256
   <212> PRT
   <213> mus musculus
<400> 22
   Gln Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Asn Lys Tyr Asn Glu Asn Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Glu Tyr Gly Asn Tyr Asp Tyr Ala Met Asp Ser Trp Gly Gln
               100                 105                 110
   Gly Thr Ser Val Thr Val Ser Ser Gly Gly Gly Gly Ser Gly Gly Gly
           115                 120                 125
   Gly Ser Gly Gly Gly Gly Ser Asp Ile Val Met Thr Gln Ala Ala Pro
       130                 135                 140
   Ser Val Pro Val Thr Pro Gly Glu Ser Val Ser Ile Ser Cys Arg Ser
   145                 150                 155                 160
   Ser Arg Ser Leu Leu His Ser Asn Gly Asn Thr Tyr Leu Cys Trp Phe
                   165                 170                 175
   Leu Gln Arg Pro Gly Gln Ser Pro Gln Leu Leu Ile Tyr Arg Met Ser
               180                 185                 190
   Asn Leu Ala Ser Gly Val Pro Asp Arg Phe Ser Gly Ser Gly Ser Gly
           195                 200                 205
   Thr Ala Phe Thr Leu Arg Ile Ser Arg Val Glu Ala Glu Asp Val Gly
       210                 215                 220
   Val Tyr Tyr Cys Met Gln His Leu Glu Tyr Pro Phe Thr Phe Gly Ser
   225                 230                 235                 240
   Gly Thr Lys Leu Glu Ile Lys Arg Asp Tyr Lys Asp Asp Asp Asp Lys
                   245                 250                 255
<210> 23
   <211> 256
   <212> PRT
   <213> mus musculus
<400> 23
   Asp Ile Val Met Thr Gln Ala Ala Pro Ser Val Pro Val Thr Pro Gly
   1               5                   10                  15
   Glu Ser Val Ser Ile Ser Cys Arg Ser Ser Arg Ser Leu Leu His Ser
               20                  25                  30
   Asn Gly Asn Thr Tyr Leu Ser Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Phe Thr Phe Gly Ser Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gln Val Gln Leu Gln Gln Ser Gly Ala Glu Leu Val Arg Pro Gly Thr
       130                 135                 140
   Ser Val Lys Ile Ser Cys Lys Ala Ser Gly Tyr Thr Phe Thr Asn Tyr
   145                 150                 155                 160
   Trp Leu Gly Trp Val Lys Gln Arg Pro Gly His Gly Leu Glu Trp Ile
                   165                 170                 175
   Gly Asp Ile Tyr Pro Gly Gly Gly Tyr Asn Lys Tyr Asn Glu Asn Phe
   180 185 190
   Lys Gly Lys Ala Thr Leu Thr Ala Asp Thr Ser Ser Ser Thr Ala Tyr
   195 200 205
   Met Gln Leu Ser Ser Leu Thr Ser Glu Asp Ser Ala Val Tyr Phe Cys
   210 215 220
   Ala Arg Glu Tyr Gly Asn Tyr Asp Tyr Ala Met Asp Ser Trp Gly Gln
   225 230 235 240
   Gly Thr Ser Val Thr Val Ser Ser Asp Tyr Lys Asp Asp Asp Asp Lys
   245 250 255
<210> 24
   <211> 31
   <212> DNA
   <213> artificial
<220>
   <223> Primer
<400> 24
   cgacgacgac aagtgctgaa agcttcgtac g 31
<210> 25
   <211> 31
   <212> DNA
   <213> artificial
<220>
   <223> Primer
<400> 25
   cgtacgaagc tttcagcact tgtcgtcgtc g 31
<210> 26
   <211> 27
   <212> DNA
   <213> artificial
<220>
   <223> Primer
<400> 26
   gtgaccgtga gctgcgacta caaggac 27
<210> 27
   <211> 27
   <212> DNA
   <213> artificial
<220>
   <223> Primer
<400> 27
   gtccttgtag tcgcagctca cggtcac 27
<210> 28
   <211> 28
   <212> DNA
   <213> artificial
<220>
   <223> Primer
<400> 28
   caacacttac ttgtcctggt tcctgcag 28
<210> 29
   <211> 28
   <212> DNA
   <213> artificial
<220>
   <223> Primer
<400> 29
   ctgcaggaac caggacaagt aagtgttg 28
<210> 30
   <211> 257
   <212> PRT
   <213> mus musculus
<400> 30
   Asp Ile Val Met Thr Gln Ala Ala Pro Ser Val Pro Val Thr Pro Gly
   1               5                   10                  15
   Glu Ser Val Ser Ile Ser Cys Arg Ser Ser Arg Ser Leu Leu His Ser
               20                  25                  30
   Asn Gly Asn Thr Tyr Leu Cys Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Phe Thr Phe Gly Ser Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gln Val Gln Leu Gln Gln Ser Gly Ala Glu Leu Val Arg Pro Gly Thr
   130 135 140
   Ser Val Lys Ile Ser Cys Lys Ala Ser Gly Tyr Thr Phe Thr Asn Tyr
   145 150 155 160
   Trp Leu Gly Trp Val Lys Gln Arg Pro Gly His Gly Leu Glu Trp Ile
   165 170 175
   Gly Asp Ile Tyr Pro Gly Gly Gly Tyr Asn Lys Tyr Asn Glu Asn Phe
   180 185 190
   Lys Gly Lys Ala Thr Leu Thr Ala Asp Thr Ser Ser Ser Thr Ala Tyr
   195 200 205
   Met Gln Leu Ser Ser Leu Thr Ser Glu Asp Ser Ala Val Tyr Phe Cys
   210 215 220
   Ala Arg Glu Tyr Gly Asn Tyr Asp Tyr Ala Met Asp Ser Trp Gly Gln
   225 230 235 240
   Gly Thr Ser Val Thr Val Ser Ser Asp Tyr Lys Asp Asp Asp Asp Lys
   245 250 255
   Cys
<210> 31
   <211> 256
   <212> PRT
   <213> mus musculus
<400> 31
   Asp Ile Val Met Thr Gln Ala Ala Pro Ser Val Pro Val Thr Pro Gly
   1               5                   10                  15
   Glu Ser Val Ser Ile Ser Cys Arg Ser Ser Arg Ser Leu Leu His Ser
               20                  25                  30
   Asn Gly Asn Thr Tyr Leu Ser Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Phe Thr Phe Gly Ser Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gln Val Gln Leu Gln Gln Ser Gly Ala Glu Leu Val Arg Pro Gly Thr
       130                 135                 140
   Ser Val Lys Ile Ser Cys Lys Ala Ser Gly Tyr Thr Phe Thr Asn Tyr
   145                 150                 155                 160
   Trp Leu Gly Trp Val Lys Gln Arg Pro Gly His Gly Leu Glu Trp Ile
                   165                 170                 175
   Gly Asp Ile Tyr Pro Gly Gly Gly Tyr Asn Lys Tyr Asn Glu Asn Phe
               180                 185                 190
   Lys Gly Lys Ala Thr Leu Thr Ala Asp Thr Ser Ser Ser Thr Ala Tyr
           195                 200                 205
   Met Gln Leu Ser Ser Leu Thr Ser Glu Asp Ser Ala Val Tyr Phe Cys
       210                 215                 220
   Ala Arg Glu Tyr Gly Asn Tyr Asp Tyr Ala Met Asp Ser Trp Gly Gln
   225                 230                 235                 240
   Gly Thr Ser Val Thr Val Ser Cys Asp Tyr Lys Asp Asp Asp Asp Lys
                   245                 250                 255
<210> 32
   <211> 30
   <212> DNA
   <213> artificial
<220>
   <223> primer
<400> 32
   cagggattgt ggttgaaagc cttgcatatg 30
<210> 33
   <211> 30
   <212> DNA
   <213> artificial
<220>
   <223> primer
<400> 33
   catatgcaag gctttcaacc acaatccctg 30
<210> 34
   <211> 223
   <212> PRT
   <213> mus musculus
<400> 34
   Gln Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Asn Lys Tyr Asn Glu Asn Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Glu Tyr Gly Asn Tyr Asp Tyr Ala Met Asp Ser Trp Gly Gln
               100                 105                 110
   Gly Thr Ser Val Thr Val Ser Ser Ala Lys Thr Thr Pro Pro Ser Val
           115                 120                 125
   Tyr Pro Leu Ala Pro Gly Ser Ala Ala Gln Thr Asn Ser Met Val Thr
       130                 135                 140
   Leu Gly Cys Leu Val Lys Gly Tyr Phe Pro Glu Pro Val Thr Val Thr
   145                 150                 155                 160
   Trp Asn Ser Gly Ser Leu Ser Ser Gly Val His Thr Phe Pro Ala Val
                   165                 170                 175
   Leu Gln Ser Asp Leu Tyr Thr Leu Ser Ser Ser Val Thr Val Pro Ser
               180                 185                 190
   Ser Thr Trp Pro Ser Glu Thr Val Thr Cys Asn Val Ala His Pro Ala
           195                 200                 205
   Ser Ser Thr Lys Val Asp Lys Lys Ile Val Pro Arg Asp Cys Gly
       210                 215                 220
<210> 35
   <211> 51
   <212> DNA
   <213> artificial
<220>
   <223> Primer
<400> 35
   aacccaccgg tcttgaaacg ccatcaacgg caggtccagc tgcagcagag c 51
<210> 36
   <211> 39
   <212> DNA
   <213> artificial
<220>
   <223> Primer
<400> 36
   gaaagctccg cgggctctgc cgcttgattt ccagcttgg 39
<210> 37
   <211> 51
   <212> DNA
   <213> artificial
<220>
   <223> Primer
<400> 37
   aacccaccgg tcttgaaacg ccatcaacgg gacatcgtga tgacccaggc t 51
<210> 38
   <211> 39
   <212> DNA
   <213> artificial
<220>
   <223> Primer
<400> 38
   gaaagctccg cgggctctgg ctgctcacgg tcacggagg 39
<210> 39
   <211> 1656
   <212> PRT
   <213> artificial
<220>
   <223> FVIII and AP3 fusion protein
<400> 39
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Asp His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
   290 295 300
   Gln Phe Leu Leu Phe Cys His Ile Ser Ser His Gln His Asp Gly Met
   305 310 315 320
   Glu Ala Tyr Val Lys Val Asp Ser Cys Pro Glu Glu Pro Gln Leu Arg
   325 330 335
   Met Lys Asn Asn Glu Glu Ala Glu Asp Tyr Asp Asp Asp Leu Thr Asp
   340 345 350
   Ser Glu Met Asp Val Val Arg Phe Asp Asp Asp Asn Ser Pro Ser Phe
   355 360 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Gln Asn
   740 745 750
   Pro Pro Val Leu Lys Arg His Gln Arg Asp Ile Val Met Thr Gln Ala
   755 760 765
   Ala Pro Ser Val Pro Val Thr Pro Gly Glu Ser Val Ser Ile Ser Cys
   770 775 780
   Arg Ser Ser Arg Ser Leu Leu His Ser Asn Gly Asn Thr Tyr Leu Cys
   785 790 795 800
   Trp Phe Leu Gln Arg Pro Gly Gln Ser Pro Gln Leu Leu Ile Tyr Arg
   805 810 815
   Met Ser Asn Leu Ala Ser Gly Val Pro Asp Arg Phe Ser Gly Ser Gly
   820 825 830
   Ser Gly Thr Ala Phe Thr Leu Arg Ile Ser Arg Val Glu Ala Glu Asp
   835 840 845
   Val Gly Val Tyr Tyr Cys Met Gln His Leu Glu Tyr Pro Phe Thr Phe
   850 855 860
   Gly Ser Gly Thr Lys Leu Glu Ile Lys Arg Gly Gly Gly Gly Ser Gly
   865 870 875 880
   Gly Gly Gly Ser Gly Gly Gly Gly Ser Gln Val Gln Leu Gln Gln Ser
   885 890 895
   Gly Ala Glu Leu Val Arg Pro Gly Thr Ser Val Lys Ile Ser Cys Lys
   900 905 910
   Ala Ser Gly Tyr Thr Phe Thr Asn Tyr Trp Leu Gly Trp Val Lys Gln
   915 920 925
   Arg Pro Gly His Gly Leu Glu Trp Ile Gly Asp Ile Tyr Pro Gly Gly
   930 935 940
   Gly Tyr Asn Lys Tyr Asn Glu Asn Phe Lys Gly Lys Ala Thr Leu Thr
   945 950 955 960
   Ala Asp Thr Ser Ser Ser Thr Ala Tyr Met Gln Leu Ser Ser Leu Thr
   965 970 975
   Ser Glu Asp Ser Ala Val Tyr Phe Cys Ala Arg Glu Tyr Gly Asn Tyr
   980 985 990
   Asp Tyr Ala Met Asp Ser Trp Gly Gln Gly Thr Ser Val Thr Val Ser
   995 1000 1005
   Ser Gln Ser Pro Arg Ser Phe Gln Lys Lys Thr Arg His Tyr Phe
   1010 1015 1020
   Ile Ala Ala Val Glu Arg Leu Trp Asp Tyr Gly Met Ser Ser Ser
   1025 1030 1035
   Pro His Val Leu Arg Asn Arg Ala Gln Ser Gly Ser Val Pro Gln
   1040 1045 1050
   Phe Lys Lys Val Val Phe Gln Glu Phe Thr Asp Gly Ser Phe Thr
   1055 1060 1065
   Gln Pro Leu Tyr Arg Gly Glu Leu Asn Glu His Leu Gly Leu Leu
   1070 1075 1080
   Gly Pro Tyr Ile Arg Ala Glu Val Glu Asp Asn Ile Met Val Thr
   1085 1090 1095
   Phe Arg Asn Gln Ala Ser Arg Pro Tyr Ser Phe Tyr Ser Ser Leu
   1100 1105 1110
   Ile Ser Tyr Glu Glu Asp Gln Arg Gln Gly Ala Glu Pro Arg Lys
   1115 1120 1125
   Asn Phe Val Lys Pro Asn Glu Thr Lys Thr Tyr Phe Trp Lys Val
   1130 1135 1140
   Gln His His Met Ala Pro Thr Lys Asp Glu Phe Asp Cys Lys Ala
   1145 1150 1155
   Trp Ala Tyr Phe Ser Asp Val Asp Leu Glu Lys Asp Val His Ser
   1160 1165 1170
   Gly Leu Ile Gly Pro Leu Leu Val Cys His Thr Asn Thr Leu Asn
   1175 1180 1185
   Pro Ala His Gly Arg Gln Val Thr Val Gln Glu Phe Ala Leu Phe
   1190 1195 1200
   Phe Thr Ile Phe Asp Glu Thr Lys Ser Trp Tyr Phe Thr Glu Asn
   1205 1210 1215
   Met Glu Arg Asn Cys Arg Ala Pro Cys Asn Ile Gln Met Glu Asp
   1220 1225 1230
   Pro Thr Phe Lys Glu Asn Tyr Arg Phe His Ala Ile Asn Gly Tyr
   1235 1240 1245
   Ile Met Asp Thr Leu Pro Gly Leu Val Met Ala Gln Asp Gln Arg
   1250 1255 1260
   Ile Arg Trp Tyr Leu Leu Ser Met Gly Ser Asn Glu Asn Ile His
   1265 1270 1275
   Ser Ile His Phe Ser Gly His Val Phe Thr Val Arg Lys Lys Glu
   1280 1285 1290
   Glu Tyr Lys Met Ala Leu Tyr Asn Leu Tyr Pro Gly Val Phe Glu
   1295 1300 1305
   Thr Val Glu Met Leu Pro Ser Lys Ala Gly Ile Trp Arg Val Glu
   1310 1315 1320
   Cys Leu Ile Gly Glu His Leu His Ala Gly Met Ser Thr Leu Phe
   1325 1330 1335
   Leu Val Tyr Ser Asn Lys Cys Gln Thr Pro Leu Gly Met Ala Ser
   1340 1345 1350
   Gly His Ile Arg Asp Phe Gln Ile Thr Ala Ser Gly Gln Tyr Gly
   1355 1360 1365
   Gln Trp Ala Pro Lys Leu Ala Arg Leu His Tyr Ser Gly Ser Ile
   1370 1375 1380
   Asn Ala Trp Ser Thr Lys Glu Pro Phe Ser Trp Ile Lys Val Asp
   1385 1390 1395
   Leu Leu Ala Pro Met Ile Ile His Gly Ile Lys Thr Gln Gly Ala
   1400 1405 1410
   Arg Gln Lys Phe Ser Ser Leu Tyr Ile Ser Gln Phe Ile Ile Met
   1415 1420 1425
   Tyr Ser Leu Asp Gly Lys Lys Trp Gln Thr Tyr Arg Gly Asn Ser
   1430 1435 1440
   Thr Gly Thr Leu Met Val Phe Phe Gly Asn Val Asp Ser Ser Gly
   1445 1450 1455
   Ile Lys His Asn Ile Phe Asn Pro Pro Ile Ile Ala Arg Tyr Ile
   1460 1465 1470
   Arg Leu His Pro Thr His Tyr Ser Ile Arg Ser Thr Leu Arg Met
   1475 1480 1485
   Glu Leu Met Gly Cys Asp Leu Asn Ser Cys Ser Met Pro Leu Gly
   1490 1495 1500
   Met Glu Ser Lys Ala Ile Ser Asp Ala Gln Ile Thr Ala Ser Ser
   1505 1510 1515
   Tyr Phe Thr Asn Met Phe Ala Thr Trp Ser Pro Ser Lys Ala Arg
   1520 1525 1530
   Leu His Leu Gln Gly Arg Ser Asn Ala Trp Arg Pro Gln Val Asn
   1535 1540 1545
   Asn Pro Lys Glu Trp Leu Gln Val Asp Phe Gln Lys Thr Met Lys
   1550 1555 1560
   Val Thr Gly Val Thr Thr Gln Gly Val Lys Ser Leu Leu Thr Ser
   1565 1570 1575
   Met Tyr Val Lys Glu Phe Leu Ile Ser Ser Ser Gln Asp Gly His
   1580 1585 1590
   Gln Trp Thr Leu Phe Phe Gln Asn Gly Lys Val Lys Val Phe Gln
   1595 1600 1605
   Gly Asn Gln Asp Ser Phe Thr Pro Val Val Asn Ser Leu Asp Pro
   1610 1615 1620
   Pro Leu Leu Thr Arg Tyr Leu Arg Ile His Pro Gln Ser Trp Val
   1625 1630 1635
   His Gln Ile Ala Leu Arg Met Glu Val Leu Gly Cys Glu Ala Gln
   1640 1645 1650
   Asp Leu Tyr
   1655
<210> 40
   <211> 1656
   <212> PRT
   <213> artificial
<220>
   <223> FVIII and AP3 fusion protein
<400> 40
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Asp His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
       290                 295                 300
   Gln Phe Leu Leu Phe Cys His Ile Ser Ser His Gln His Asp Gly Met
   305                 310                 315                 320
   Glu Ala Tyr Val Lys Val Asp Ser Cys Pro Glu Glu Pro Gln Leu Arg
                   325                 330                 335
   Met Lys Asn Asn Glu Glu Ala Glu Asp Tyr Asp Asp Asp Leu Thr Asp
               340                 345                 350
   Ser Glu Met Asp Val Val Arg Phe Asp Asp Asp Asn Ser Pro Ser Phe
           355                 360                 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Gln Asn
   740 745 750
   Pro Pro Val Leu Lys Arg His Gln Arg Gln Val Gln Leu Gln Gln Ser
   755 760 765
   Gly Ala Glu Leu Val Arg Pro Gly Thr Ser Val Lys Ile Ser Cys Lys
   770 775 780
   Ala Ser Gly Tyr Thr Phe Thr Asn Tyr Trp Leu Gly Trp Val Lys Gln
   785 790 795 800
   Arg Pro Gly His Gly Leu Glu Trp Ile Gly Asp Ile Tyr Pro Gly Gly
   805 810 815
   Gly Tyr Asn Lys Tyr Asn Glu Asn Phe Lys Gly Lys Ala Thr Leu Thr
   820 825 830
   Ala Asp Thr Ser Ser Ser Thr Ala Tyr Met Gln Leu Ser Ser Leu Thr
   835 840 845
   Ser Glu Asp Ser Ala Val Tyr Phe Cys Ala Arg Glu Tyr Gly Asn Tyr
   850 855 860
   Asp Tyr Ala Met Asp Ser Trp Gly Gln Gly Thr Ser Val Thr Val Ser
   865 870 875 880
   Ser Gly Gly Gly Gly Ser Gly Gly Gly Gly Ser Gly Gly Gly Gly Ser
   885 890 895
   Asp Ile Val Met Thr Gln Ala Ala Pro Ser Val Pro Val Thr Pro Gly
   900 905 910
   Glu Ser Val Ser Ile Ser Cys Arg Ser Ser Arg Ser Leu Leu His Ser
   915 920 925
   Asn Gly Asn Thr Tyr Leu Cys Trp Phe Leu Gln Arg Pro Gly Gln Ser
   930 935 940
   Pro Gln Leu Leu Ile Tyr Arg Met Ser Asn Leu Ala Ser Gly Val Pro
   945 950 955 960
   Asp Arg Phe Ser Gly Ser Gly Ser Gly Thr Ala Phe Thr Leu Arg Ile
   965 970 975
   Ser Arg Val Glu Ala Glu Asp Val Gly Val Tyr Tyr Cys Met Gln His
   980 985 990
   Leu Glu Tyr Pro Phe Thr Phe Gly Ser Gly Thr Lys Leu Glu Ile Lys
   995 1000 1005
   Arg Gln Ser Pro Arg Ser Phe Gln Lys Lys Thr Arg His Tyr Phe
   1010 1015 1020
   Ile Ala Ala Val Glu Arg Leu Trp Asp Tyr Gly Met Ser Ser Ser
   1025 1030 1035
   Pro His Val Leu Arg Asn Arg Ala Gln Ser Gly Ser Val Pro Gln
   1040 1045 1050
   Phe Lys Lys Val Val Phe Gln Glu Phe Thr Asp Gly Ser Phe Thr
   1055 1060 1065
   Gln Pro Leu Tyr Arg Gly Glu Leu Asn Glu His Leu Gly Leu Leu
   1070 1075 1080
   Gly Pro Tyr Ile Arg Ala Glu Val Glu Asp Asn Ile Met Val Thr
   1085 1090 1095
   Phe Arg Asn Gln Ala Ser Arg Pro Tyr Ser Phe Tyr Ser Ser Leu
   1100 1105 1110
   Ile Ser Tyr Glu Glu Asp Gln Arg Gln Gly Ala Glu Pro Arg Lys
   1115 1120 1125
   Asn Phe Val Lys Pro Asn Glu Thr Lys Thr Tyr Phe Trp Lys Val
   1130 1135 1140
   Gln His His Met Ala Pro Thr Lys Asp Glu Phe Asp Cys Lys Ala
   1145 1150 1155
   Trp Ala Tyr Phe Ser Asp Val Asp Leu Glu Lys Asp Val His Ser
   1160 1165 1170
   Gly Leu Ile Gly Pro Leu Leu Val Cys His Thr Asn Thr Leu Asn
   1175 1180 1185
   Pro Ala His Gly Arg Gln Val Thr Val Gln Glu Phe Ala Leu Phe
   1190 1195 1200
   Phe Thr Ile Phe Asp Glu Thr Lys Ser Trp Tyr Phe Thr Glu Asn
   1205 1210 1215
   Met Glu Arg Asn Cys Arg Ala Pro Cys Asn Ile Gln Met Glu Asp
   1220 1225 1230
   Pro Thr Phe Lys Glu Asn Tyr Arg Phe His Ala Ile Asn Gly Tyr
   1235 1240 1245
   Ile Met Asp Thr Leu Pro Gly Leu Val Met Ala Gln Asp Gln Arg
   1250 1255 1260
   Ile Arg Trp Tyr Leu Leu Ser Met Gly Ser Asn Glu Asn Ile His
   1265 1270 1275
   Ser Ile His Phe Ser Gly His Val Phe Thr Val Arg Lys Lys Glu
   1280 1285 1290
   Glu Tyr Lys Met Ala Leu Tyr Asn Leu Tyr Pro Gly Val Phe Glu
   1295 1300 1305
   Thr Val Glu Met Leu Pro Ser Lys Ala Gly Ile Trp Arg Val Glu
   1310 1315 1320
   Cys Leu Ile Gly Glu His Leu His Ala Gly Met Ser Thr Leu Phe
   1325 1330 1335
   Leu Val Tyr Ser Asn Lys Cys Gln Thr Pro Leu Gly Met Ala Ser
   1340 1345 1350
   Gly His Ile Arg Asp Phe Gln Ile Thr Ala Ser Gly Gln Tyr Gly
   1355 1360 1365
   Gln Trp Ala Pro Lys Leu Ala Arg Leu His Tyr Ser Gly Ser Ile
   1370 1375 1380
   Asn Ala Trp Ser Thr Lys Glu Pro Phe Ser Trp Ile Lys Val Asp
   1385 1390 1395
   Leu Leu Ala Pro Met Ile Ile His Gly Ile Lys Thr Gln Gly Ala
   1400 1405 1410
   Arg Gln Lys Phe Ser Ser Leu Tyr Ile Ser Gln Phe Ile Ile Met
   1415 1420 1425
   Tyr Ser Leu Asp Gly Lys Lys Trp Gln Thr Tyr Arg Gly Asn Ser
   1430 1435 1440
   Thr Gly Thr Leu Met Val Phe Phe Gly Asn Val Asp Ser Ser Gly
   1445 1450 1455
   Ile Lys His Asn Ile Phe Asn Pro Pro Ile Ile Ala Arg Tyr Ile
   1460 1465 1470
   Arg Leu His Pro Thr His Tyr Ser Ile Arg Ser Thr Leu Arg Met
   1475 1480 1485
   Glu Leu Met Gly Cys Asp Leu Asn Ser Cys Ser Met Pro Leu Gly
   1490 1495 1500
   Met Glu Ser Lys Ala Ile Ser Asp Ala Gln Ile Thr Ala Ser Ser
   1505 1510 1515
   Tyr Phe Thr Asn Met Phe Ala Thr Trp Ser Pro Ser Lys Ala Arg
   1520 1525 1530
   Leu His Leu Gln Gly Arg Ser Asn Ala Trp Arg Pro Gln Val Asn
   1535 1540 1545
   Asn Pro Lys Glu Trp Leu Gln Val Asp Phe Gln Lys Thr Met Lys
   1550 1555 1560
   Val Thr Gly Val Thr Thr Gln Gly Val Lys Ser Leu Leu Thr Ser
   1565 1570 1575
   Met Tyr Val Lys Glu Phe Leu Ile Ser Ser Ser Gln Asp Gly His
   1580 1585 1590
   Gln Trp Thr Leu Phe Phe Gln Asn Gly Lys Val Lys Val Phe Gln
   1595 1600 1605
   Gly Asn Gln Asp Ser Phe Thr Pro Val Val Asn Ser Leu Asp Pro
   1610 1615 1620
   Pro Leu Leu Thr Arg Tyr Leu Arg Ile His Pro Gln Ser Trp Val
   1625 1630 1635
   His Gln Ile Ala Leu Arg Met Glu Val Leu Gly Cys Glu Ala Gln
   1640 1645 1650
   Asp Leu Tyr
   1655
<210> 41
   <211> 1694
   <212> PRT
   <213> artificial
<220>
   <223> FVIII and AP3 fusion protein
<400> 41
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Asp His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
       290                 295                 300
   Gln Phe Leu Leu Phe Cys His Ile Ser Ser His Gln His Asp Gly Met
   305                 310                 315                 320
   Glu Ala Tyr Val Lys Val Asp Ser Cys Pro Glu Glu Pro Gln Leu Arg
                   325                 330                 335
   Met Lys Asn Asn Glu Glu Ala Glu Asp Tyr Asp Asp Asp Leu Thr Asp
               340                 345                 350
   Ser Glu Met Asp Val Val Arg Phe Asp Asp Asp Asn Ser Pro Ser Phe
           355                 360                 365
   Ile Gln Ile Arg Ser Val Ala Lys Lys His Pro Lys Thr Trp Val His
       370                 375                 380
   Tyr Ile Ala Ala Glu Glu Glu Asp Trp Asp Tyr Ala Pro Leu Val Leu
   385                 390                 395                 400
   Ala Pro Asp Asp Arg Ser Tyr Lys Ser Gln Tyr Leu Asn Asn Gly Pro
                   405                 410                 415
   Gln Arg Ile Gly Arg Lys Tyr Lys Lys Val Arg Phe Met Ala Tyr Thr
               420                 425                 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Gln Val
   740 745 750
   Gln Leu Gln Gln Ser Gly Ala Glu Leu Val Arg Pro Gly Thr Ser Val
   755 760 765
   Lys Ile Ser Cys Lys Ala Ser Gly Tyr Thr Phe Thr Asn Tyr Trp Leu
   770 775 780
   Gly Trp Val Lys Gln Arg Pro Gly His Gly Leu Glu Trp Ile Gly Asp
   785 790 795 800
   Ile Tyr Pro Gly Gly Gly Tyr Asn Lys Tyr Asn Glu Asn Phe Lys Gly
   805 810 815
   Lys Ala Thr Leu Thr Ala Asp Thr Ser Ser Ser Thr Ala Tyr Met Gln
   820 825 830
   Leu Ser Ser Leu Thr Ser Glu Asp Ser Ala Val Tyr Phe Cys Ala Arg
   835 840 845
   Glu Tyr Gly Asn Tyr Asp Tyr Ala Met Asp Ser Trp Gly Gln Gly Thr
   850 855 860
   Ser Val Thr Val Ser Ser Gly Gly Gly Gly Ser Gly Gly Gly Gly Ser
   865 870 875 880
   Gly Gly Gly Gly Ser Asp Ile Val Met Thr Gln Ala Ala Pro Ser Val
   885 890 895
   Pro Val Thr Pro Gly Glu Ser Val Ser Ile Ser Cys Arg Ser Ser Arg
   900 905 910
   Ser Leu Leu His Ser Asn Gly Asn Thr Tyr Leu Cys Trp Phe Leu Gln
   915 920 925
   Arg Pro Gly Gln Ser Pro Gln Leu Leu Ile Tyr Arg Met Ser Asn Leu
   930 935 940
   Ala Ser Gly Val Pro Asp Arg Phe Ser Gly Ser Gly Ser Gly Thr Ala
   945 950 955 960
   Phe Thr Leu Arg Ile Ser Arg Val Glu Ala Glu Asp Val Gly Val Tyr
   965 970 975
   Tyr Cys Met Gln His Leu Glu Tyr Pro Phe Thr Phe Gly Ser Gly Thr
   980 985 990
   Lys Leu Glu Ile Lys Arg Ser Gln Asn Pro Pro Val Leu Lys Arg His
   995 1000 1005
   Gln Arg Glu Ile Thr Arg Thr Thr Leu Gln Ser Asp Gln Glu Glu
   1010 1015 1020
   Ile Asp Tyr Asp Asp Thr Ile Ser Val Glu Met Lys Lys Glu Asp
   1025 1030 1035
   Phe Asp Ile Tyr Asp Glu Asp Glu Asn Gln Ser Pro Arg Ser Phe
   1040 1045 1050
   Gln Lys Lys Thr Arg His Tyr Phe Ile Ala Ala Val Glu Arg Leu
   1055 1060 1065
   Trp Asp Tyr Gly Met Ser Ser Ser Pro His Val Leu Arg Asn Arg
   1070 1075 1080
   Ala Gln Ser Gly Ser Val Pro Gln Phe Lys Lys Val Val Phe Gln
   1085 1090 1095
   Glu Phe Thr Asp Gly Ser Phe Thr Gln Pro Leu Tyr Arg Gly Glu
   1100 1105 1110
   Leu Asn Glu His Leu Gly Leu Leu Gly Pro Tyr Ile Arg Ala Glu
   1115 1120 1125
   Val Glu Asp Asn Ile Met Val Thr Phe Arg Asn Gln Ala Ser Arg
   1130 1135 1140
   Pro Tyr Ser Phe Tyr Ser Ser Leu Ile Ser Tyr Glu Glu Asp Gln
   1145 1150 1155
   Arg Gln Gly Ala Glu Pro Arg Lys Asn Phe Val Lys Pro Asn Glu
   1160 1165 1170
   Thr Lys Thr Tyr Phe Trp Lys Val Gln His His Met Ala Pro Thr
   1175 1180 1185
   Lys Asp Glu Phe Asp Cys Lys Ala Trp Ala Tyr Phe Ser Asp Val
   1190 1195 1200
   Asp Leu Glu Lys Asp Val His Ser Gly Leu Ile Gly Pro Leu Leu
   1205 1210 1215
   Val Cys His Thr Asn Thr Leu Asn Pro Ala His Gly Arg Gln Val
   1220 1225 1230
   Thr Val Gln Glu Phe Ala Leu Phe Phe Thr Ile Phe Asp Glu Thr
   1235 1240 1245
   Lys Ser Trp Tyr Phe Thr Glu Asn Met Glu Arg Asn Cys Arg Ala
   1250 1255 1260
   Pro Cys Asn Ile Gln Met Glu Asp Pro Thr Phe Lys Glu Asn Tyr
   1265 1270 1275
   Arg Phe His Ala Ile Asn Gly Tyr Ile Met Asp Thr Leu Pro Gly
   1280 1285 1290
   Leu Val Met Ala Gln Asp Gln Arg Ile Arg Trp Tyr Leu Leu Ser
   1295 1300 1305
   Met Gly Ser Asn Glu Asn Ile His Ser Ile His Phe Ser Gly His
   1310 1315 1320
   Val Phe Thr Val Arg Lys Lys Glu Glu Tyr Lys Met Ala Leu Tyr
   1325 1330 1335
   Asn Leu Tyr Pro Gly Val Phe Glu Thr Val Glu Met Leu Pro Ser
   1340 1345 1350
   Lys Ala Gly Ile Trp Arg Val Glu Cys Leu Ile Gly Glu His Leu
   1355 1360 1365
   His Ala Gly Met Ser Thr Leu Phe Leu Val Tyr Ser Asn Lys Cys
   1370 1375 1380
   Gln Thr Pro Leu Gly Met Ala Ser Gly His Ile Arg Asp Phe Gln
   1385 1390 1395
   Ile Thr Ala Ser Gly Gln Tyr Gly Gln Trp Ala Pro Lys Leu Ala
   1400 1405 1410
   Arg Leu His Tyr Ser Gly Ser Ile Asn Ala Trp Ser Thr Lys Glu
   1415 1420 1425
   Pro Phe Ser Trp Ile Lys Val Asp Leu Leu Ala Pro Met Ile Ile
   1430 1435 1440
   His Gly Ile Lys Thr Gln Gly Ala Arg Gln Lys Phe Ser Ser Leu
   1445 1450 1455
   Tyr Ile Ser Gln Phe Ile Ile Met Tyr Ser Leu Asp Gly Lys Lys
   1460 1465 1470
   Trp Gln Thr Tyr Arg Gly Asn Ser Thr Gly Thr Leu Met Val Phe
   1475 1480 1485
   Phe Gly Asn Val Asp Ser Ser Gly Ile Lys His Asn Ile Phe Asn
   1490 1495 1500
   Pro Pro Ile Ile Ala Arg Tyr Ile Arg Leu His Pro Thr His Tyr
   1505 1510 1515
   Ser Ile Arg Ser Thr Leu Arg Met Glu Leu Met Gly Cys Asp Leu
   1520 1525 1530
   Asn Ser Cys Ser Met Pro Leu Gly Met Glu Ser Lys Ala Ile Ser
   1535 1540 1545
   Asp Ala Gln Ile Thr Ala Ser Ser Tyr Phe Thr Asn Met Phe Ala
   1550 1555 1560
   Thr Trp Ser Pro Ser Lys Ala Arg Leu His Leu Gln Gly Arg Ser
   1565 1570 1575
   Asn Ala Trp Arg Pro Gln Val Asn Asn Pro Lys Glu Trp Leu Gln
   1580 1585 1590
   Val Asp Phe Gln Lys Thr Met Lys Val Thr Gly Val Thr Thr Gln
   1595 1600 1605
   Gly Val Lys Ser Leu Leu Thr Ser Met Tyr Val Lys Glu Phe Leu
   1610 1615 1620
   Ile Ser Ser Ser Gln Asp Gly His Gln Trp Thr Leu Phe Phe Gln
   1625 1630 1635
   Asn Gly Lys Val Lys Val Phe Gln Gly Asn Gln Asp Ser Phe Thr
   1640 1645 1650
   Pro Val Val Asn Ser Leu Asp Pro Pro Leu Leu Thr Arg Tyr Leu
   1655 1660 1665
   Arg Ile His Pro Gln Ser Trp Val His Gln Ile Ala Leu Arg Met
   1670 1675 1680
   Glu Val Leu Gly Cys Glu Ala Gln Asp Leu Tyr
   1685 1690
<210> 42
   <211> 1694
   <212> PRT
   <213> artificial
<220>
   <223> FVIII and AP3 fusion protein
<400> 42
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Asp His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
   210 215 220
   Ala Arg Ala Trp Pro Lys Met His Thr Val Asn Gly Tyr Val Asn Arg
   225 230 235 240
   Ser Leu Pro Gly Leu Ile Gly Cys His Arg Lys Ser Val Tyr Trp His
   245 250 255
   Val Ile Gly Met Gly Thr Thr Pro Glu Val His Ser Ile Phe Leu Glu
   260 265 270
   Gly His Thr Phe Leu Val Arg Asn His Arg Gln Ala Ser Leu Glu Ile
   275 280 285
   Ser Pro Ile Thr Phe Leu Thr Ala Gln Thr Leu Leu Met Asp Leu Gly
   290 295 300
   Gln Phe Leu Leu Phe Cys His Ile Ser Ser His Gln His Asp Gly Met
   305 310 315 320
   Glu Ala Tyr Val Lys Val Asp Ser Cys Pro Glu Glu Pro Gln Leu Arg
   325 330 335
   Met Lys Asn Asn Glu Glu Ala Glu Asp Tyr Asp Asp Asp Leu Thr Asp
   340 345 350
   Ser Glu Met Asp Val Val Arg Phe Asp Asp Asp Asn Ser Pro Ser Phe
   355 360 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Asp Ile
   740 745 750
   Val Met Thr Gln Ala Ala Pro Ser Val Pro Val Thr Pro Gly Glu Ser
   755 760 765
   Val Ser Ile Ser Cys Arg Ser Ser Arg Ser Leu Leu His Ser Asn Gly
   770 775 780
   Asn Thr Tyr Leu Cys Trp Phe Leu Gln Arg Pro Gly Gln Ser Pro Gln
   785 790 795 800
   Leu Leu Ile Tyr Arg Met Ser Asn Leu Ala Ser Gly Val Pro Asp Arg
   805 810 815
   Phe Ser Gly Ser Gly Ser Gly Thr Ala Phe Thr Leu Arg Ile Ser Arg
   820 825 830
   Val Glu Ala Glu Asp Val Gly Val Tyr Tyr Cys Met Gln His Leu Glu
   835 840 845
   Tyr Pro Phe Thr Phe Gly Ser Gly Thr Lys Leu Glu Ile Lys Arg Gly
   850 855 860
   Gly Gly Gly Ser Gly Gly Gly Gly Ser Gly Gly Gly Gly Ser Gln Val
   865 870 875 880
   Gln Leu Gln Gln Ser Gly Ala Glu Leu Val Arg Pro Gly Thr Ser Val
   885 890 895
   Lys Ile Ser Cys Lys Ala Ser Gly Tyr Thr Phe Thr Asn Tyr Trp Leu
   900 905 910
   Gly Trp Val Lys Gln Arg Pro Gly His Gly Leu Glu Trp Ile Gly Asp
   915 920 925
   Ile Tyr Pro Gly Gly Gly Tyr Asn Lys Tyr Asn Glu Asn Phe Lys Gly
   930 935 940
   Lys Ala Thr Leu Thr Ala Asp Thr Ser Ser Ser Thr Ala Tyr Met Gln
   945 950 955 960
   Leu Ser Ser Leu Thr Ser Glu Asp Ser Ala Val Tyr Phe Cys Ala Arg
   965 970 975
   Glu Tyr Gly Asn Tyr Asp Tyr Ala Met Asp Ser Trp Gly Gln Gly Thr
   980 985 990
   Ser Val Thr Val Ser Ser Ser Gln Asn Pro Pro Val Leu Lys Arg His
   995 1000 1005
   Gln Arg Glu Ile Thr Arg Thr Thr Leu Gln Ser Asp Gln Glu Glu
   1010 1015 1020
   Ile Asp Tyr Asp Asp Thr Ile Ser Val Glu Met Lys Lys Glu Asp
   1025 1030 1035
   Phe Asp Ile Tyr Asp Glu Asp Glu Asn Gln Ser Pro Arg Ser Phe
   1040 1045 1050
   Gln Lys Lys Thr Arg His Tyr Phe Ile Ala Ala Val Glu Arg Leu
   1055 1060 1065
   Trp Asp Tyr Gly Met Ser Ser Ser Pro His Val Leu Arg Asn Arg
   1070 1075 1080
   Ala Gln Ser Gly Ser Val Pro Gln Phe Lys Lys Val Val Phe Gln
   1085 1090 1095
   Glu Phe Thr Asp Gly Ser Phe Thr Gln Pro Leu Tyr Arg Gly Glu
   1100 1105 1110
   Leu Asn Glu His Leu Gly Leu Leu Gly Pro Tyr Ile Arg Ala Glu
   1115 1120 1125
   Val Glu Asp Asn Ile Met Val Thr Phe Arg Asn Gln Ala Ser Arg
   1130 1135 1140
   Pro Tyr Ser Phe Tyr Ser Ser Leu Ile Ser Tyr Glu Glu Asp Gln
   1145 1150 1155
   Arg Gln Gly Ala Glu Pro Arg Lys Asn Phe Val Lys Pro Asn Glu
   1160 1165 1170
   Thr Lys Thr Tyr Phe Trp Lys Val Gln His His Met Ala Pro Thr
   1175 1180 1185
   Lys Asp Glu Phe Asp Cys Lys Ala Trp Ala Tyr Phe Ser Asp Val
   1190 1195 1200
   Asp Leu Glu Lys Asp Val His Ser Gly Leu Ile Gly Pro Leu Leu
   1205 1210 1215
   Val Cys His Thr Asn Thr Leu Asn Pro Ala His Gly Arg Gln Val
   1220 1225 1230
   Thr Val Gln Glu Phe Ala Leu Phe Phe Thr Ile Phe Asp Glu Thr
   1235 1240 1245
   Lys Ser Trp Tyr Phe Thr Glu Asn Met Glu Arg Asn Cys Arg Ala
   1250 1255 1260
   Pro Cys Asn Ile Gln Met Glu Asp Pro Thr Phe Lys Glu Asn Tyr
   1265 1270 1275
   Arg Phe His Ala Ile Asn Gly Tyr Ile Met Asp Thr Leu Pro Gly
   1280 1285 1290
   Leu Val Met Ala Gln Asp Gln Arg Ile Arg Trp Tyr Leu Leu Ser
   1295 1300 1305
   Met Gly Ser Asn Glu Asn Ile His Ser Ile His Phe Ser Gly His
   1310 1315 1320
   Val Phe Thr Val Arg Lys Lys Glu Glu Tyr Lys Met Ala Leu Tyr
   1325 1330 1335
   Asn Leu Tyr Pro Gly Val Phe Glu Thr Val Glu Met Leu Pro Ser
   1340 1345 1350
   Lys Ala Gly Ile Trp Arg Val Glu Cys Leu Ile Gly Glu His Leu
   1355 1360 1365
   His Ala Gly Met Ser Thr Leu Phe Leu Val Tyr Ser Asn Lys Cys
   1370 1375 1380
   Gln Thr Pro Leu Gly Met Ala Ser Gly His Ile Arg Asp Phe Gln
   1385 1390 1395
   Ile Thr Ala Ser Gly Gln Tyr Gly Gln Trp Ala Pro Lys Leu Ala
   1400 1405 1410
   Arg Leu His Tyr Ser Gly Ser Ile Asn Ala Trp Ser Thr Lys Glu
   1415 1420 1425
   Pro Phe Ser Trp Ile Lys Val Asp Leu Leu Ala Pro Met Ile Ile
   1430 1435 1440
   His Gly Ile Lys Thr Gln Gly Ala Arg Gln Lys Phe Ser Ser Leu
   1445 1450 1455
   Tyr Ile Ser Gln Phe Ile Ile Met Tyr Ser Leu Asp Gly Lys Lys
   1460 1465 1470
   Trp Gln Thr Tyr Arg Gly Asn Ser Thr Gly Thr Leu Met Val Phe
   1475 1480 1485
   Phe Gly Asn Val Asp Ser Ser Gly Ile Lys His Asn Ile Phe Asn
   1490 1495 1500
   Pro Pro Ile Ile Ala Arg Tyr Ile Arg Leu His Pro Thr His Tyr
   1505 1510 1515
   Ser Ile Arg Ser Thr Leu Arg Met Glu Leu Met Gly Cys Asp Leu
   1520 1525 1530
   Asn Ser Cys Ser Met Pro Leu Gly Met Glu Ser Lys Ala Ile Ser
   1535 1540 1545
   Asp Ala Gln Ile Thr Ala Ser Ser Tyr Phe Thr Asn Met Phe Ala
   1550 1555 1560
   Thr Trp Ser Pro Ser Lys Ala Arg Leu His Leu Gln Gly Arg Ser
   1565 1570 1575
   Asn Ala Trp Arg Pro Gln Val Asn Asn Pro Lys Glu Trp Leu Gln
   1580 1585 1590
   Val Asp Phe Gln Lys Thr Met Lys Val Thr Gly Val Thr Thr Gln
   1595 1600 1605
   Gly Val Lys Ser Leu Leu Thr Ser Met Tyr Val Lys Glu Phe Leu
   1610 1615 1620
   Ile Ser Ser Ser Gln Asp Gly His Gln Trp Thr Leu Phe Phe Gln
   1625 1630 1635
   Asn Gly Lys Val Lys Val Phe Gln Gly Asn Gln Asp Ser Phe Thr
   1640 1645 1650
   Pro Val Val Asn Ser Leu Asp Pro Pro Leu Leu Thr Arg Tyr Leu
   1655 1660 1665
   Arg Ile His Pro Gln Ser Trp Val His Gln Ile Ala Leu Arg Met
   1670 1675 1680
   Glu Val Leu Gly Cys Glu Ala Gln Asp Leu Tyr
   1685 1690